# EUROPEAN PATENT APPLICATION

(11) **EP 0 826 674 A1**
(43) Date of publication of application: **04.03.1998**
(21) Application number: 96907753.6
(22) Date of filing: 02.04.1996
(51) Int. Cl.: C07D 239/47, C07D 401/12, A61K 31/505

(54) **NOVEL PYRIMIDINE DERIVATIVES EFFICACIOUS AS PSYCHOTROPIC DRUG AND PROCESS FOR THE PRODUCTION THEREOF**

(30) Priority: 03.04.1995 JP 103121/95
(71) Applicant: SUMITOMO PHARMACEUTICALS COMPANY, LIMITED, Osaka-shi, Osaka-fu 541 (JP)
(72) Inventor: IGARASHI, Jun-etsu, Osaka-shi, Osaka 552 (JP); NISHIMURA, Tamiki, Toyonaka-shi, Osaka 561 (JP); SUNAGAWA, Makoto, Itami-shi, Hyogo 664 (JP)
(74) Representative: Cresswell, Thomas Anthony
(86) International application number: JP9600895
(87) International publication number: WO9631488

(57) **Abstract**

A compound represented by the formula, or a pharmacologically acceptable salt thereof: 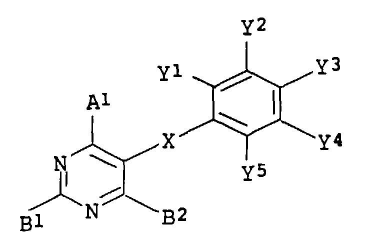
wherein A¹ is a halogen atom, a lower alkyl group, a cycloalkyl group, a substituted lower alkyl group or a substituted cycloalkyl group;
B¹ and B², which may be the same or different, are hydrogen atoms, amino groups or substituted amino groups;
X is a sulfur atom or an oxygen atom; and
Y¹, Y², Y³, Y⁴ and Y⁵, which may be the same or different, are hydrogen atoms, halogen atoms, lower alkyl groups, cycloalkyl groups, lower alkenyl groups, lower alkynyl groups, aryl groups, heterocyclic groups, substituted lower alkyl groups, substituted cycloalkyl groups, substituted lower alkenyl groups, substituted lower alkynyl groups, substituted aryl groups, or substituted heterocyclic groups, interacts strongly with receptors of central nervous system, such as dopamine, serotonin and the like, is excellent in bioabsorbability, stability and the like, and hence is very useful as a psychotropic drug having reduced side effects.

## Description

### TECHNICAL FIELD

The present invention relates to novel pyrimidine derivatives, a process for production thereof, and psychotropic drugs containing the pyrimidine derivaive as an active ingredient. Further, the present invention relates to intermediates for producing said derivatives and a process for production thereof.

### BACKGROUND ART

Psychotropic drugs such as phenothiazine type drugs (e.g. chlorpromazine), butyrophenone type drugs (e.g. haloperidol), benzamide type drugs (e.g. Sulpiride), etc. have been used as useful therapeutic drugs for schizophrenia, manic-depressive psychosis, Parkinson's disease, psychosis caused by drug abuse, psychosis associated with senile dementia, etc. with close attention to the balance between their desired effects and adverse side effects. Japanese Patent Examined Publication Nos. 54-16510 and 58-049548 and French Patent No. 7206981 disclose that pyrimidine derivatives having a methylthio group are pharmaceutically useful compounds having psychotropic effect.

The present invention is intended to provide psychotropic drugs which interact strongly with receptors of central nervous system, such as dopamine, serotonin and the like, are superior in bioabsorbability and stability, hardly cause extrapyramidal diseases, and exercise reduced side effects such as drop of consciousness level and orthostatic hypotension which are due to α₁-receptor blocking effect. The present invention is also intended to provide intermediates useful for production of such compounds.

### DISCLOSURE OF THE INVENTION

The present inventors earnestly investigated and consequently have found novel pyrimidine derivatives which interact strongly with receptors of central nervous system, such as dopamine, serotonin and the like.

That is, the present invention relates to a pyrimidine derivative represented by the formula [I], or a pharmacologically acceptable salt thereof: 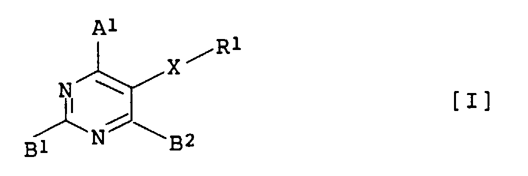
wherein X is a sulfur atom or an oxygen atom;
A¹ is a halogen atom, a lower alkyl group, a cycloalkyl group, an aryl group, a substituted lower alkyl group or a substituted cycloalkyl group in the case of X being a sulfur atom, and A¹ is a lower alkyl group, a cycloalkyl group, an aryl group, a substituted lower alkyl group or a substituted cycloalkyl group in the case of X being an oxygen atom;
B¹ is a hydrogen atom, a lower alkyl group, a cycloalkyl group, an aryl group, a substituted aryl group, an amino group or a substituted amino group;
B² is an amino group or a substituted amino group; and
R¹ is a group selected from groups represented by the formulas (1) to (6): 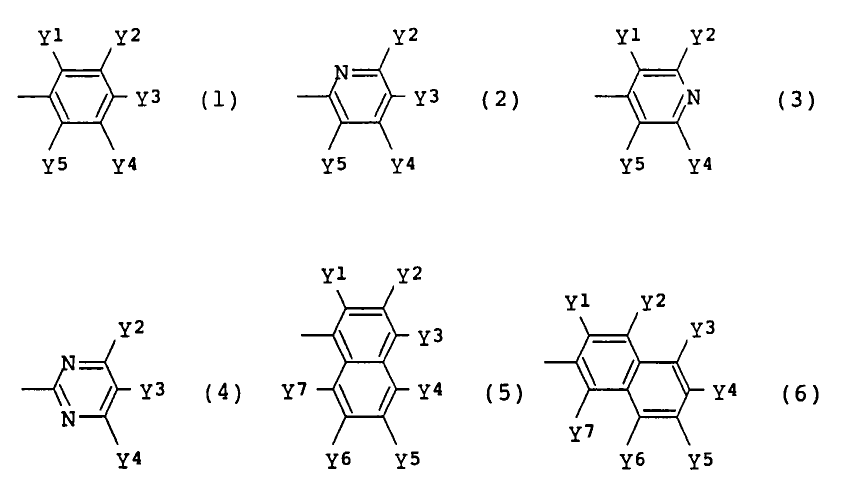
wherein Y¹, Y², Y³, Y⁴, Y⁵, Y⁶ and Y⁷, which may be the same or different, are hydrogen atoms, halogen atoms, lower alkyl groups, cycloalkyl groups, lower alkenyl groups, lower alkynyl groups, aryl groups, heterocyclic groups, substituted lower alkyl groups, substituted cycloalkyl groups, substituted lower alkenyl groups, substituted lower alkynyl groups, substituted aryl groups, or substituted heterocyclic groups.

The present invention also relates to a pyrimidine derivative represented by the formula [II], or a pharmacologically acceptable salt thereof: 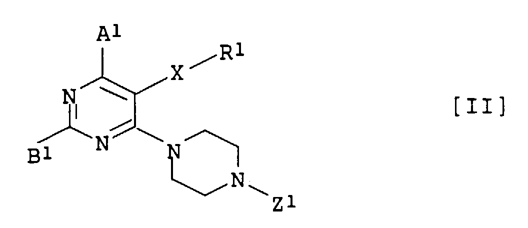 wherein A¹, B¹, X and R¹ are as defined above, and Z¹ is a hydrogen atom, a lower alkyl group or a substituted lower alkyl group.

The present invention further relates to a process for producing the above-mentioned pyrimidine derivative [I] or a pharmacologically acceptable salt thereof which is characterized by reacting a compound represented by the formula [III]: 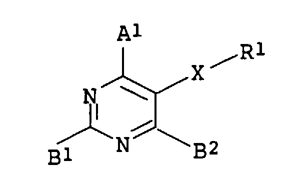 wherein A¹, B¹, X and R¹ are as defined above and A² is a halogen atom, with an amine represented by the formula H-B² wherein B² is an amino group or a substituted amino group.

The present invention still further relates to a compound represented by the formula [IV]: 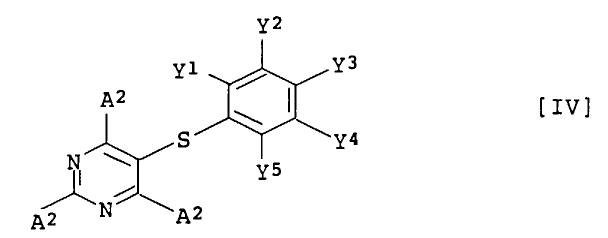 wherein A² is a halogen atom, and Y¹, Y², Y³, Y⁴ and Y⁵, which may be the same or different, are hydrogen atoms, halogen atoms, lower alkyl groups, cycloalkyl groups, lower alkenyl groups, lower alkynyl groups, aryl groups, heterocyclic groups, substituted lower alkyl groups, substituted cycloalkyl groups, substituted lower alkenyl groups, substituted lower alkynyl groups, substituted aryl groups, or substituted heterocyclic groups.

### BEST MODE FOR CARRYING OUT THE INVENTION

In the present specification, the lower alkyl group includes, for example, linear or branched alkyl groups of 1 to 6 carbon atoms. Specific examples thereof are methyl, ethyl, propyl, 1-methylethyl, butyl, 1-methylpropyl, 2-methylpropyl, 1,1-dimethylethyl, pentyl, 1-methylbutyl, 1-ethylpropyl, hexyl, 1-methylpentyl, 1-ethylbutyl, 2-methylpentyl, etc.

The cycloalkyl group includes, for example, cycloalkyl groups of 5 to 7 carbon atoms. Specific examples thereof are cyclopentyl, cyclohexyl, cycloheptyl, etc.

The lower alkenyl group includes, for example, linear or branched alkenyl groups of 2 to 6 carbon atoms having 1 to 3 double bonds. Specific examples thereof are vinyl, 1-propenyl, 2-propenyl, 1-methylvinyl, 1-butenyl, 2-butenyl, 1-pentenyl, 1-hexenyl, etc.

The lower alkynyl group includes, for example, linear or branched alkynyl groups of 2 to 6 carbon atoms having 1 to 3 triple bonds. Specific examples thereof are ethynyl, 1-propynyl, 2-propynyl, 1-butynyl, 1-pentynyl, 1-hexynyl, etc.

The aryl group includes, for example, aryl groups of 6 to 14 carbon atoms. Specific examples thereof are phenyl, 1-naphthyl, 2-naphthyl, 1-anthranyl, 2-anthranyl, etc.

The heterocyclic group includes, for example, 5- to 7-membered monocyclic, 9- to 10-membered bicyclic, or 12- to 14-membered tricyclic saturated or unsaturated heterocyclic groups containing 1 to 4 heteroatoms optionally selected from the group consisting of oxygen atom, nitrogen atom and sulfur atom. The nitrogen atom or the sulfur atom, or both may be oxidized if necessary. The bonding position of the heterocyclic group is any of the nitrogen and carbon atoms. Specific examples of the heterocyclic group include, for example, 6-membered monocyclic saturated heterocyclic groups containing 1 or 2 heteroatoms optionally selected from the group consisting of oxygen atom, nitrogen atom and sulfur atom, such as piperidyl, piperazinyl, morpholinyl, etc.; 6-membered monocyclic aromatic heterocyclic groups containing 1 or 2 nitrogen atoms, such as pyridyl, pyridazinyl, pyrazinyl, etc.; 5-membered monocyclic saturated heterocyclic groups containing 1 or 2 heteroatoms optionally selected from the group consisting of oxygen atom, nitrogen atom and sulfur atom, such as oxolanyl, pyrrolidinyl, etc.; 5-membered monocyclic aromatic heterocyclic groups containing 1 to 3 heteroatoms optionally selected from the group consisting of oxygen atom, nitrogen atom and sulfur atom, such as imidazolyl, furyl, 2-oxo-1,3-dioxolenyl, pyrrolyl, 5-oxo-2-tetrahydrofuranyl, etc.; 9- to 10-membered bicyclic aromatic heterocyclic groups containing 1 to 3 heteroatoms optionally selected from the group consisting of oxygen atom, nitrogen atom and sulfur atom, such as quinolyl, isoquinolyl, indolyl, 1,3-dihydro-3-oxo-1-isobenzofuranyl, etc.; and 12- to 14-membered tricyclic aromatic heterocyclic groups containing 1 to 3 heteroatoms optionally selected from the group consisting of oxygen atom, nitrogen atom and sulfur atom, such as anthraquinolyl, etc.

The substituted lower alkyl group includes substituted lower alkyl groups having 1 to 5 substituents optionally selected from the group consisting of cycloalkyl groups; substituted cycloalkyl groups; aryl groups; substituted aryl groups; heterocyclic groups; substituted heterocyclic groups; halogen atoms; -OP¹ wherein P¹ is a hydrogen atom, a lower alkyl group, a cycloalkyl group, an aryl group or a hydroxyl-protecting group; -OCOP² wherein P² is a hydrogen atom, a lower alkyl group, a cycloalkyl group, an aryl group or a heterocyclic group; -NP³P⁴ wherein P³ is a hydrogen atom or a lower alkyl group, and P⁴ is a hydrogen atom, a lower alkyl group, a cycloalkyl group, an aryl group, a heterocyclic group or an amino-protecting group; -C(=NP⁵)NHP⁶ wherein P⁵ and P⁶, which may be the same or different, are hydrogen atoms, lower alkyl groups, cycloalkyl groups or guanidino-protecting groups; -NHC(=NP⁵)NHP⁶ wherein P⁵ and P⁶ are as defined above; nitro groups; cyano groups; -COOP⁷ wherein P⁷ is a hydrogen atom, a lower alkyl group, a cycloalkyl group, an aryl group, a heterocyclic group or a carboxyl-protecting group; -CONP⁸P⁹ wherein P⁸ is a hydrogen atom or a lower alkyl group, and P⁹ is a hydrogen atom, a lower alkyl group or an amide-protecting group; -SP¹⁰ wherein P¹⁰ is a hydrogen atom, a lower alkyl group, a cycloalkyl group, an aryl group, a heterocyclic group or a thiol-protecting group; -COP¹¹ wherein P¹¹ is a hydrogen atom, a lower alkyl group, a cycloalkyl group, an aryl group or a heterocyclic group; -NHCOP¹² wherein P¹² is a hydrogen atom, a lower alkyl group, a cycloalkyl group or an aryl group; -S(O)sP¹³ wherein P¹³ is a lower alkyl group, a cycloalkyl group or an aryl group, and s is 1 or 2; -SO₂NP¹⁴P¹⁵ wherein P¹⁴ and P¹⁵, which may be the same or different, are hydrogen atoms, lower alkyl groups, cycloalkyl groups or aryl groups; and -OCOOP¹⁶ wherein P¹⁶ is a lower alkyl group, a cycloalkyl group, an aryl group or a heterocyclic group.

Specific examples of such substituted lower alkyl groups are benzyl, 2-phenylethyl, 3-phenylpropyl, 4-phenylbutyl, 5-phenylpentyl, 6-phenylhexyl, 4-chlorobenzyl, indolylmethyl, 2-(3-indolyl)ethyl, bromomethyl, pivaloyloxymethyl, 1-ethoxycarbonyloxyethyl, 5-methyl-2-oxo-1,3-dioxolen-4-ylmethyl, 2-chloroethyl, 2-dimethylaminoethyl, diethylaminoethyl, 3-dimethylamino-2-(dimethylaminomethyl)propyl, 2-(1-morpholinyl)ethyl, 1-acetoxyethyl, 1-methoxycarbonyloxyethyl, acetoxymethyl, 1-acetoxy-1-phenylmethyl, methoxycarbonylmethyl, 1-pivaloyloxyethyl, 2-hydroxyethyl, 3-hydroxypropyl, etc.

The substituted lower alkenyl group includes substituted lower alkenyl groups having 1 to 5 substituents optionally selected from the group consisting of cycloalkyl groups; aryl groups; heterocyclic groups; halogen atoms; -OP¹⁷ wherein P¹⁷ is a hydrogen atom, a lower alkyl group or a hydroxyl-protecting group; -OCOP¹⁸ wherein P¹⁸ is a hydrogen atom or a lower alkyl group; nitro groups; cyano groups; -COOP¹⁹ wherein P¹⁹ is a hydrogen atom or a carboxyl-protecting group; -CONP⁸P⁹ wherein P⁸ and P⁹ are as defined above; -COP²⁰ wherein P²⁰ is a hydrogen atom or a lower alkyl group; and -OCOOP²¹ wherein P²¹ is a hydrogen atom or a lower alkyl group. Specific examples thereof are 3-phenyl-2-propenyl, 5-methoxy-2-pentenyl, 2-carboxyvinyl, etc.

The substituted lower alkynyl group includes substituted lower alkynyl groups having 1 to 5 substituents optionally selected from the group consisting of cycloalkyl groups; aryl groups; heterocyclic groups; halogen atoms; -OP¹⁷ wherein P¹⁷ is as defined above; -OCOP¹⁸ wherein P¹⁸ is as defined above; nitro groups; cyano groups; -COOP¹⁹ wherein P¹⁹ is as defined above; -CONP⁸P⁹ wherein P⁸ and P⁹ are as defined above; -COP²⁰ wherein P²⁰ is as defined above; and -OCOOP²¹ wherein P²¹ is as defined above. Specific examples thereof are 3-phenyl-2-propynyl, etc.

The substituted cycloalkyl group includes substituted cycloalkyl groups having 1 to 3 substituents optionally selected from the group consisting of lower alkyl groups; aryl groups; heterocyclic groups; halogen atoms; -OP¹⁷ wherein P¹⁷ is as defined above; -OCOP¹⁸ wherein P¹⁸ is as defined above; -NP²²P²³ wherein P²² is a hydrogen atom or a lower alkyl group, and P²³ is a hydrogen atom, a lower alkyl group or an amino-protecting group; -C(=NP²⁴)NHP²⁵ wherein P²⁴ and P²⁵, which may be the same or different, are hydrogen atoms or guanidino-protecting groups; -NHC(=NP²⁴)NHP²⁵ wherein P²⁴ and P²⁵ are as defined above; nitro groups; cyano groups; -COOP¹⁹ wherein P¹⁹ is as defined above; -CONP⁸P⁹ wherein P⁸ and P⁹ are as defined above; -SP²⁶ wherein P²⁶ is a hydrogen atom, a lower alkyl group or a thiol-protecting group; -COP²⁰ wherein P²⁰ is as defined above; -NHCOP²⁷ wherein P²⁷ is a hydrogen atom or a lower alkyl group; -S(O)sP²⁸ wherein P²⁸ is a lower alkyl group, and s is as defined above; -SO₂NP²⁹P³⁰ wherein P²⁹ and P³⁰, which may be the same or different, are hydrogen atoms or lower alkyl groups; and -OCOOP²¹ wherein P²¹ is as defined above. Specific examples thereof are 4-chlorocyclohexyl, 4-cyanocyclohexyl, 2-dimethylaminocyclohexyl, 4-methoxycyclohexyl, etc.

The substituted heterocyclic group includes substituted heterocyclic groups having 1 to 3 substituents optionally selected from the group consisting of lower alkyl groups; cycloalkyl groups; aryl groups; heterocyclic groups; halogen atoms; -OP¹⁷ wherein P¹⁷ is as defined above; -OCOP¹⁸ wherein P¹⁸ is as defined above; -NP²²P²³ wherein P²² and P²³ are as defined above; -C(=NP²⁴)NHP²⁵ wherein P²⁴ and P²⁵ are as defined above; -NHC(=NP²⁴)NHP²⁵ wherein P²⁴ and P²⁵ are as defined above; nitro groups; cyano groups; -COOP⁷ wherein P⁷ is as defined above; -CONP⁸P⁹ wherein P⁸ and P⁹ are as defined above; -SP²⁶ wherein P²⁶ is as defined above; -COP²⁰ wherein P²⁰ is as defined above; -NHCOP²⁷ wherein P²⁷ is as defined above; -S(O)sP²⁸ wherein s and P²⁸ are as defined above; -SO₂NP²⁹P³⁰ wherein P²⁹ and P³⁰ are as defined above; and -OCOOP²¹ wherein P²¹ is as defined above. Specific examples thereof are 1-acetyl-4-piperidinyl, 1-benzyl-4-imidazolyl, 1-methyl-3-indolyl, 5-methyl-2-oxo-1,3-dioxolen-4-yl, etc.

The substituted aryl group includes substituted aryl groups having 1 to 5 substituents optionally selected from the following substituents in the groups (a), (b) and (c):
(a) a group consisting of lower alkyl groups; lower alkenyl groups; lower alkynyl groups; cycloalkyl groups; aryl groups; heterocyclic groups; substituted alkenyl groups; substituted alkynyl groups; substituted cycloalkyl groups; substituted heterocyclic groups; halogen atoms; -OP¹ wherein P¹ is as defined above; -OCOP² wherein P² is as defined above; -NP³P⁴ wherein P³ and P⁴ are as defined above; -C(=NP⁵)NHP⁶ wherein P⁵ and P⁶ are as defined above; -NHC(=NP⁵)NHP⁶ wherein P⁵ and P⁶ are as defined above; nitro groups; cyano groups; -COOP⁷ wherein P⁷ is as defined above; -CONP⁸P⁹ wherein P⁸ and P⁹ are as defined above; -SP¹⁰ wherein P¹⁰ is as defined above; -COP¹¹ wherein P¹¹ is as defined above; -NHCOP¹² wherein P¹² is as defined above; -S(O)sP¹³ wherein s and P¹³ are as defined above; -SO₂NP¹⁴P¹⁵ wherein P¹⁴ and P¹⁵ are as defined above; and -OCOOP¹⁶ wherein P¹⁶ is as defined above,
(b) substituted lower alkyl groups having, as the substituent, -OP¹ wherein P¹ is as defined above, or -NP³P⁴ wherein P³ and P⁴ are as defined above, and
(c) substituted lower alkyl groups having as the substituent(s) 1 to 5 atoms optionally selected from fluorine atom, chlorine atom and bromine atom.

When the substituted aryl group has two adjacent substituents, these substituents may be taken together to represent a 4- to 7-membered ring, like methylenedioxy group or the like.

Specific examples of the substituted aryl group are 4-fluorophenyl, 3-chlorophenyl, 4-chlorophenyl, 3-bromophenyl, 4-bromophenyl, 4-iodophenyl, 2,4-dichlorophenyl, 4-hydroxyphenyl, 3,4-dihydroxyphenyl, 4-methylphenyl, 4-methoxyphenyl, 3,4-dimethoxyphenyl, 3,4-methylenedioxyphenyl, 4-aminophenyl, 4-guanidinophenyl, 4-aminomethylphenyl, 4-cyanophenyl, 4-carboxylphenyl, 4-acetylphenyl, 4-chloro-1-naphthyl, 4-amidinophenyl, 4-nitrophenyl, 4-ethoxycarbonylphenyl, 4-acetoxyphenyl, 4-benzyloxyphenyl, 2-fluoro-4-hydroxyphenyl, 5-indanyl, 4-(2-carboxylvinyl)phenyl, 4-(2-butyl)phenyl, 3,5-dichloro-2-hydroxyphenyl, 2,3,4-trichlorophenyl, 2,4,5-trichlorophenyl, 2,4,6-tri-(2-propyl)phenyl, 2,5-dimethylphenyl, 2-nitrophenyl, 3-nitrophenyl, 2,4-dinitrophenyl, 4-chloro-3-nitrophenyl, 2,5-dimethoxyphenyl, 2,5-dimethylphenyl, 2-methoxycarbonylphenyl, 3-carboxylphenyl, 2-methoxy-5-carboxylphenyl, 4-t-butylphenyl, 4-ethylphenyl, 2-methylphenyl, 3-methylphenyl, 2,4,6-trimethylphenyl, 5-dimethylamino-1-naphthyl, 4-acetaminophenyl, 2,3,4,5,6-pentafluorophenyl, 2,3,4,5,6-pentamethylphenyl, 4-dimethylamino-3-nitrophenyl, 2,3,5,6-tetramethylphenyl, 2,5-dichlorophenyl, 4-trifluoromethylphenyl, 2-trifluoromethylphenyl, 3-trifluoromethylphenyl, 3,5-ditrifluoromethylphenyl, etc.

The halogen atom includes, for example, fluorine atom, chlorine atom, bromine atom, iodine atom, etc.

The amino or substituted amino group includes amino or substituted amino groups represented by the formula -NQ¹Q² wherein Q¹ and Q², which may be the same or different, are hydrogen atoms, lower alkyl groups, cycloalkyl groups, substituted lower alkyl groups or substituted cycloalkyl groups, or Q¹ and Q², when taken together with the nitrogen atom to which they are bonded, represent a 5- to 8-membered saturated or unsaturated cyclic amino group containing 1 to 3 nitrogen atoms in the ring, said cyclic amino group, when having two or three nitrogen atoms, being able to have a lower alkyl group as a substituent on any of the nitrogen atoms. Specific examples of the amino or substituted amino group are methylamino group, dimethylamino group, ethylmethylamino group, cyclohexylmethylamino group, dicyclohexylamino group, 4-methylcyclohexylethylamino group, piperidyl group, pyridyl group, N-methylpiperazyl group, piridazinyl group, etc.

The amine represented by the formula H-B² wherein B² is an amino group or a substituted amino group, includes the amine represented by the formula HNQ¹Q² wherein Q¹ and Q² are as defined above. Specific examples of the amine are methylamine, dimethylamine, ethylmethylamine, cyclohexylmethylamine, dicyclohexylamine, 4-methylcyclohexylethylamine, piperidine, pyridine, N-methylpiperazine, pyridazine, etc.

As each of the hydroxyl-protecting group, the amino-protecting group, the guanidino-protecting group, the carboxyl-protecting group, the amide-protecting group and the thiol-protecting group, any protecting group may be used so long as it is usually used in reaction. There may be used, for example, the protecting groups for amino acid side chains described in Izumiya et al. "Fundamentals and Practice of Peptide Synthesis", Maruzen Co., Ltd., 1985 or Green et al. "Protective Groups in Organic Synthesis", John Willey & Sons, 1991.

The hydroxyl-protecting group includes ether type protecting groups, acyl type protecting groups, etc. The ether type protecting groups include, for example, benzyl, 2-nitrobenzyl, 2,6-dichlorobenzyl, t-butyl, etc. The acyl type protecting groups include lower alkanoyl groups, etc. The lower alkanoyl groups include, for example, linear or branched alkanoyl groups of 5 or less carbon atoms. Specific examples thereof are acetyl, propanoyl, butanoyl, etc.

The amino-protecting group includes urethane type protecting groups and acyl type protecting groups. The urethane type protecting groups include, for example, benzyloxycarbonyl, 4-nitrobenzyloxycarbonyl, 4-methoxybenzyloxycarbonyl, 2-methanesulfonylethoxycarbonyl, 2,2,2-trichloroethoxycarbonyl, t-butoxycarbonyl, fluorenyloxycarbonyl, etc. The acyl type protecting groups include, for example, formyl, acetyl, benzoyl, trifluoroacetyl, etc.

The guanidino-protecting group includes, for example, urethane type protecting groups such as benzyloxycarbonyl, t-butoxycarbonyl, etc., and 4-toluenesulfonyl, 4-methoxybenzenesulfonyl, nitro groups, etc.

The carboxyl-protecting group includes ester type protecting groups such as a lower alkyl group, 2,2,2-trichloroethyl, benzyl, 4-methoxybenzyl, diphenylethyl, etc.

The amide-protecting group includes 2,4-dimethoxybenzyl, etc. The thiol-protecting group includes sulfide type protecting groups such as benzyl, 4-methylbenzyl, 4-methoxybenzyl, 4-nitrobenzyl, acetamidomethyl, etc.

Although the compound having any of the above-mentioned protecting groups may be used as an intermediate, it may be used as a pharmacologically active compound.

Preferable specific examples of A¹ are halogen atoms such as chlorine atom, bromine atom, etc.; and lower alkyl groups such as methyl, ethyl, etc.

Preferable specific examples of A² are chlorine atom, bromine atom, etc.

Preferable specific examples of Y¹, Y², Y³, Y⁴, Y⁵, Y⁶ and Y⁷ are hydrogen atom; lower alkyl groups such as methyl, ethyl, propyl, etc.; cyclohexyl group; benzyl group; substituted lower alkyl groups having, as the substituent, -OCOP³¹ wherein P³¹ is a lower alkyl group, a cycloalkyl group, an aryl group or a heterocyclic group, for example, acetoxymethyl, 1-acetoxyethyl, 1-acetoxy-1-phenylmethyl, pivaloyloxymethyl, 1-pivaloyloxyethyl, etc; substituted lower alkyl groups having, as the substituent, -OCOOP³² wherein P³² is a lower alkyl group, a cycloalkyl group, an aryl group or a heterocyclic group, for example, 1-methoxycarbonyloxyethyl, 1-ethoxycarbonyloxyethyl, etc; substituted lower alkyl groups having a substituted heterocyclic group as the substituent, such as 5-methyl-2-oxo-1,3-dioxolen-4-ylmethyl, 2-(1-morpholinyl)ethyl, etc.; heterocyclic groups such as 5-oxo-2-tetrahydrofuranyl, 1,3-dihydro-3-oxo-1-isobenzofuranyl, etc.; substituted aryl groups such as 5-indanyl, etc.; 3-dimethylamino-2-(dimethylaminomethyl)propyl; 2-dimethylaminoethyl; 2-diethylaminoethyl; methoxycarbonylmethyl; 2-dimethylaminocyclohexyl; and the like.

Preferable specific examples of Z¹ are hydrogen atom and lower alkyl groups such as methyl, ethyl, propyl, etc.

Preferable specific examples of B¹ are hydrogen atom, methylamino group, dimethylamino group, N-methylpiperazinone group, etc.

Preferable specific examples of B² are methylamino group, dimethylamino group, N-methylpiperazinone group, etc.

When the compound of the formula [I] has an asymmetric carbon atom, it may be either of isomers or a mixture of the isomers. When said compound has two or more asymmetric carbon atoms, it may be any of enatiomers, diastereomers and mixtures of the enantiomers or the diastereomers, such as a racemic mixture.

The compound of the formula [I] of the present invention may be produced by a well-known synthetic reaction by using easily available starting materials and reagents, unless otherwise specified. The compound may be produced, for example, by the following process or a modification thereof: 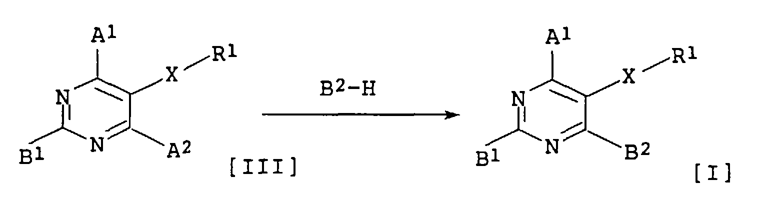 wherein A¹, A², B¹, B², X and R¹ are as defined above.

The compound [I] is obtained by reacting an amine represented by B²-H with a compound [III] in an amount of 1 to 3 equivalents per equivalent of the compound [III] at an appropriate temperature of room temperature to 80°C for 30 minutes to 2 hours in an alcohol such as methanol, ethanol or the like, or an inert solvent such as benzene, toluene, xylene, dichloromethane, chloroform, carbon tetrachloride or the like, optionally in the presence of a base such as pyridine, triethylamine, diisopropylethylamine or the like.

The compound [III], the starting material in the above reaction is a novel compound when X is S.

Next, production processes of the compound [III] are explained below in (A) to (C).

### (A) Production process in the case where X is S and B¹ is a lower alkyl group, a cycloalkyl group, an aryl group, a substituted aryl group, an amino group or a substituted amino group.

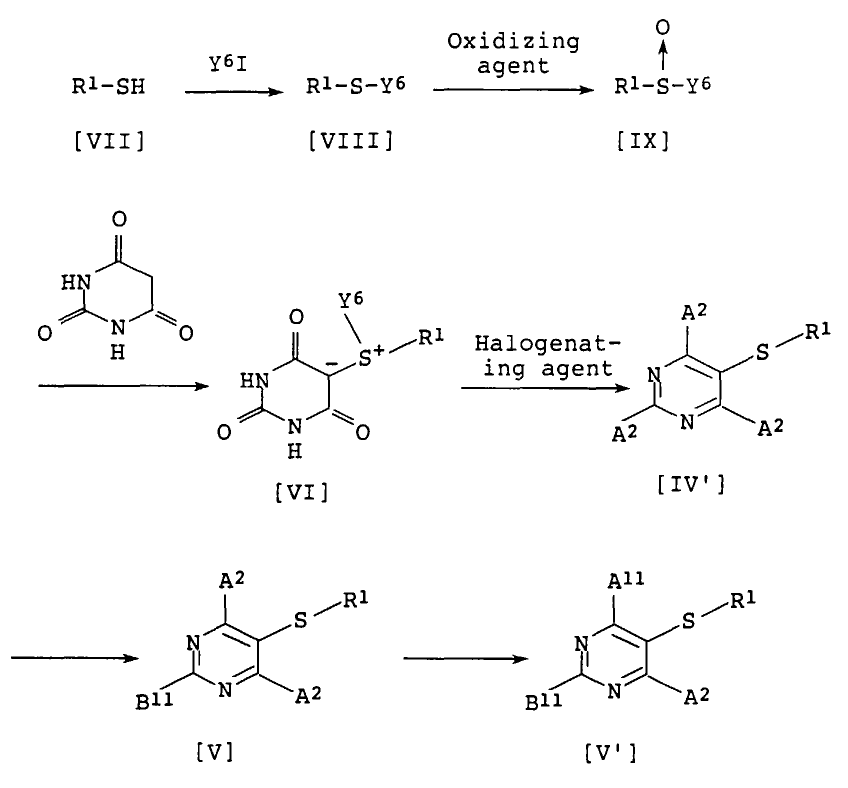

wherein A¹, A² and R¹ are as defined above, A¹¹ is a lower alkyl group, a cycloalkyl group, an aryl group, a substituted lower alkyl group or a substituted cycloalkyl group, B¹¹ is a lower alkyl group, a cycloalkyl group, an aryl group, a substituted aryl group, an amino group or a substituted amino group, and Y⁶ is a lower alkyl group, a cycloalkyl group, an aryl group, a substituted lower alkyl group or a substituted cycloalkyl group.

A thiol derivative [VII] is dissolved in water or dimethylformamide, followed by adding thereto a base (e.g. sodium hydroxide, potassium hydroxide, sodium carbonate, potassium carbonate or sodium hydrogen-carbonate, etc) or an aqueous solution of the base at a suitable concentration and then an iodine compound Y⁶I wherein Y⁶ is as defined above, and the resulting mixture is stirred at an appropriate temperature of 0 - 40°C for 2 to 24 hours to complete the reaction. After-treatment is carried out by a conventional method to obtain a sulfide derivative [VIII]. This compound [VIII] is suspended in water, followed by adding thereto an oxidizing agent such as hydrogen peroxide, sodium periodate or the like, and the resulting mixture is stirred at an appropriate temperature of 0°C to room temperature for 1 to 2 days to complete the reaction. After-treatment is carried out by a conventional method to obtain a sulfoxide [IX]. The obtained sulfoxide [IX] and barbituric acid are dissolved in acetic acid, followed by adding thereto acetic anhydride as an dehydrating agent, and the reaction is carried out at an appropriate temperature of 60 - 120°C, whereby a stabilized compound sulfur ylide [VI] can be obtained.

The thus obtained sulfur ylide [VI] includes 4-chlorophenylmethylsulfonium barbitylide, 3-chlorophenylmethylsulfonium barbitylide, methylphenylsulfonium barbitylide, etc.

A novel compound [IV'] is obtained by halogenating the compound [VI] with a halogenating agent. The halogenating agent includes thionyl halides such as thionyl chloride, thionyl bromide, etc.; phosphorus halides such as phosphorus oxychloride, phosphorus oxybromide, etc.; and hydrogen halides such as hydrogen chloride, hydrogen bromide, etc. In the halogenation, there may be used solvents such as benzene, toluene, xylene, dichloromethane, chloroform, carbon tetrachloride, dimethylformamide, diethyl ether, etc. The compound [IV'] is obtained by reacting the halogenating agent with the compound [VI] in an excess amount over the compound [VI] at an appropriate temperature of 0 - 90°C for 1 to 24 hours in the above-exemplified solvent without or optionally in the presence of a tertiary amine such as pyridine, quinoline, dimethylaniline, triethylamine, diisopropylamine or the like.

A compound [V] in which B¹¹ is an amino group or a substituted amino group may be obtained by regio-specific amination of the compound [IV'] with an amine represented by B¹¹-H.

In detail, the compound [IV'] is suspended in a mixture of water and a hydrophilic solvent (e.g. acetone, methyl ethyl ketone, etc) in a suitable ratio, followed by adding thereto the amine in an amount of 1 to 1.5 equivalents per equivalent of the compound [IV'], and the resulting mixture is stirred at an appropriate temperature of -10°C - 0°C for 30 minutes to 3 hours to complete the reaction. Then, after-treatment is carried out according to a conventional method to obtain the compound [V].

A compound [V] in which B¹¹ is a lower alkyl group, a cycloalkyl group, an aryl group or a substituted aryl group may be obtained by a conventional regio-selective Grignard reaction with the compound [IV'].

In detail, this compound [V] may be obtained by dissolving the compound [IV'] in an ether type inert solvent such as tetrahydrofuran, diethyl ether or the like, adding the resulting solution to metallic magnesium to obtain a Grignard reagent, and then reacting the Grignard reagent with a ketone, an aldehyde or an epoxide.

As an alternate process, the compound [V] in which B¹¹ is a lower alkyl group, a cycloalkyl group, an aryl group or a substituted aryl group may be obtained, by reacting the compound [IV'] with a metal halide (e.g. NaH, KH etc), and an alkyl halide, an aryl halide or the like.

In addition, a compound [V'] in which A¹¹ is a lower alkyl group, a cycloalkyl group, an aryl group, a substituted lower alkyl group or a substituted cycloalkyl group may be obtained by dissolving the compound [V] in an ether type inert solvent such as tetrahydrofuran, diethyl ether or the like, adding the resulting solution to metallic magnesium to obtain a Grignard reagent, and then reacting the Grignard reagent with a ketone, an aldehyde or an epoxide.

### (B) Production process in the case where X is O and B¹ is a lower alkyl group, a cycloalkyl group, an aryl group, a substituted aryl group, an amino group or a substituted amino group.

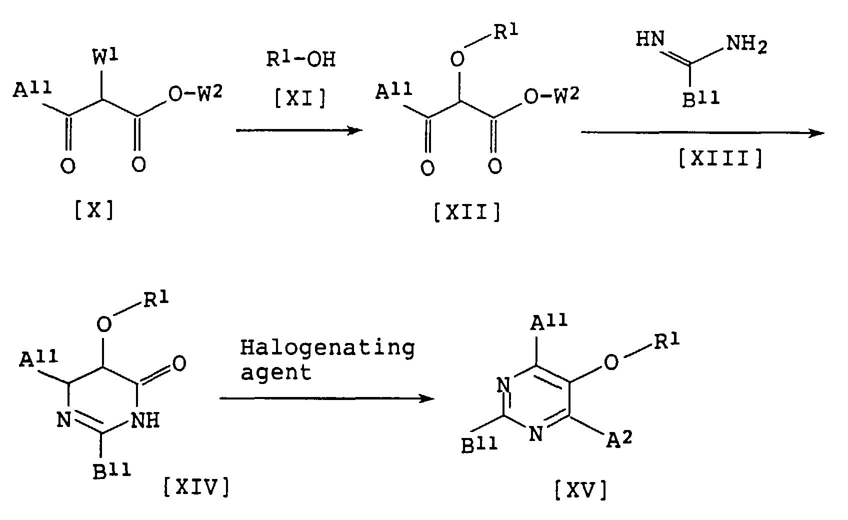

wherein A¹¹, A², B¹¹, B² and R¹ are as defined above, W¹ is a halogen atom, and W² is a lower alkyl group, a cycloalkyl group, a substituted lower alkyl group or a substituted cycloalkyl group.

A compound [XII] may be obtained by reacting sodium hydride in an amount of 1 to 2 equivalents and a phenol derivative [XI] in an amount of 1 to 1.2 equivalents with a compound [X] in an amount of one equivalent at an appropriate temperature of room temperature to 90°C for 5 minutes to 2 hours in an inert solvent such as benzene, toluene, xylene, dichloromethane, chloroform, carbon tetrachloride, diethyl ether, tetrahydrofuran, dioxane or the like, and then treating the reaction product by a conventional method.

Next, a compound [XIV] may be obtained by reacting a guanidine derivative [XIII] with a compound [XII] in an amount of 2 to 4 equivalents per equivalent of the compound [XII] at an appropriate temperature of room temperature to 60°C for 1 to 2 hours in an alcohol such as methanol, ethanol or the like, or an inert solvent such as benzene, toluene, xylene, dichloromethane, chloroform, carbon tetrachloride, diethyl ether, tetrahydrofuran, dioxane or the like, and then treating the reaction product by a conventional method.

Subsequently, a compound [XV] is obtained by halogenating the compound [XIV] with a halogenating agent in the same manner as in (A) above, and a compound [XVI] may be obtained by reacting the compound [XV] with an amine represented by B²-H, in the same manner as in (A) above.

### (C) Production process of the compound [III] in the case where B¹ is a hydrogen atom.

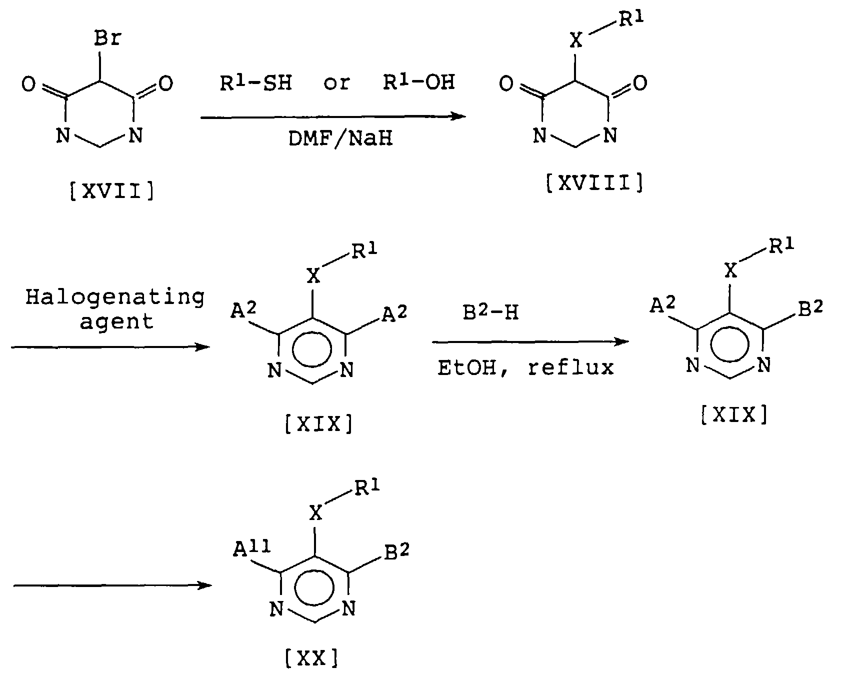

wherein A¹¹, A², B², R¹ and X are as defined above.

A compound [XVIII] may be obtained by reacting a compound [XVII] with a compound represented by R¹-SH or R¹-OH at an appropriate temperature of 0°C to 100°C in the presence of NaOH in an inert solvent such as benzene, toluene, xylene, dichloromethane, chloroform, carbon tetrachloride or the like.

Next, a compound [XIX] may be obtained by halogenating the compound [XVIII] with a halogenating agent in the same manner as in (A) above, and a compound [XX] may be obtained by reacting the compound [XIX] with an amine represented by B²-H, in the same manner as in (A) above. In addition, a compound [XXI] may be obtained by treating the compound [XX] with Grignard reaction in the same manner as in (A) above.

The above-mentioned compound [II] can easily be obtained by reacting the compound [III] with a substituted piperazine derivative having on one of the nitrogen atoms a substituent Z¹ which is as defined above. This reaction may be carried out in the same manner as for the above-mentioned reaction of the compound [III] with the amine.

As the pharmacologically acceptable salt, pharmacologically acceptable acid addition salts may be exemplified. The acid addition salts include inorganic acid addition salts such as chloride, sulfate, phosphate, etc.; and organic acid addition salts such as acetate, methanesulfonate, trifluoroacetate, citrate, fumarate, maleate, succinate, tartrate, salicylate, etc. Such salts can easily be produced by a well-known means. For example, the acetate is produced, by dissolving the compound of the formula [I] in water and adding thereto a necessary amount of acetic acid.

The compound of the formula [I] may be purified by a conventional purification method such as recrystallization, thin-layer chromatography, high performance liquid chromatography or the like.

Specific examples of the thus obtained compounds and intermediates are listed in the following Tables 1 to 77.

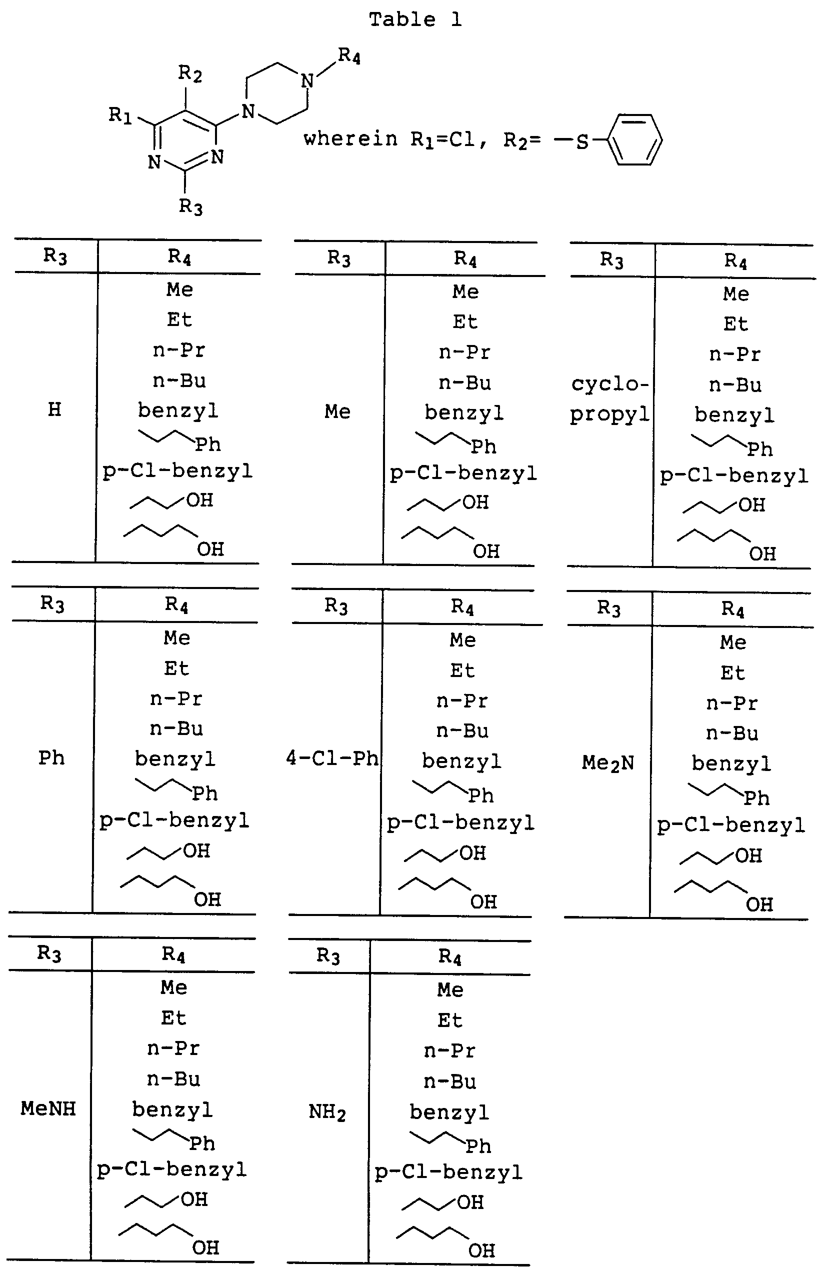

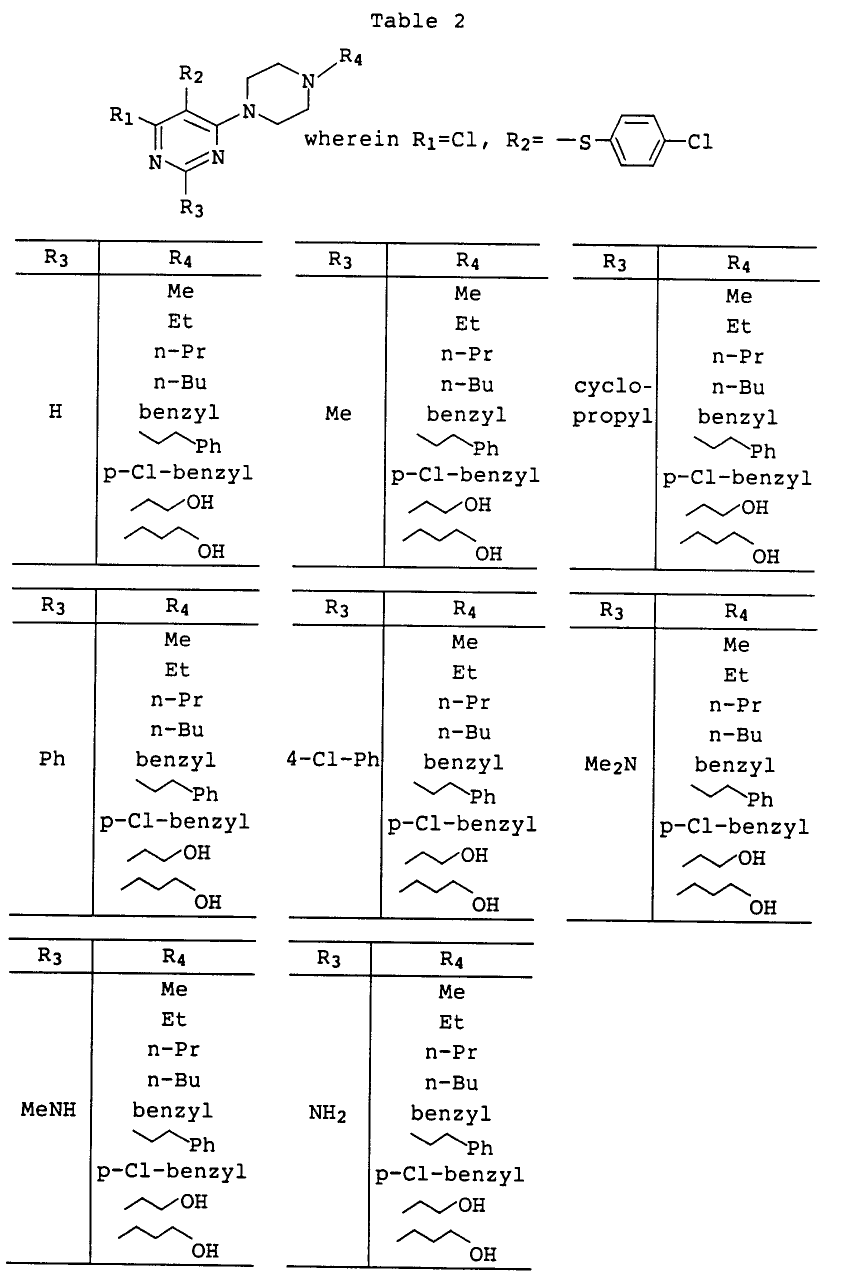

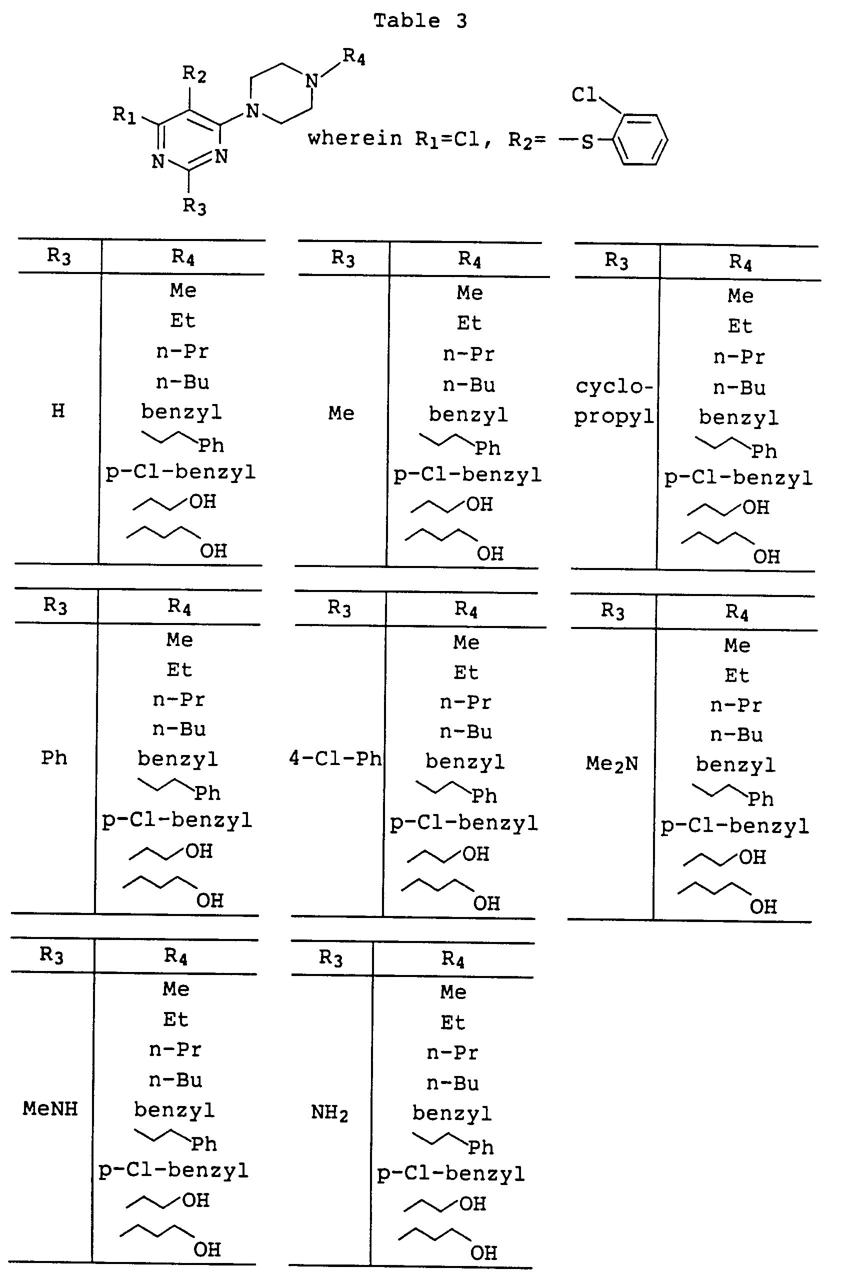

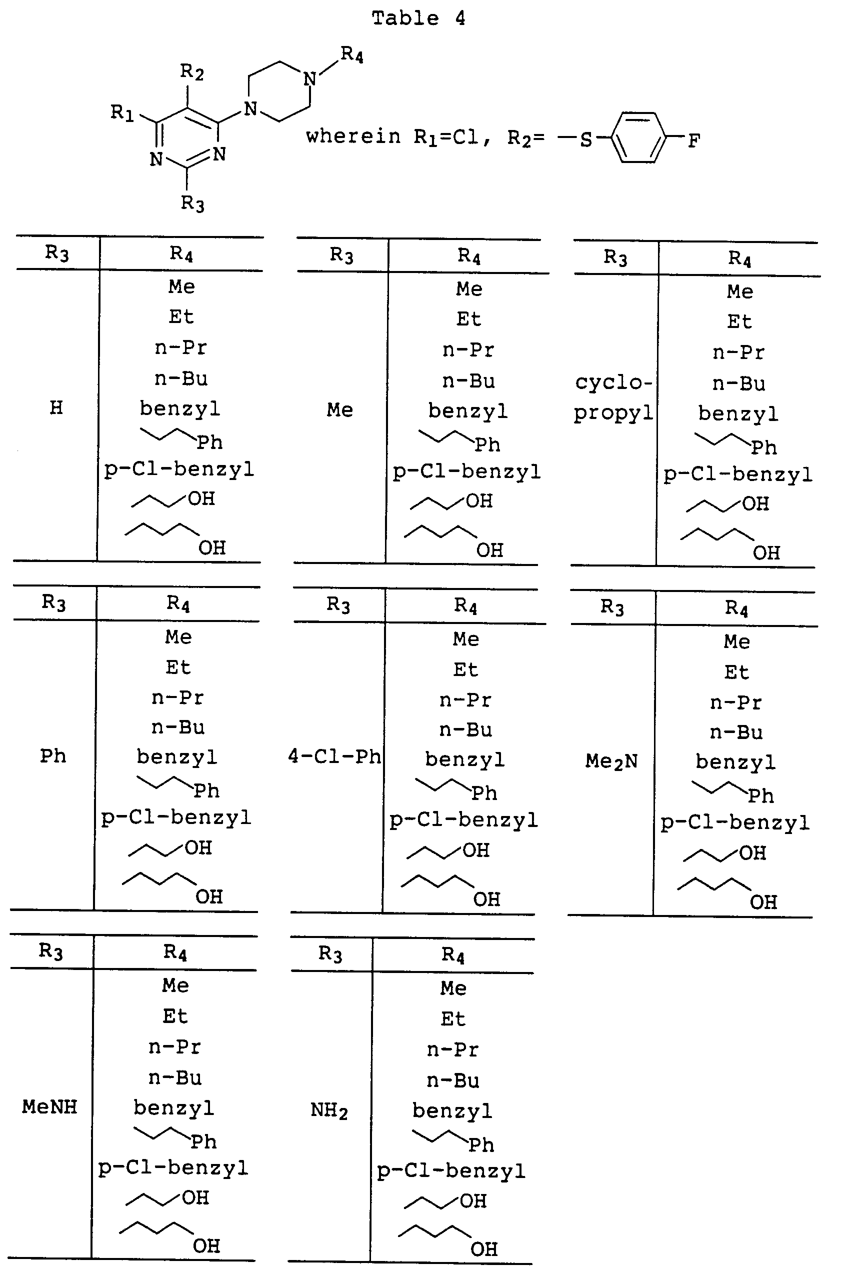

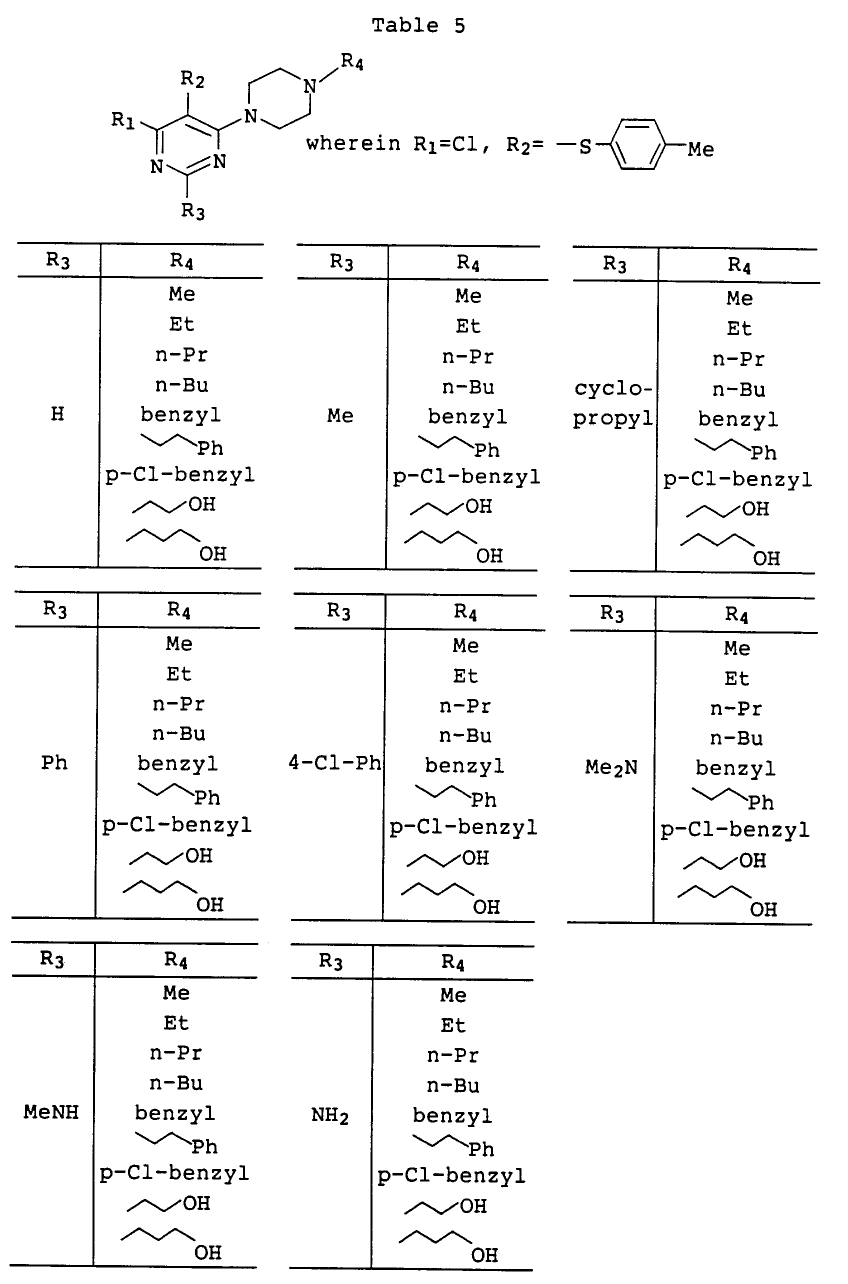

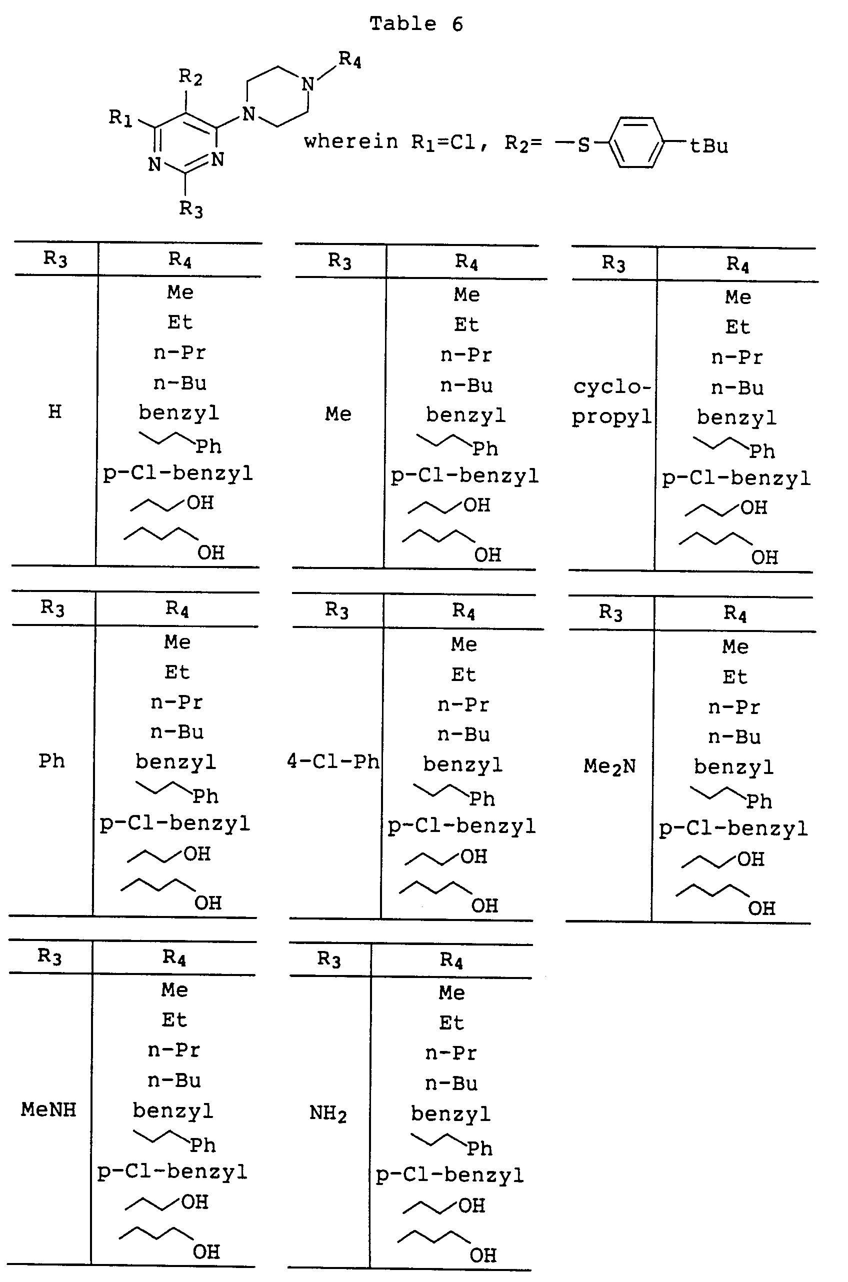

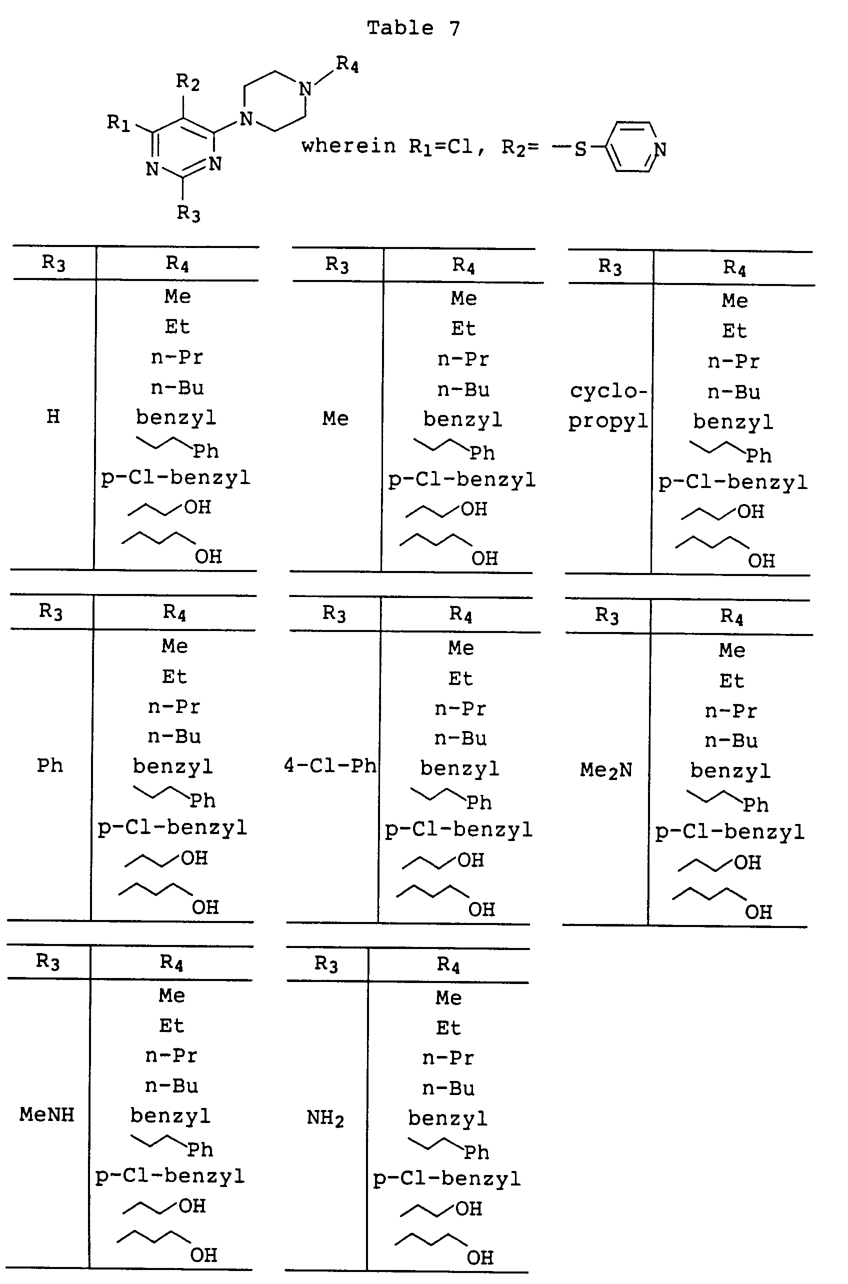

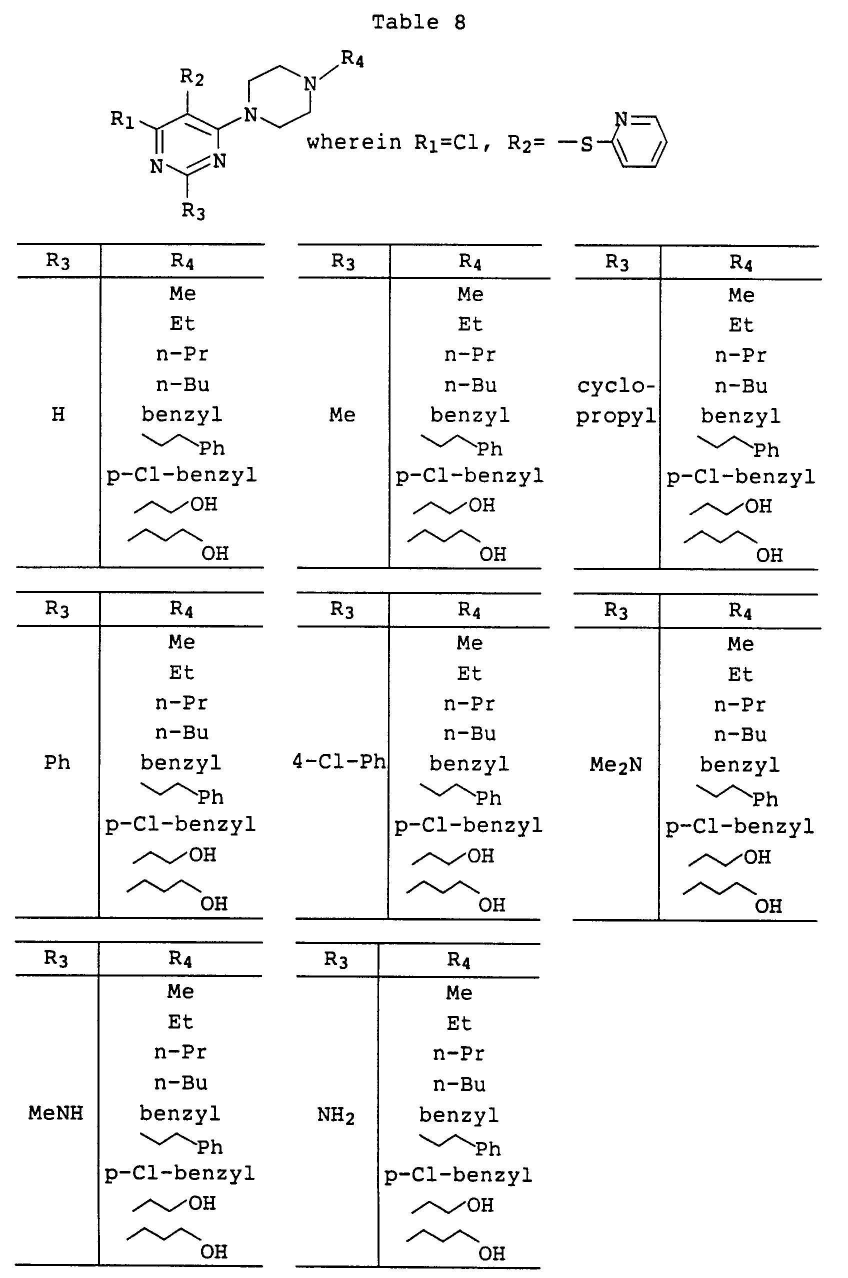

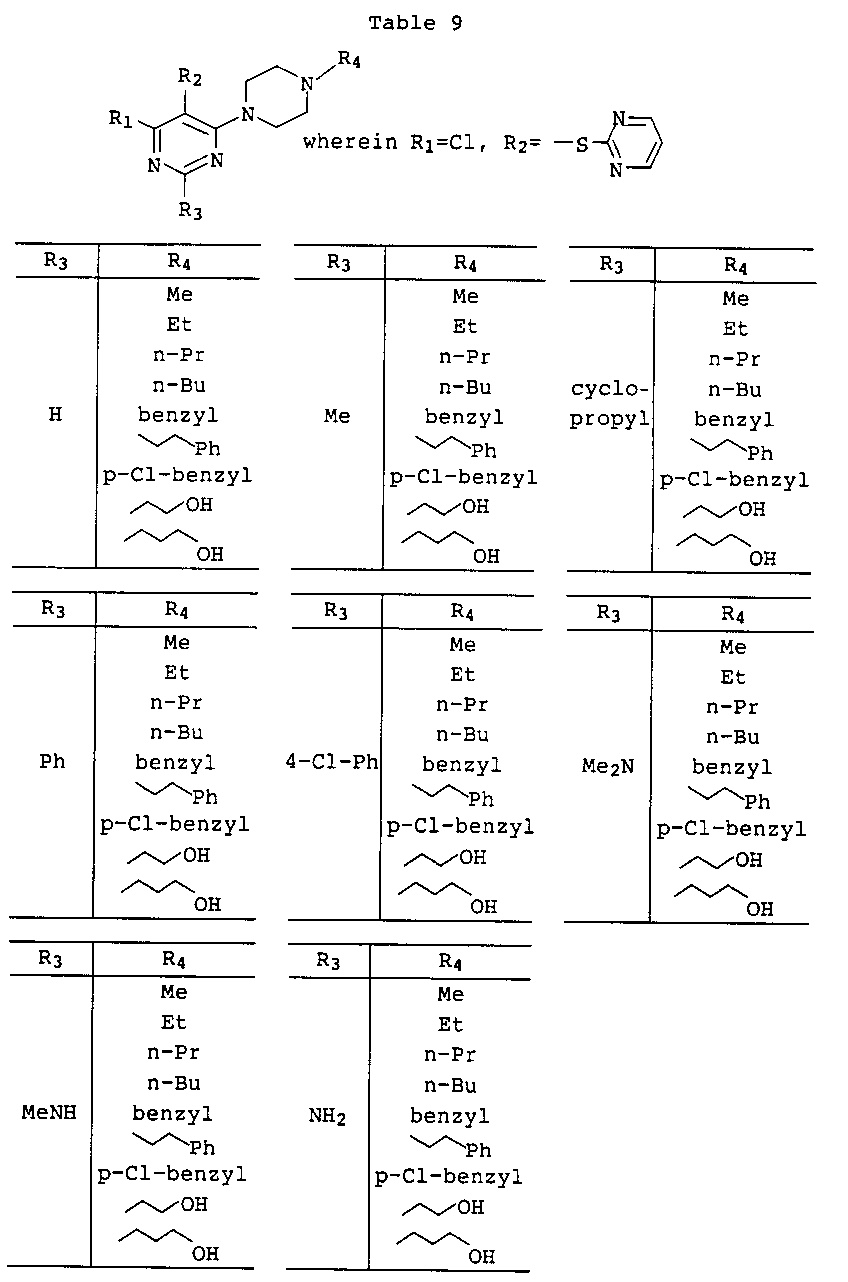

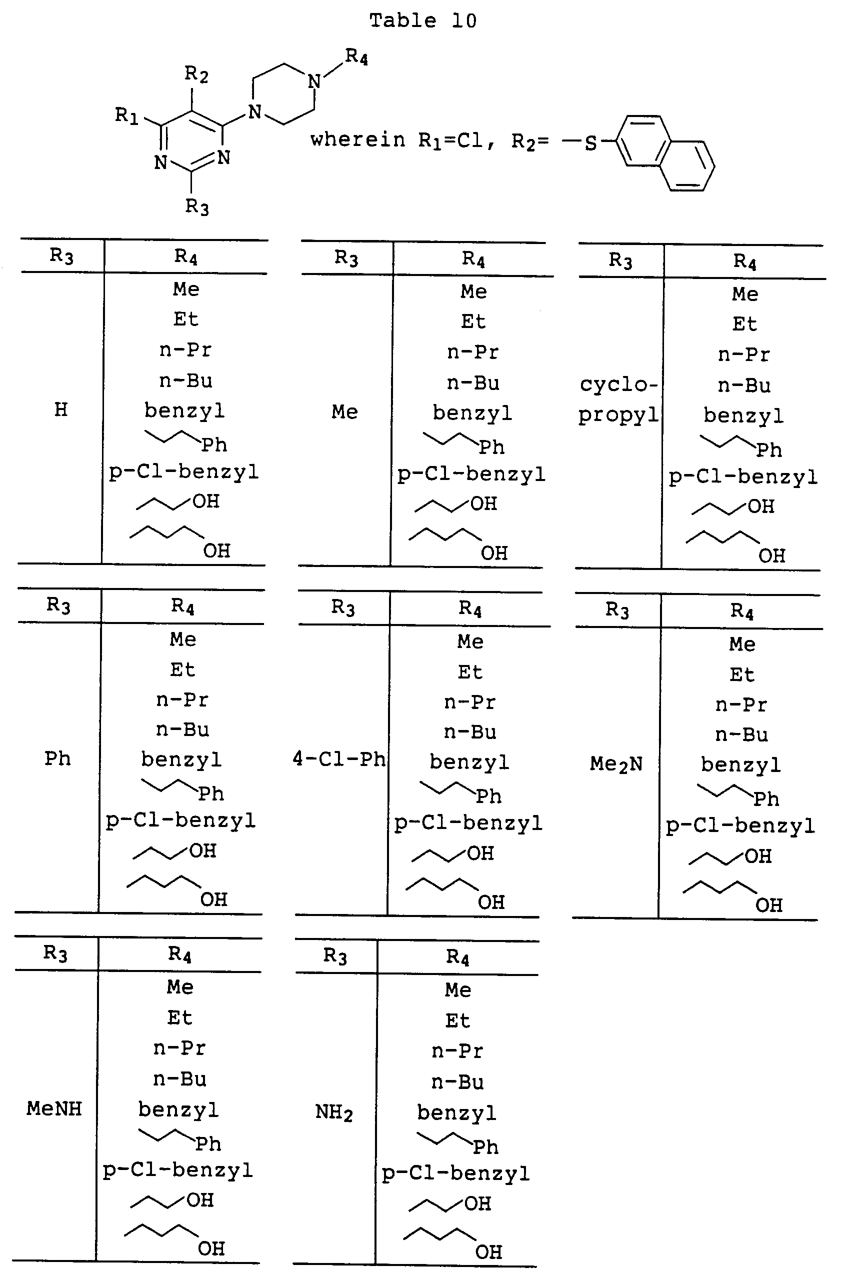

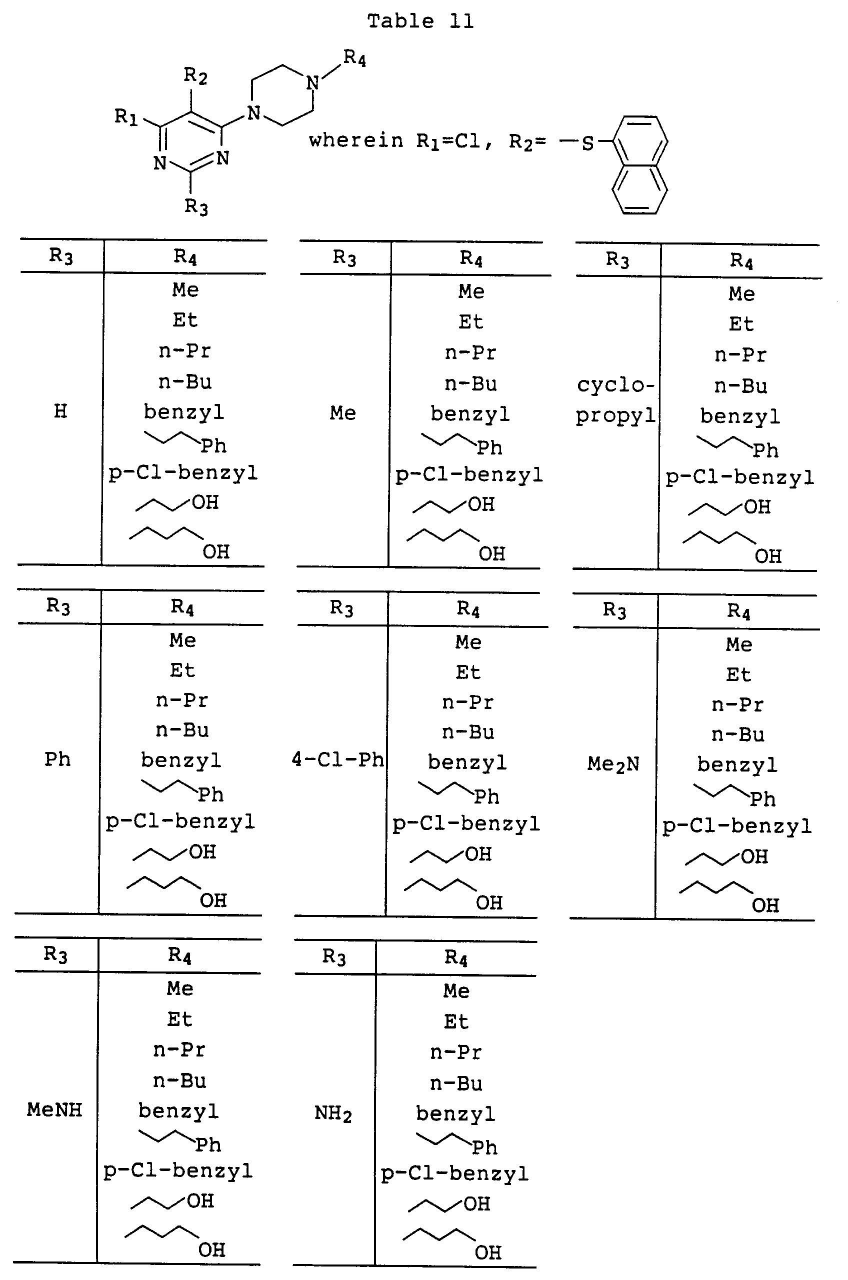

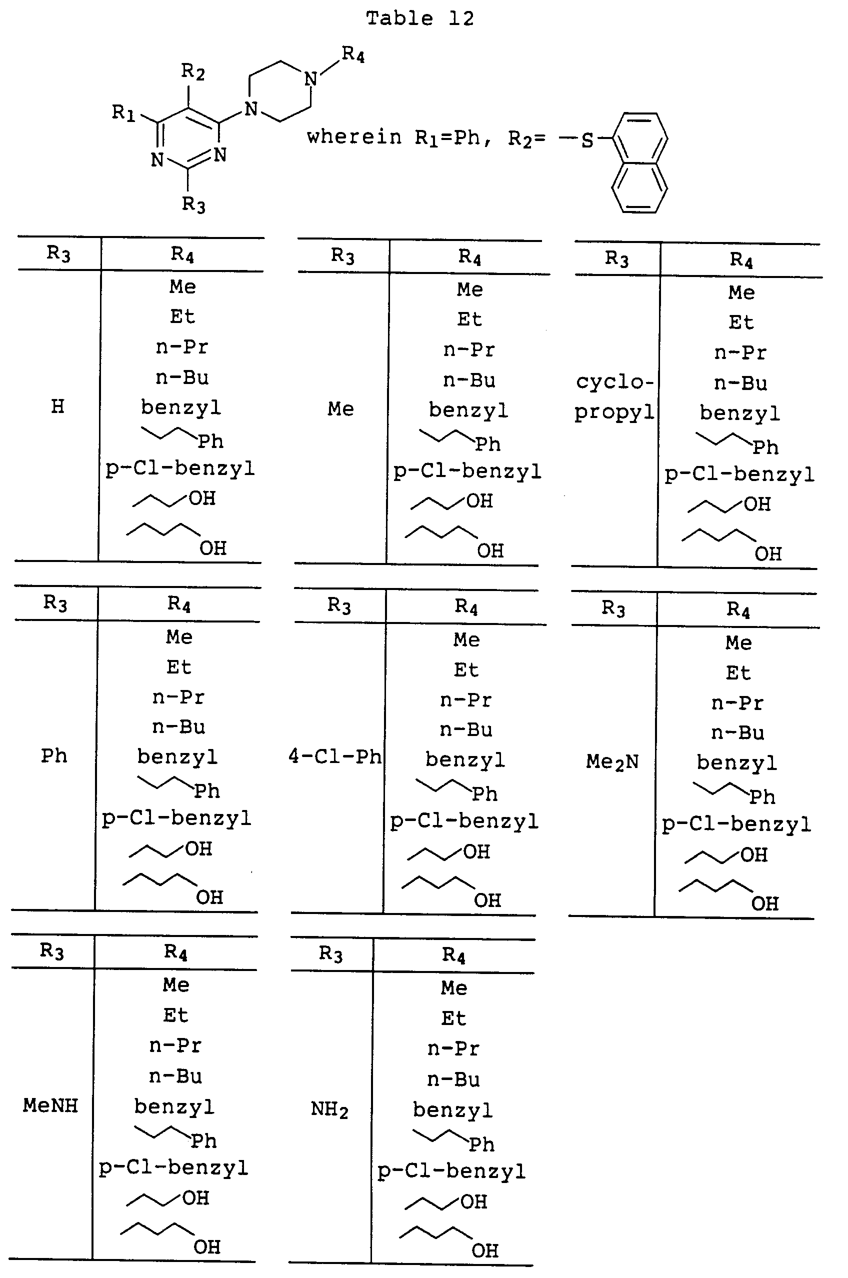

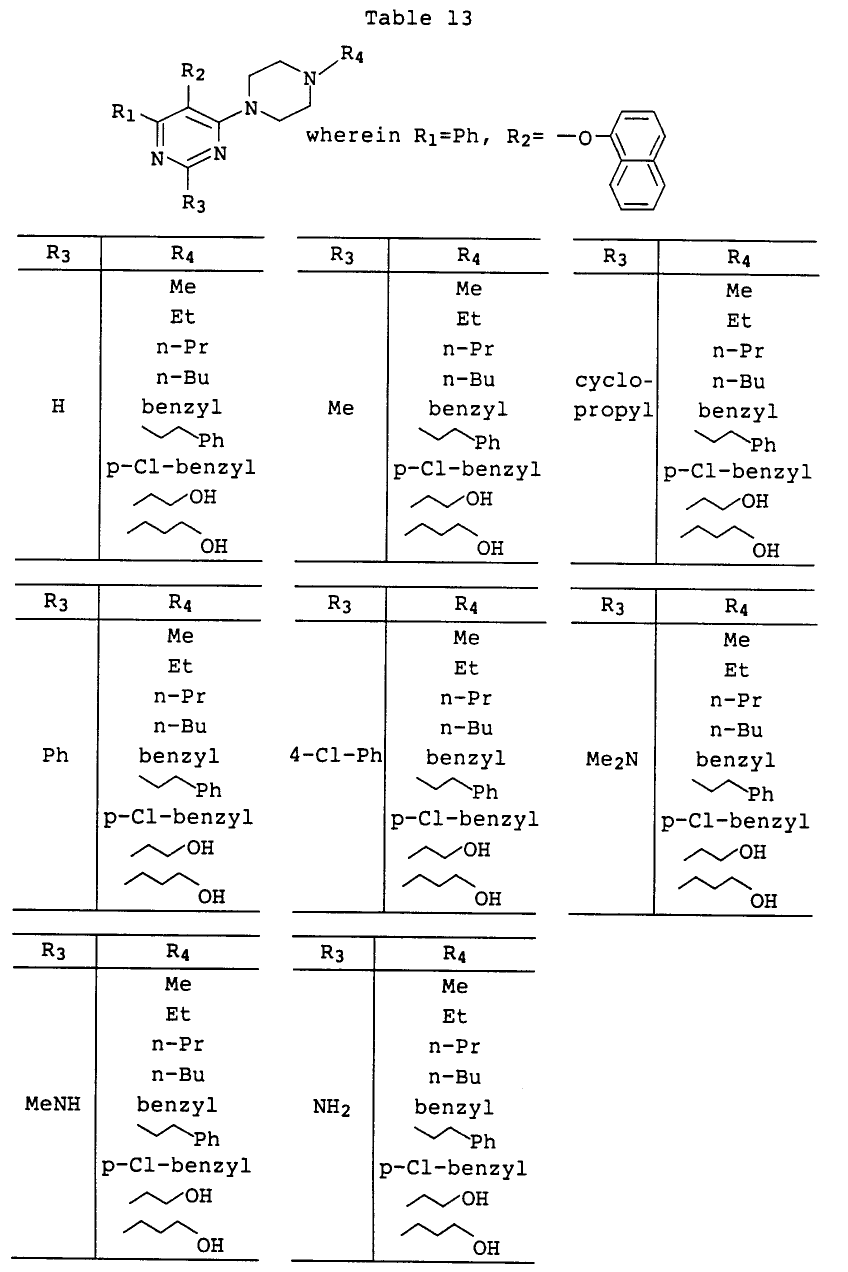

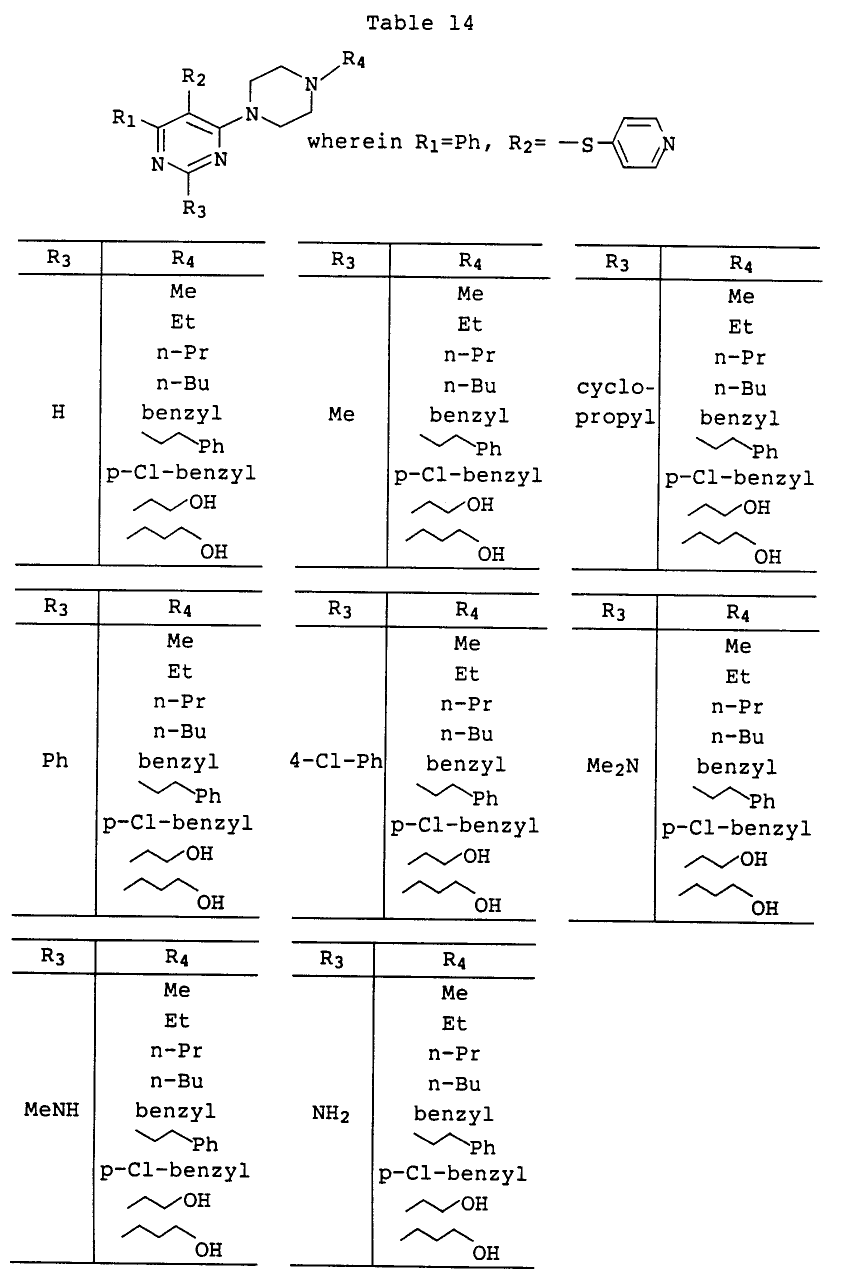

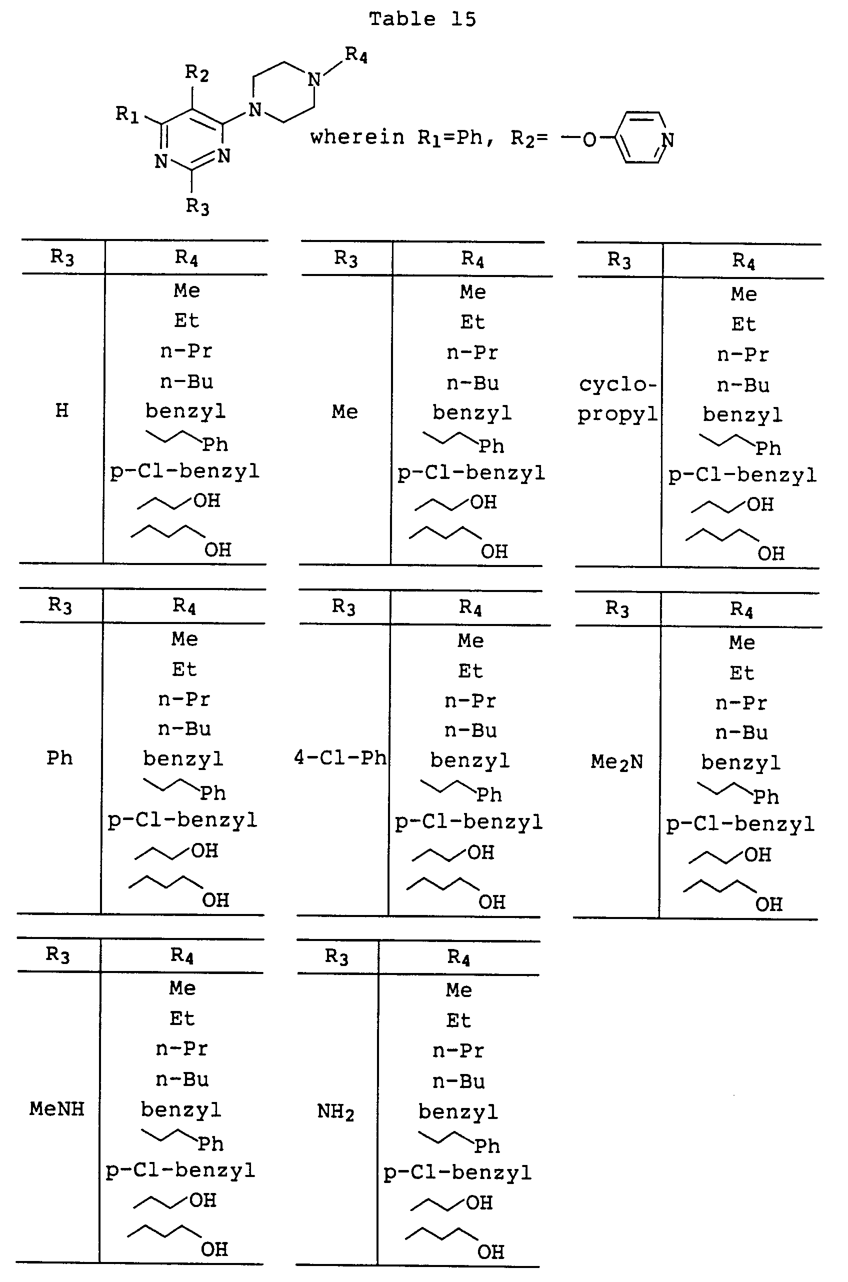

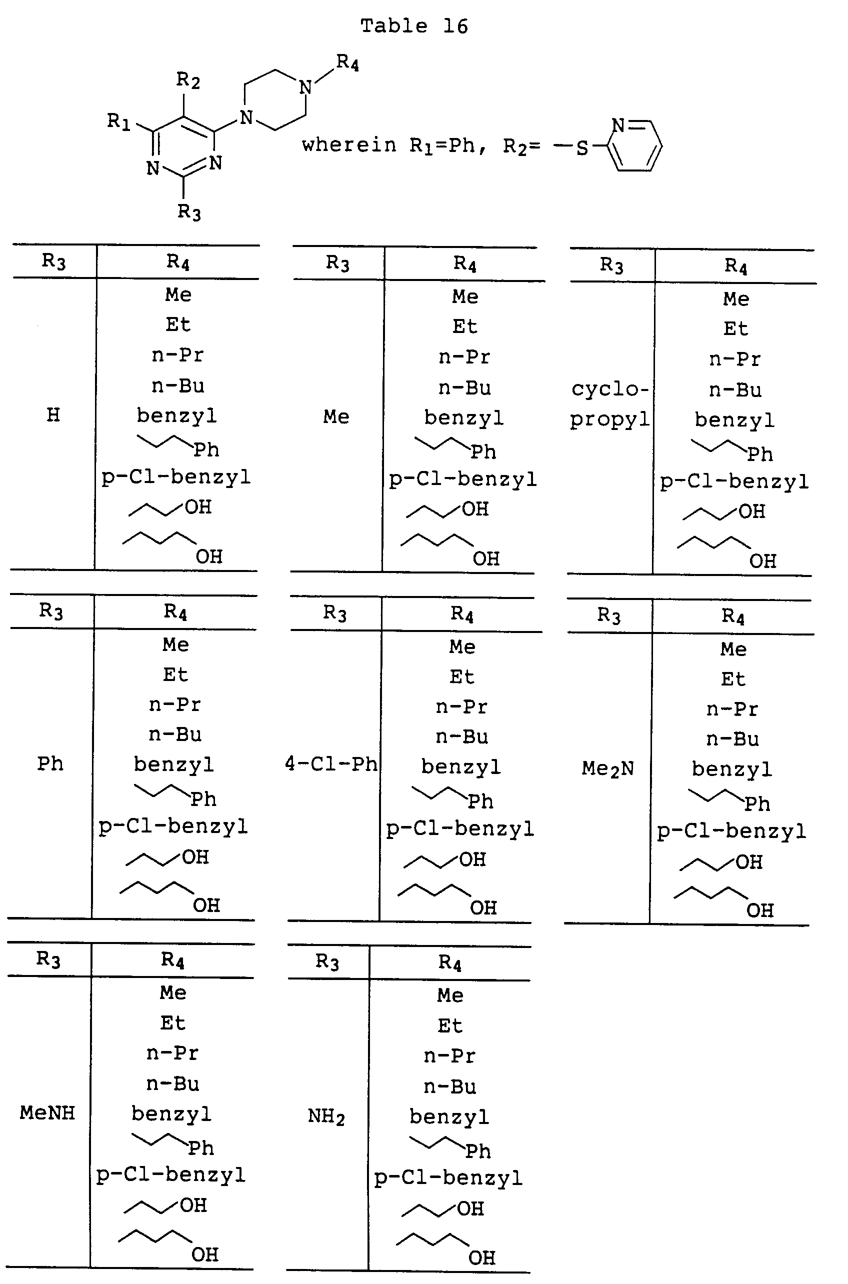

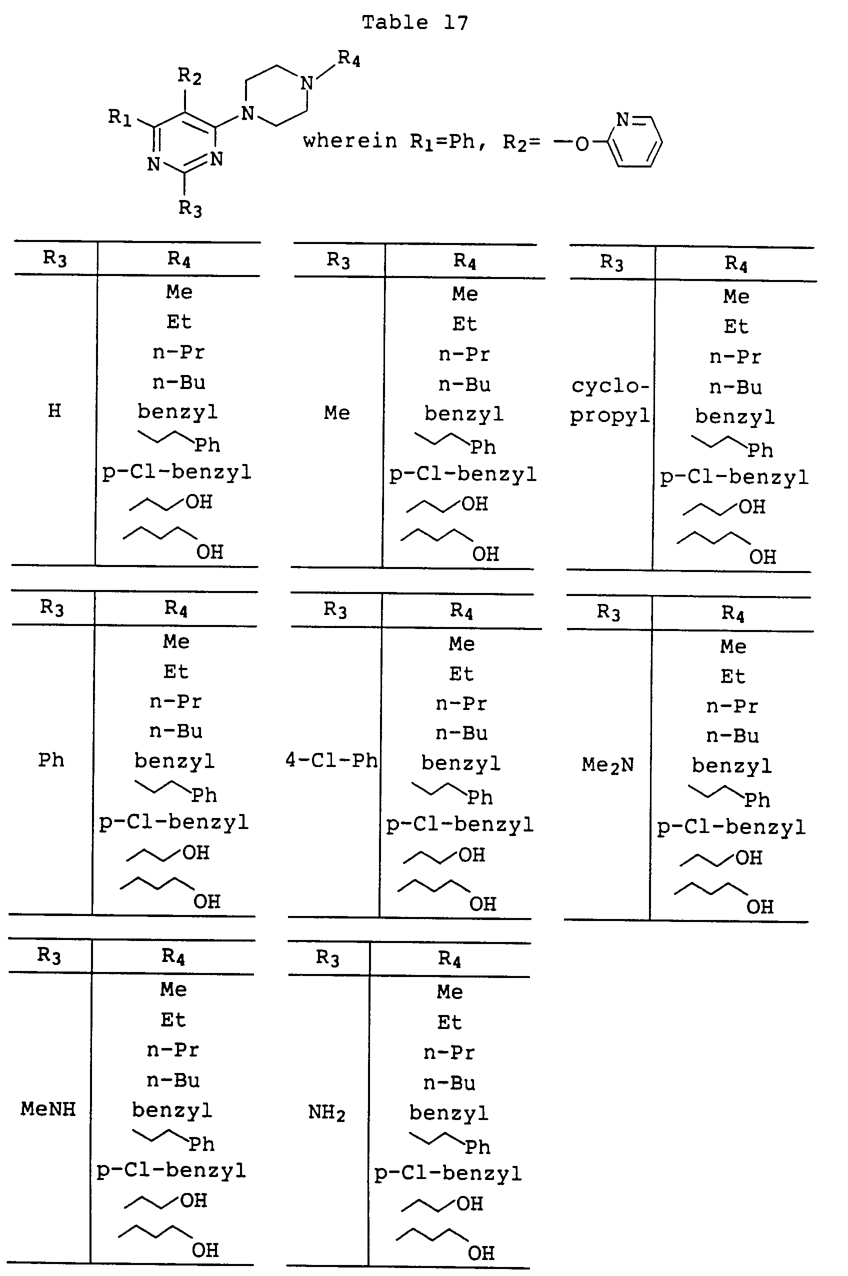

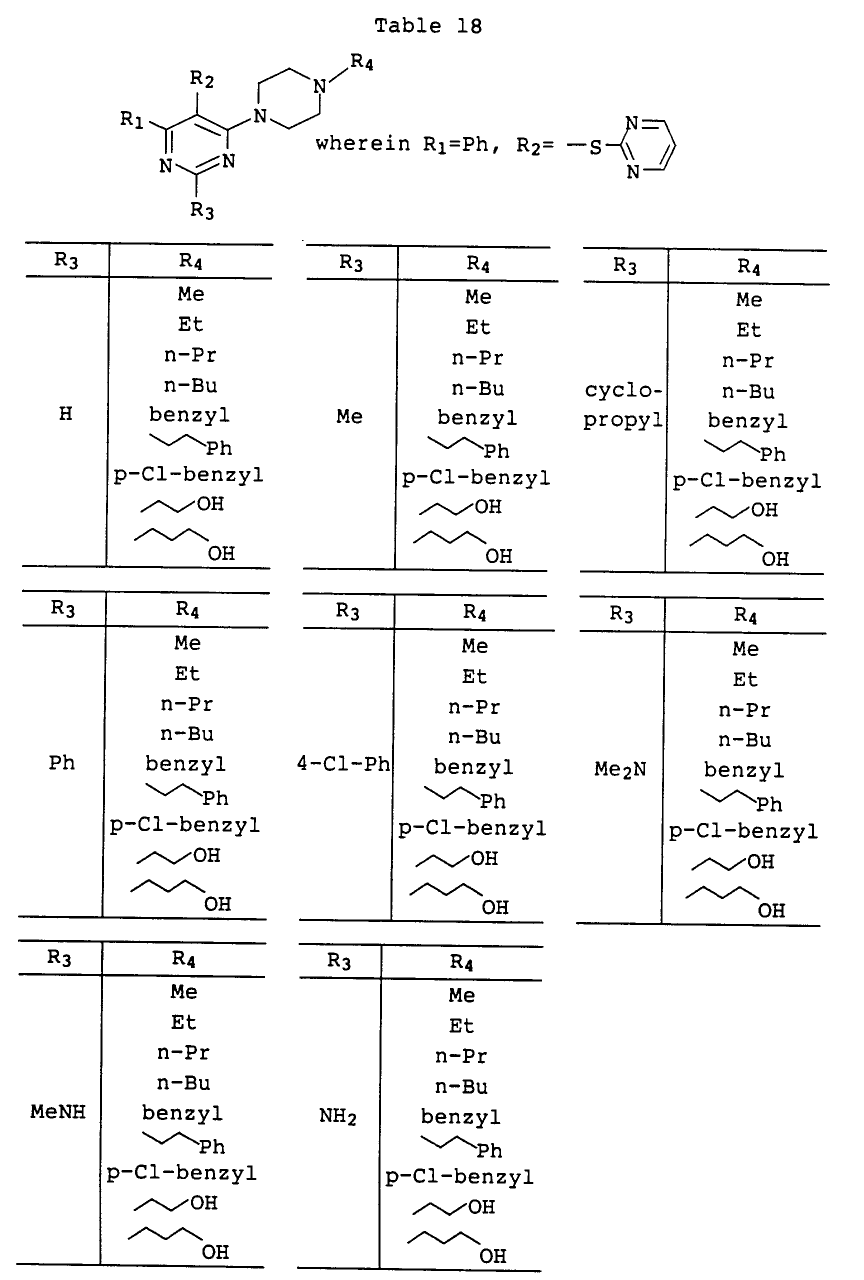

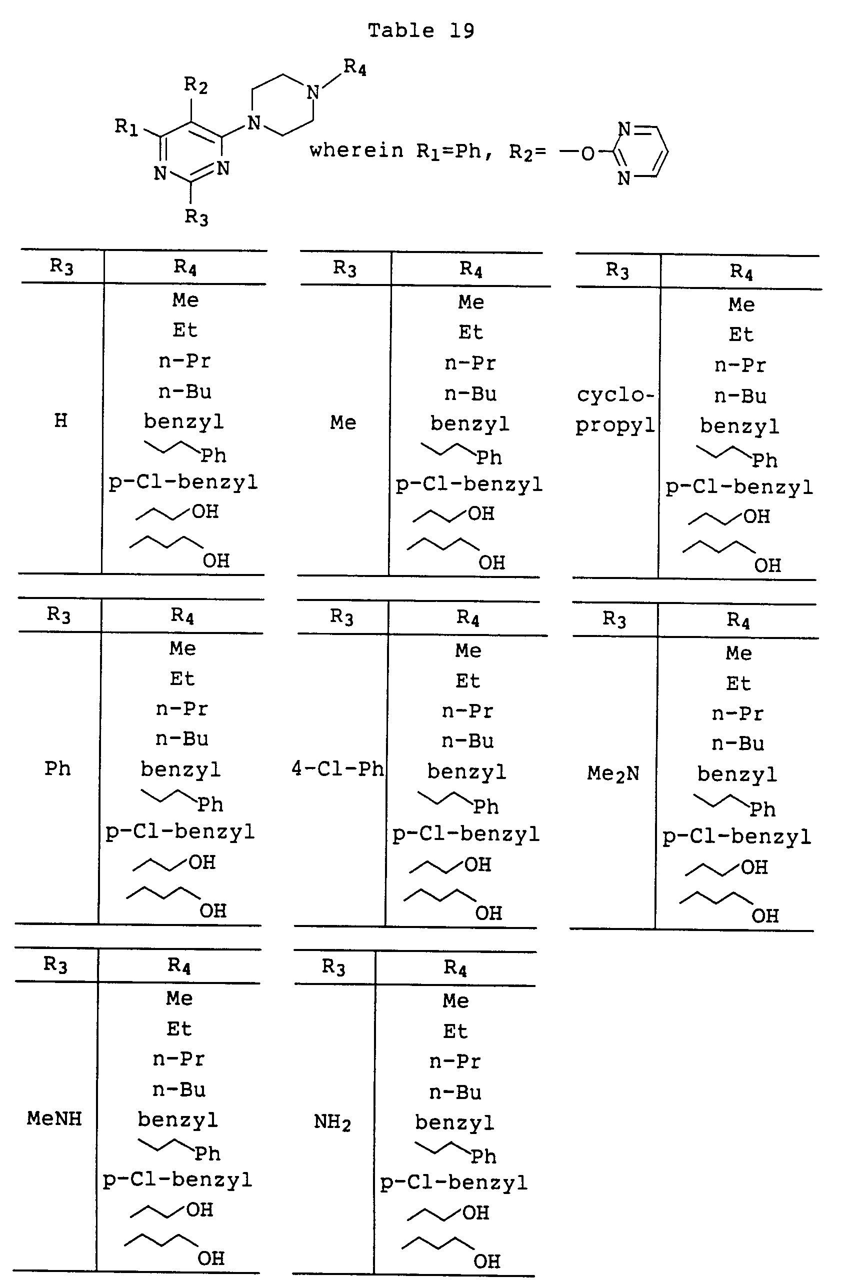

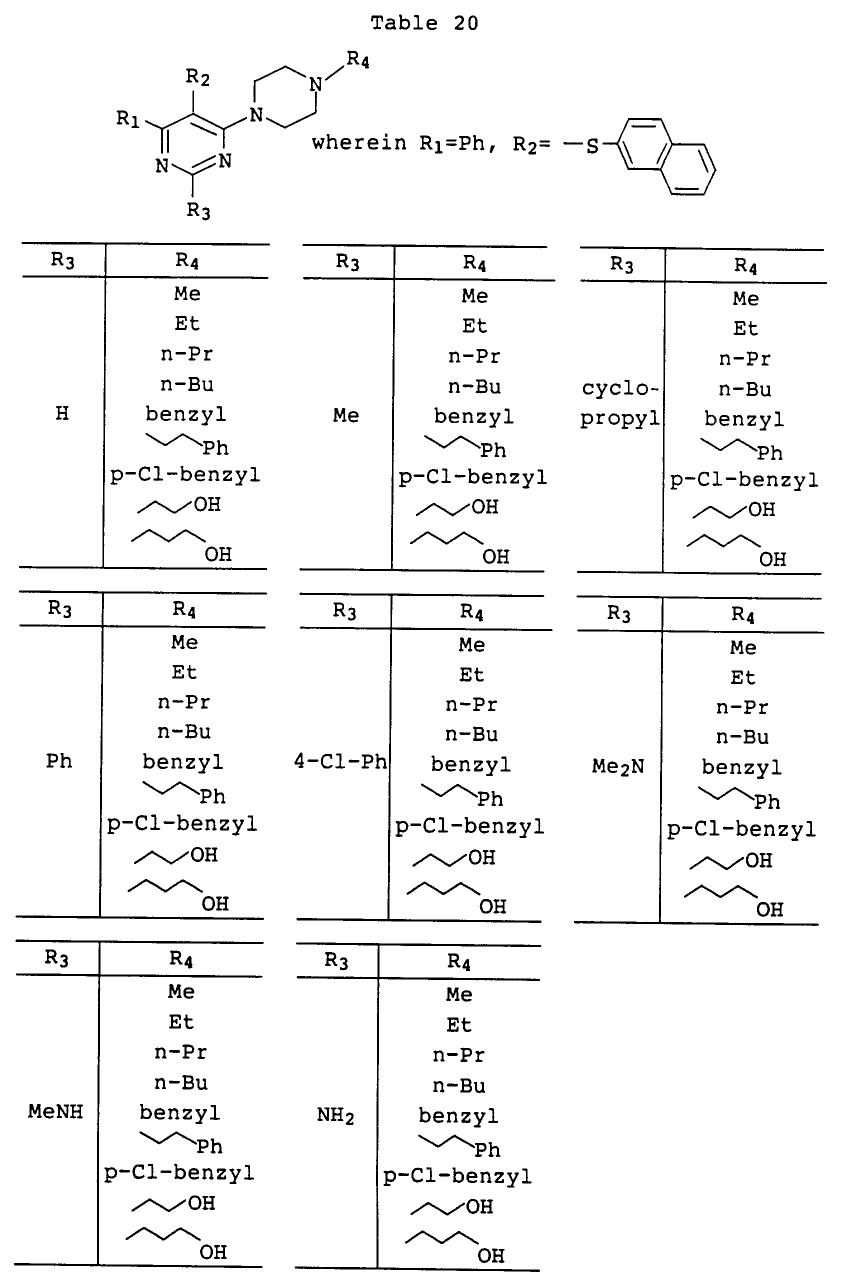

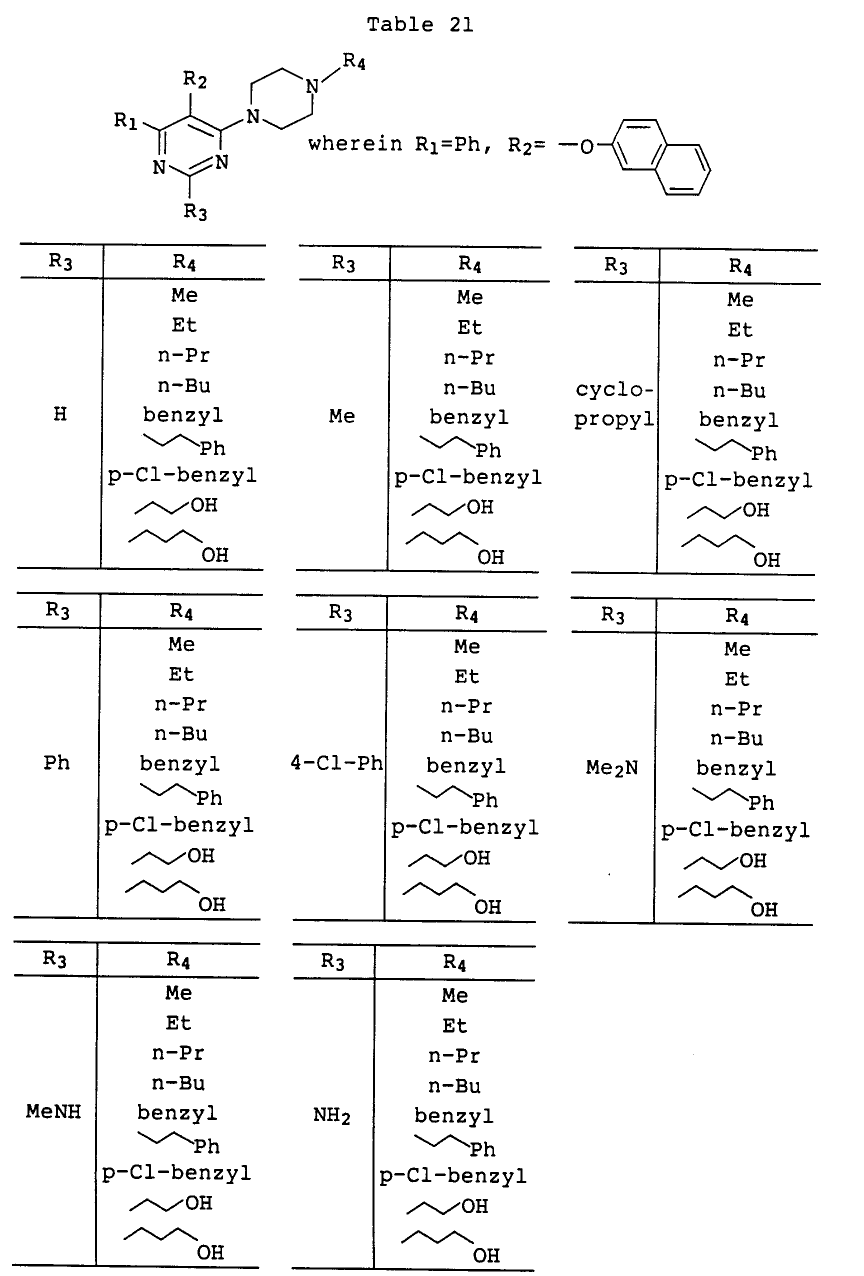

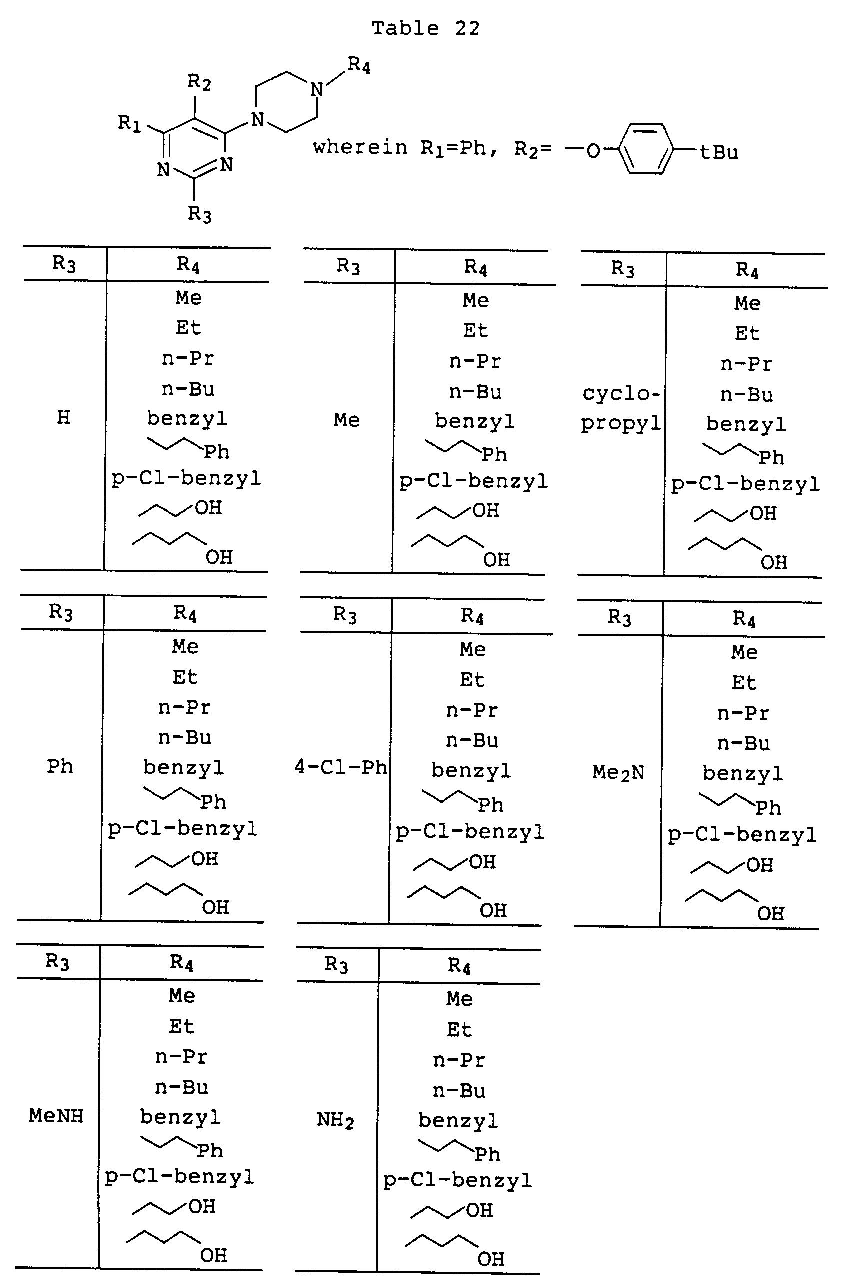

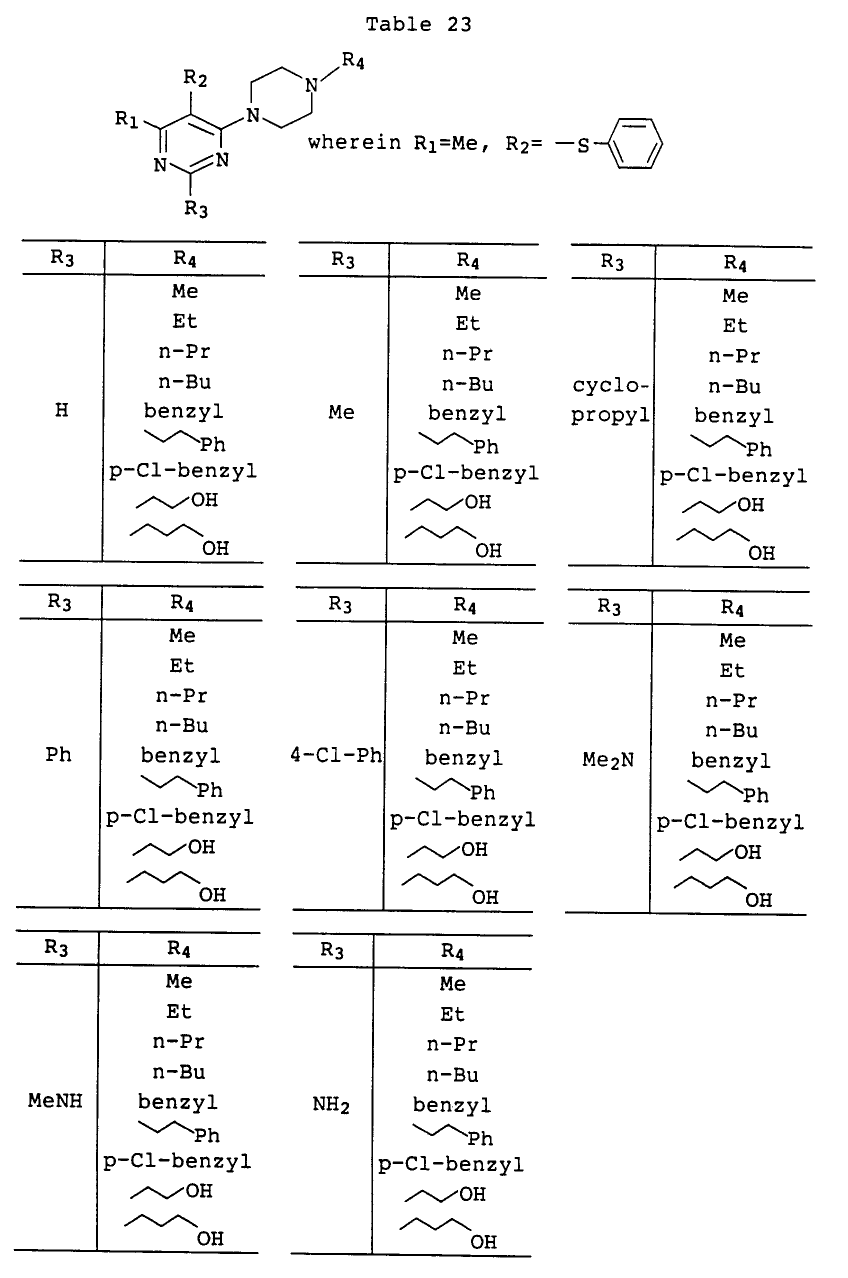

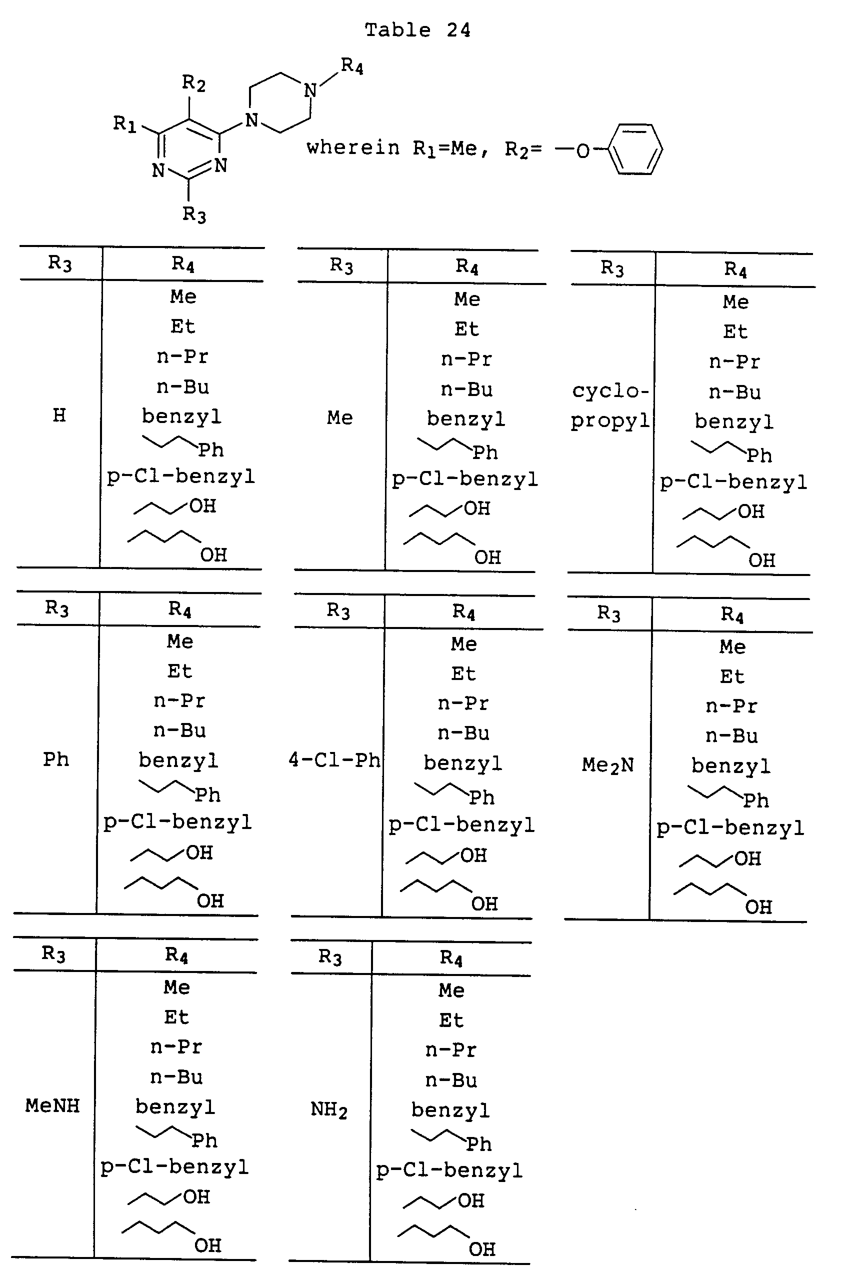

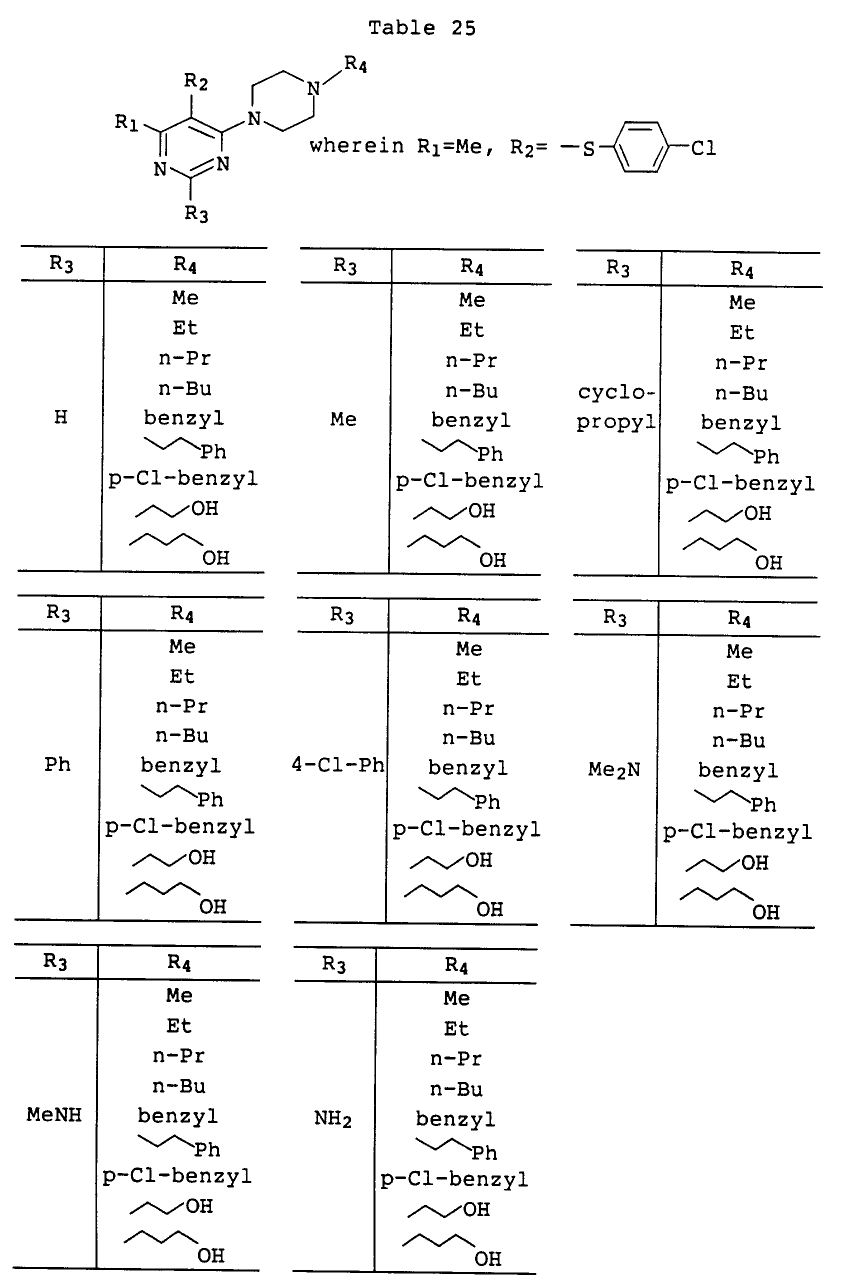

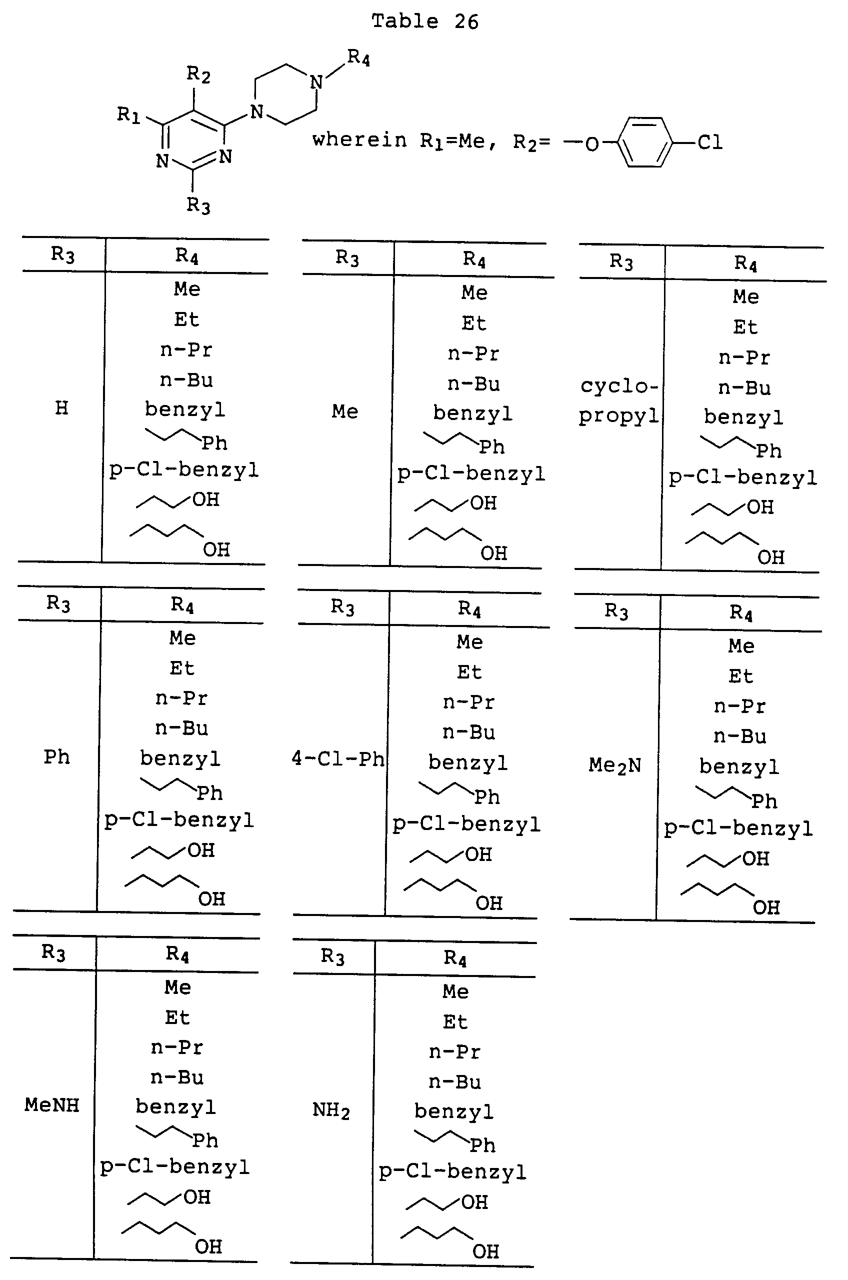

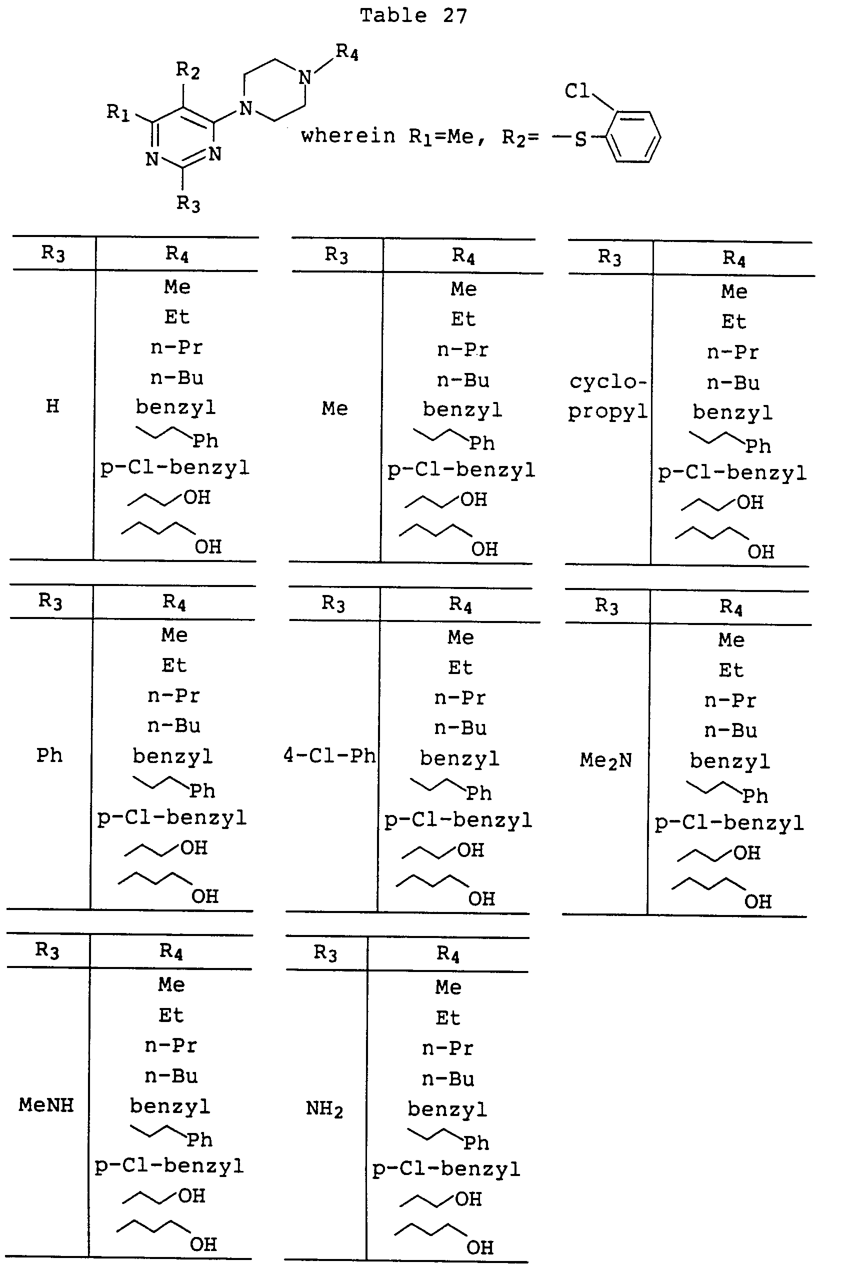

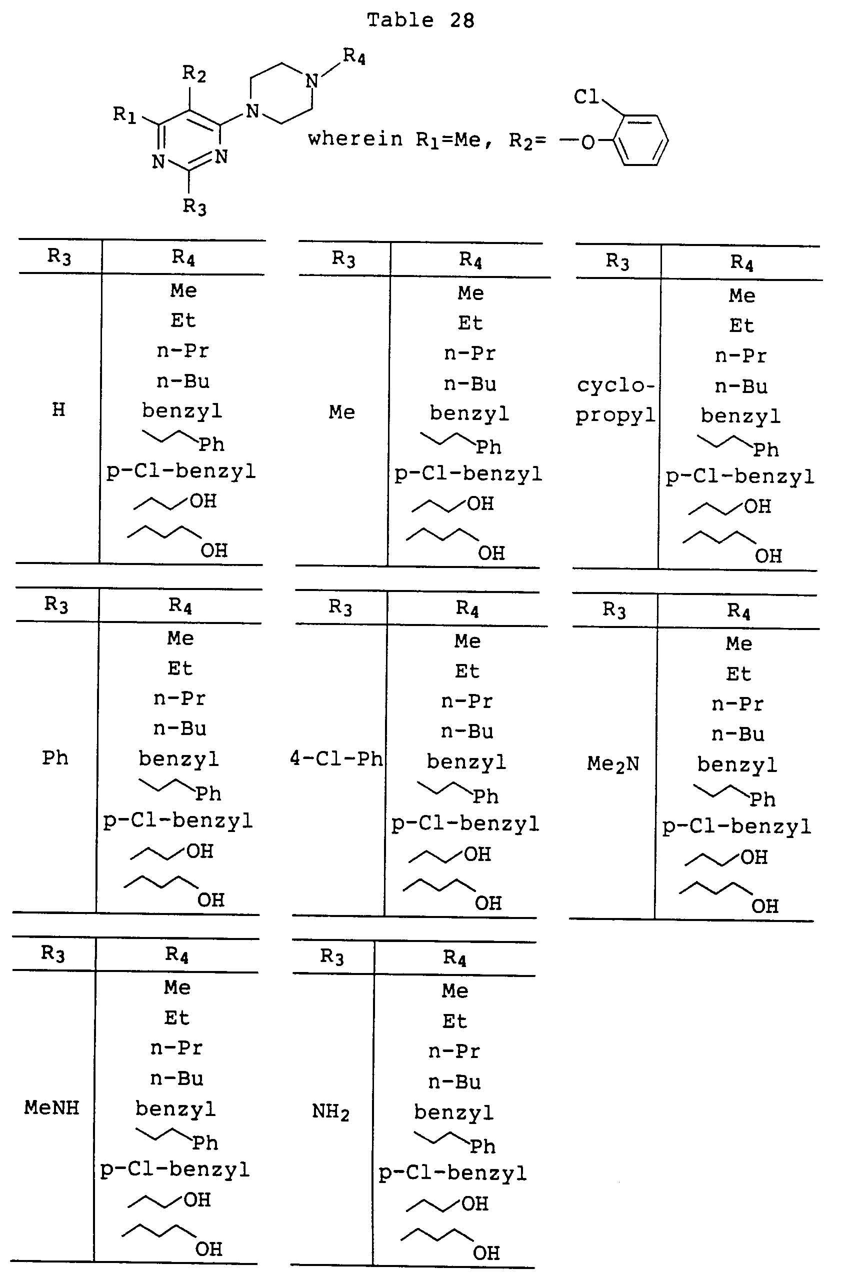

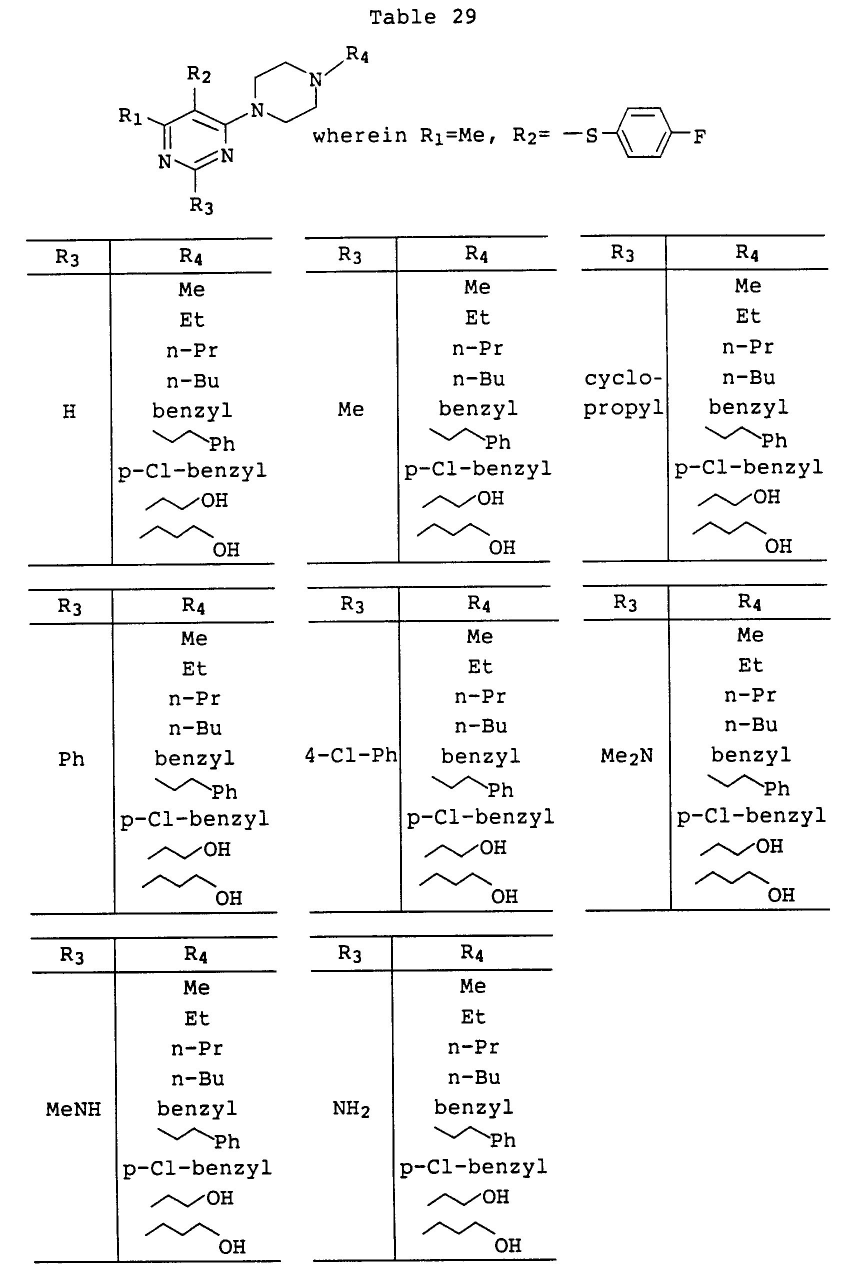

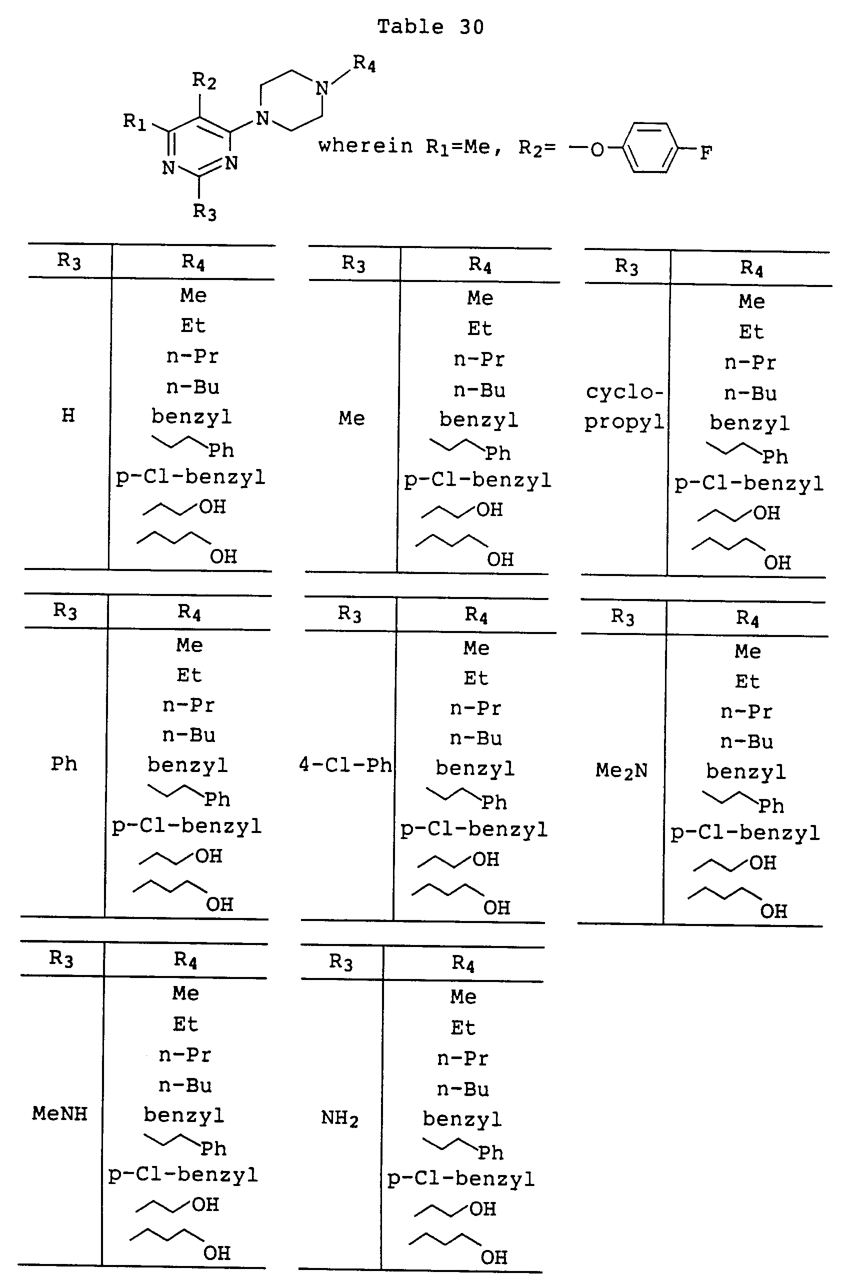

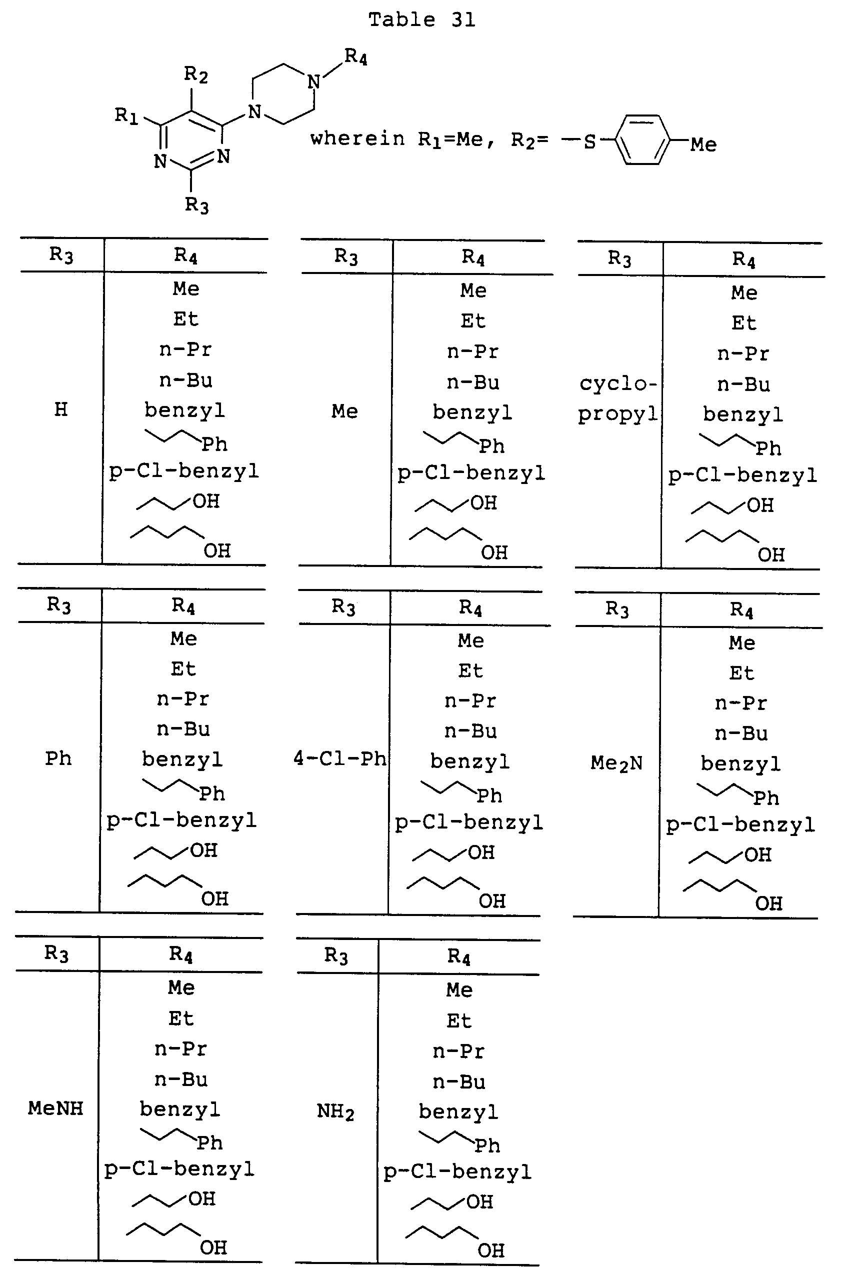

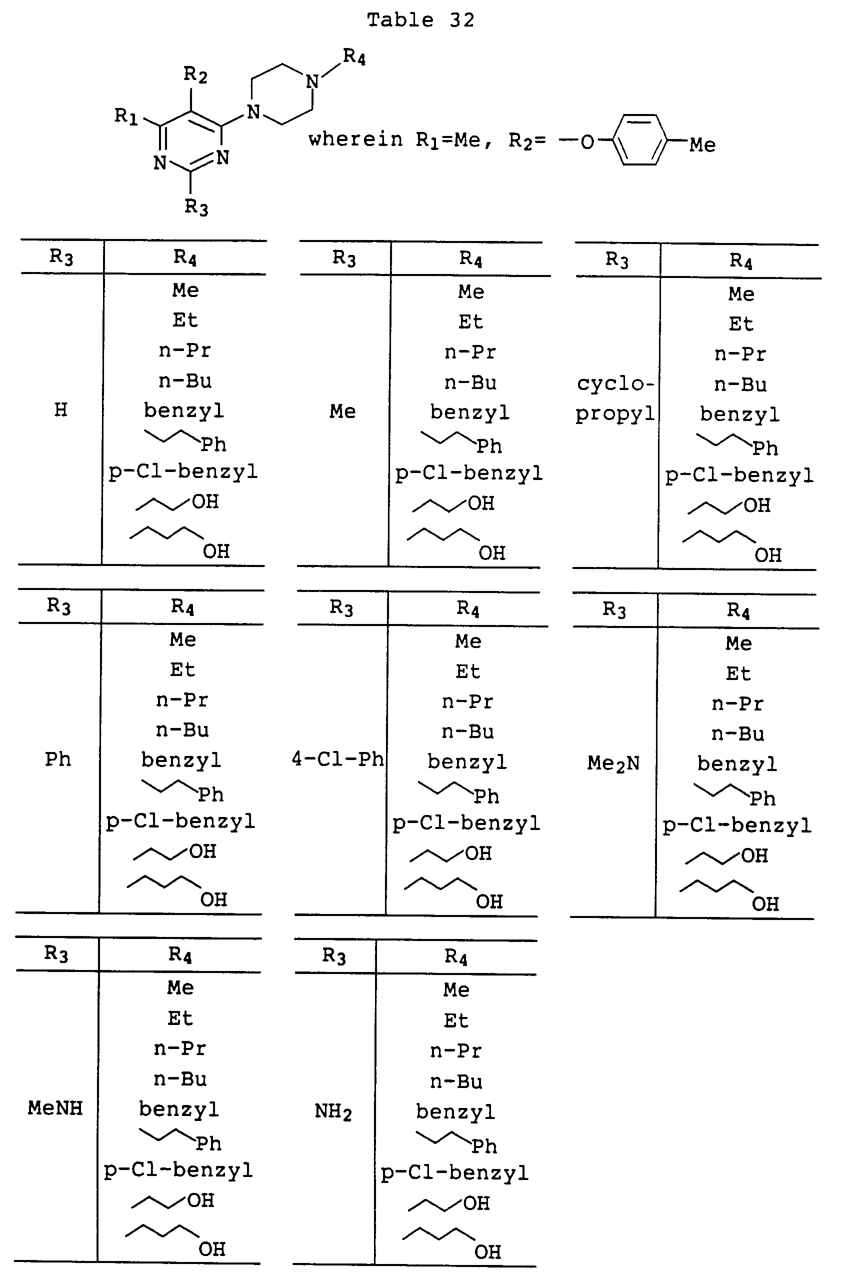

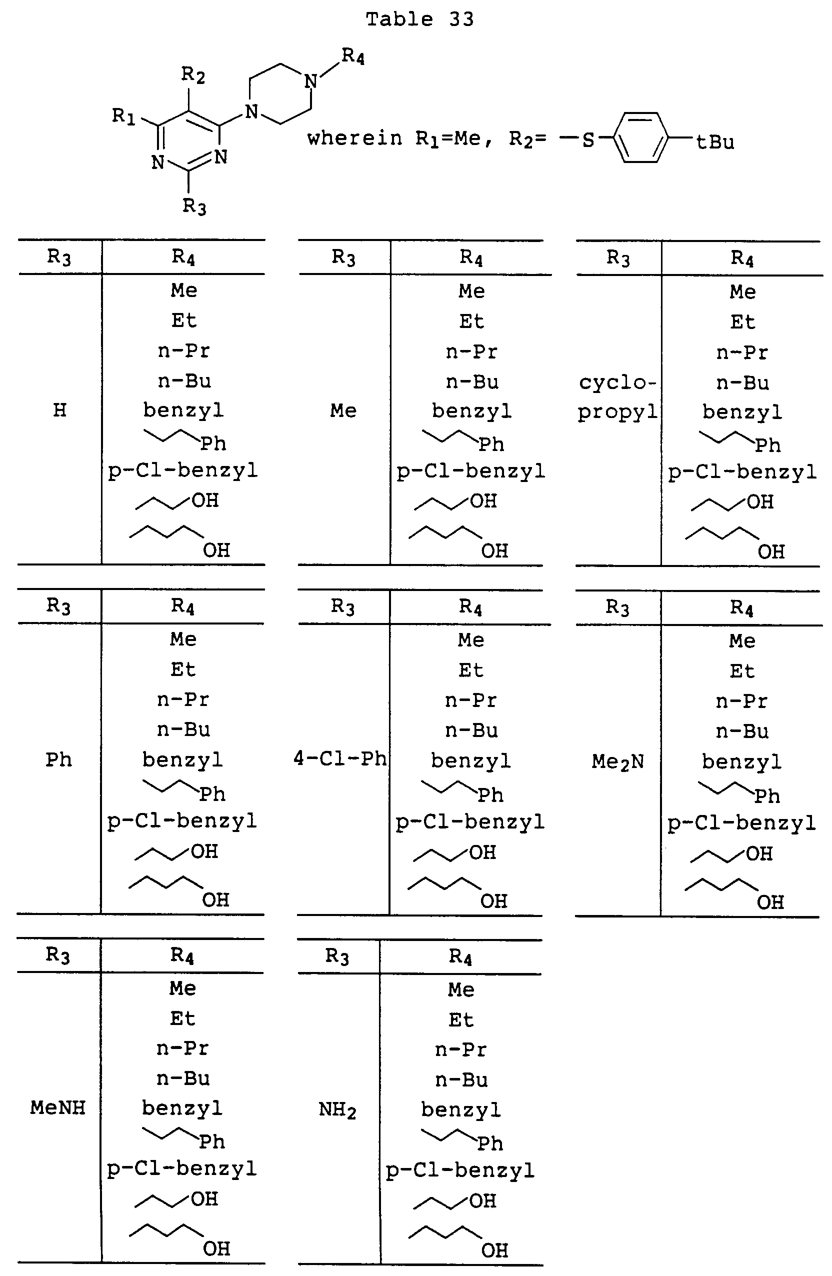

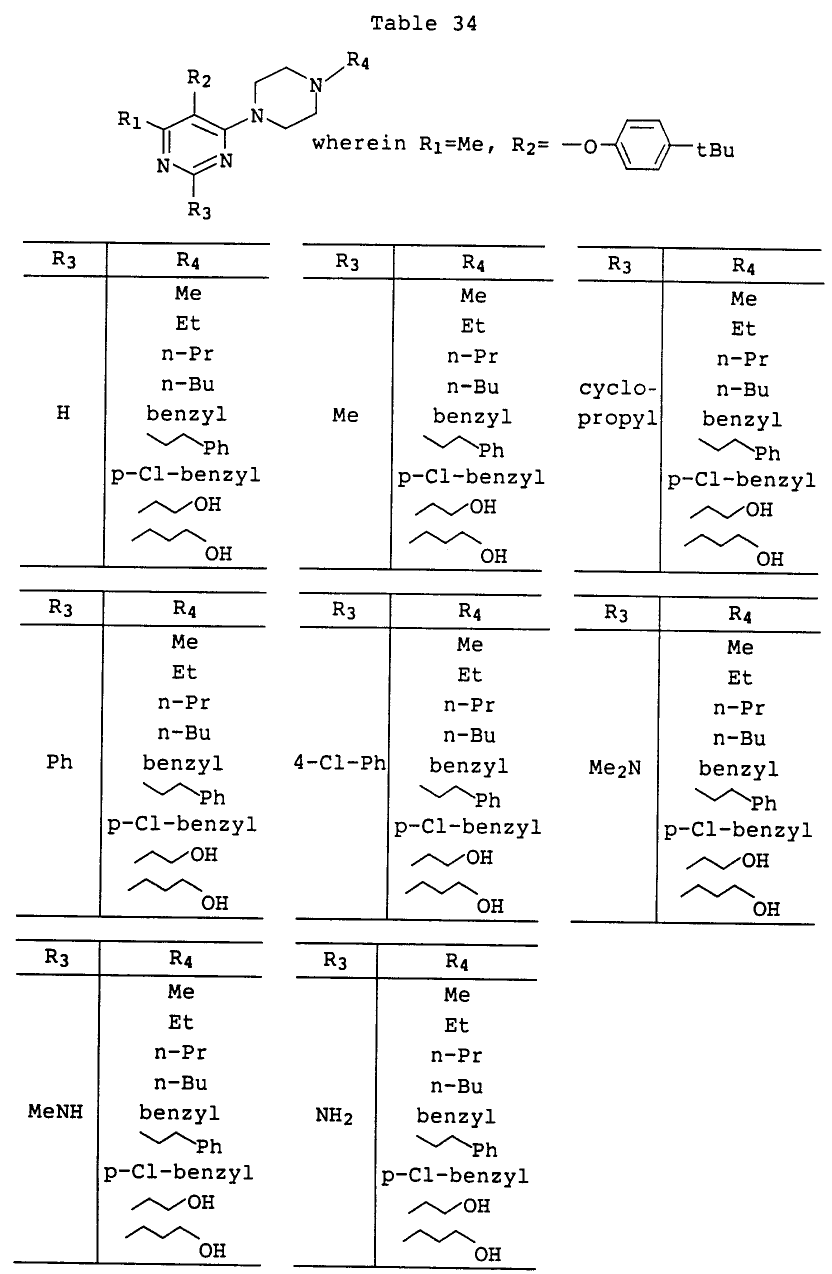

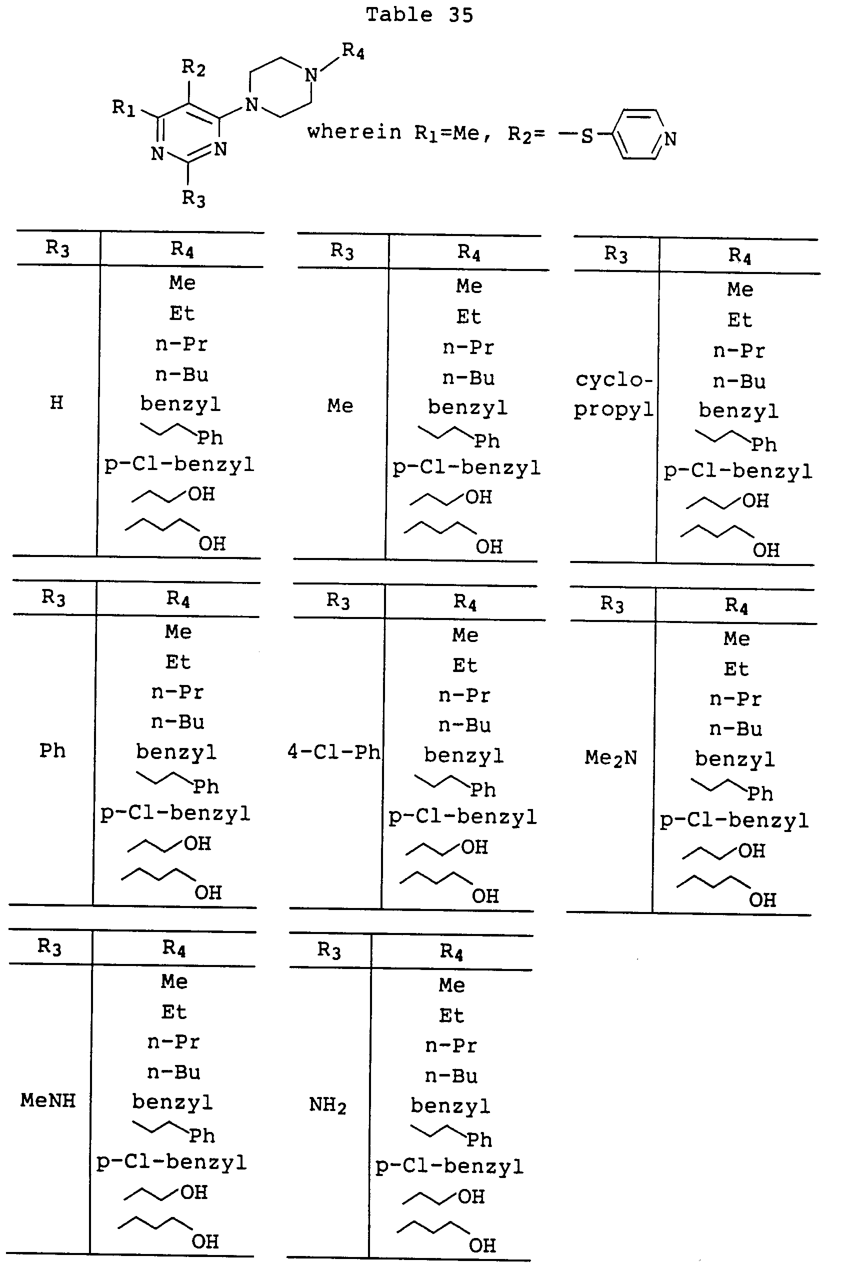

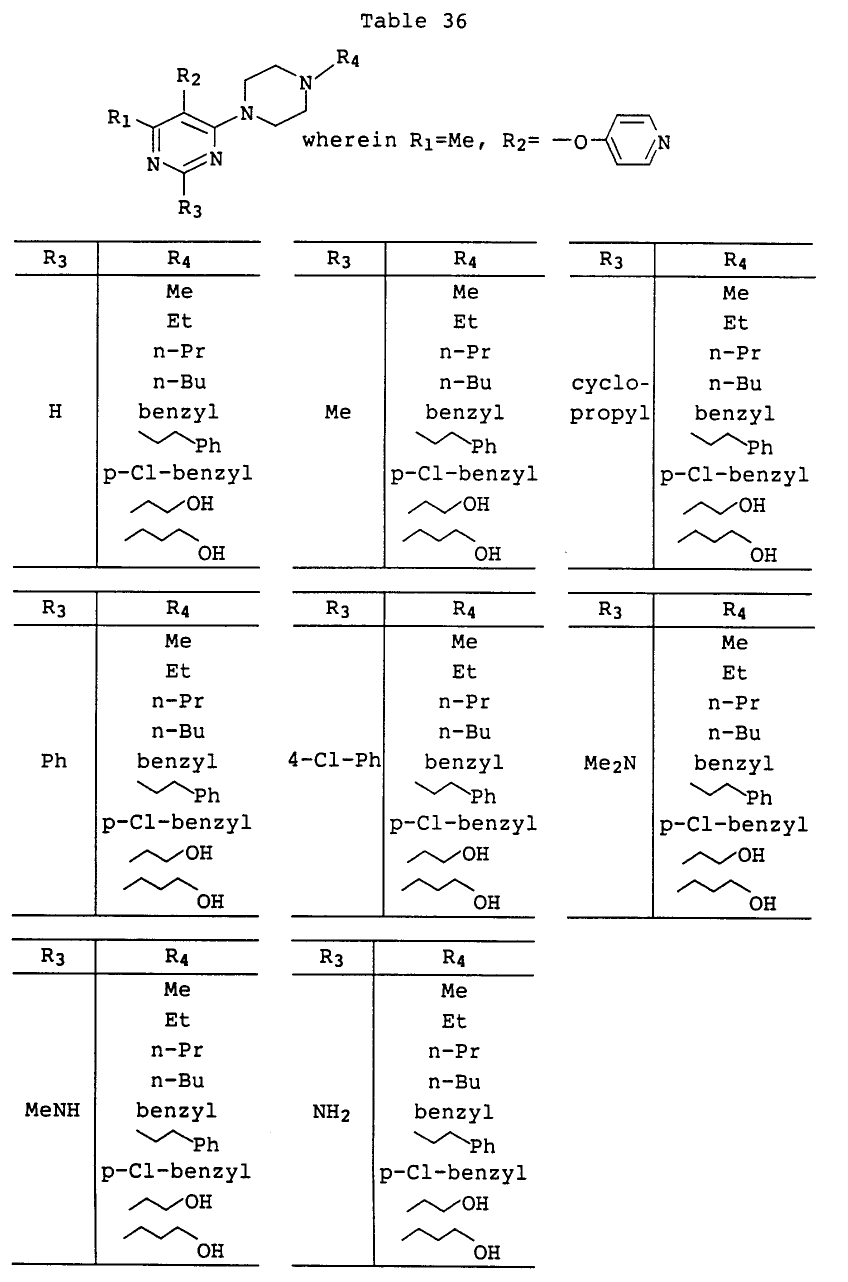

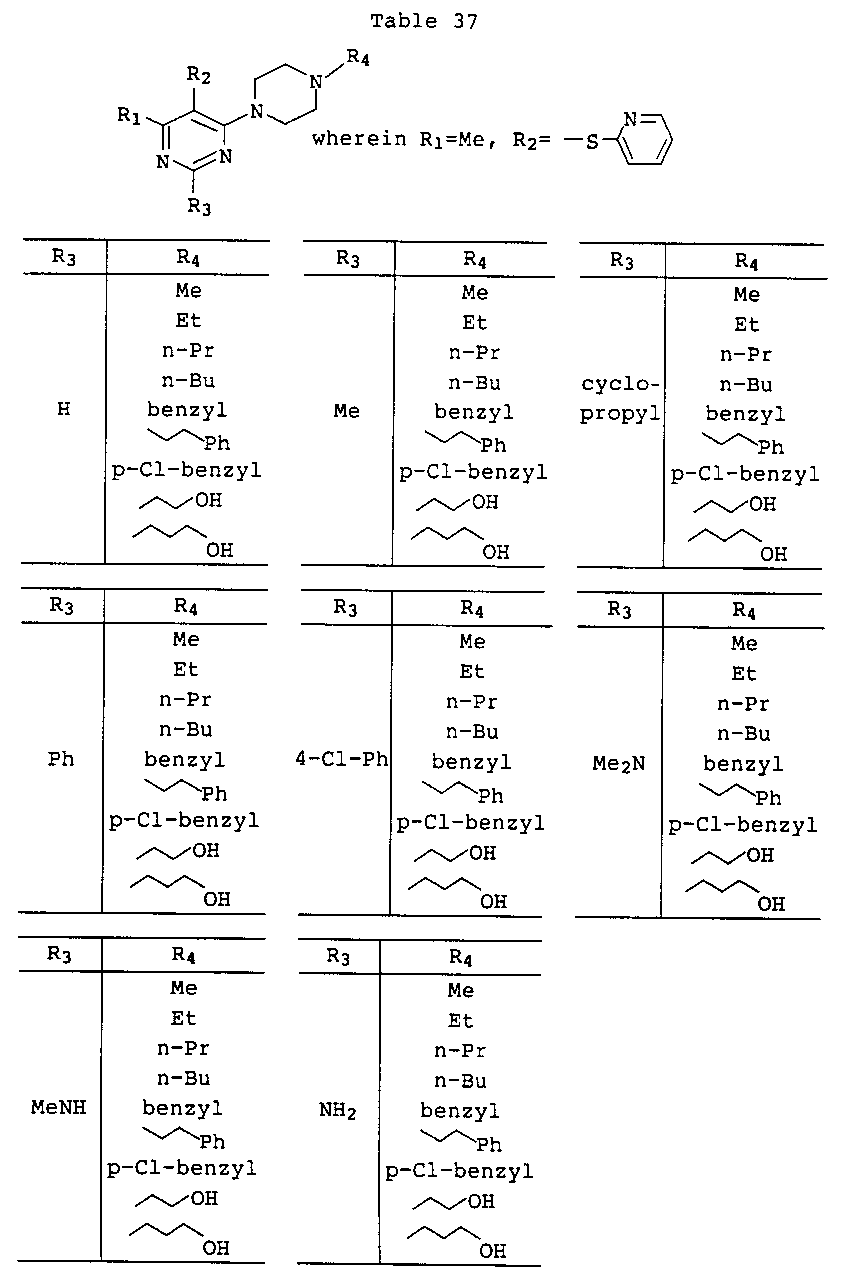

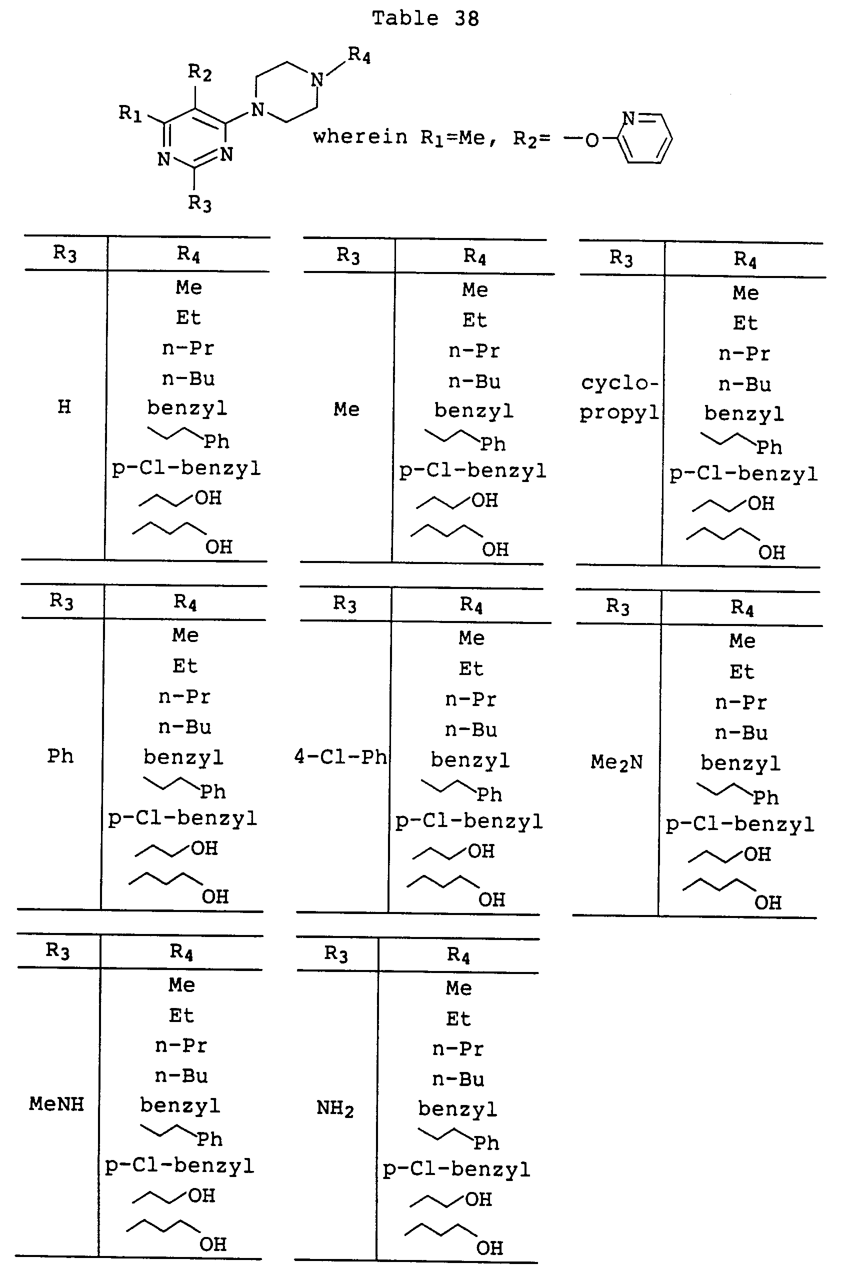

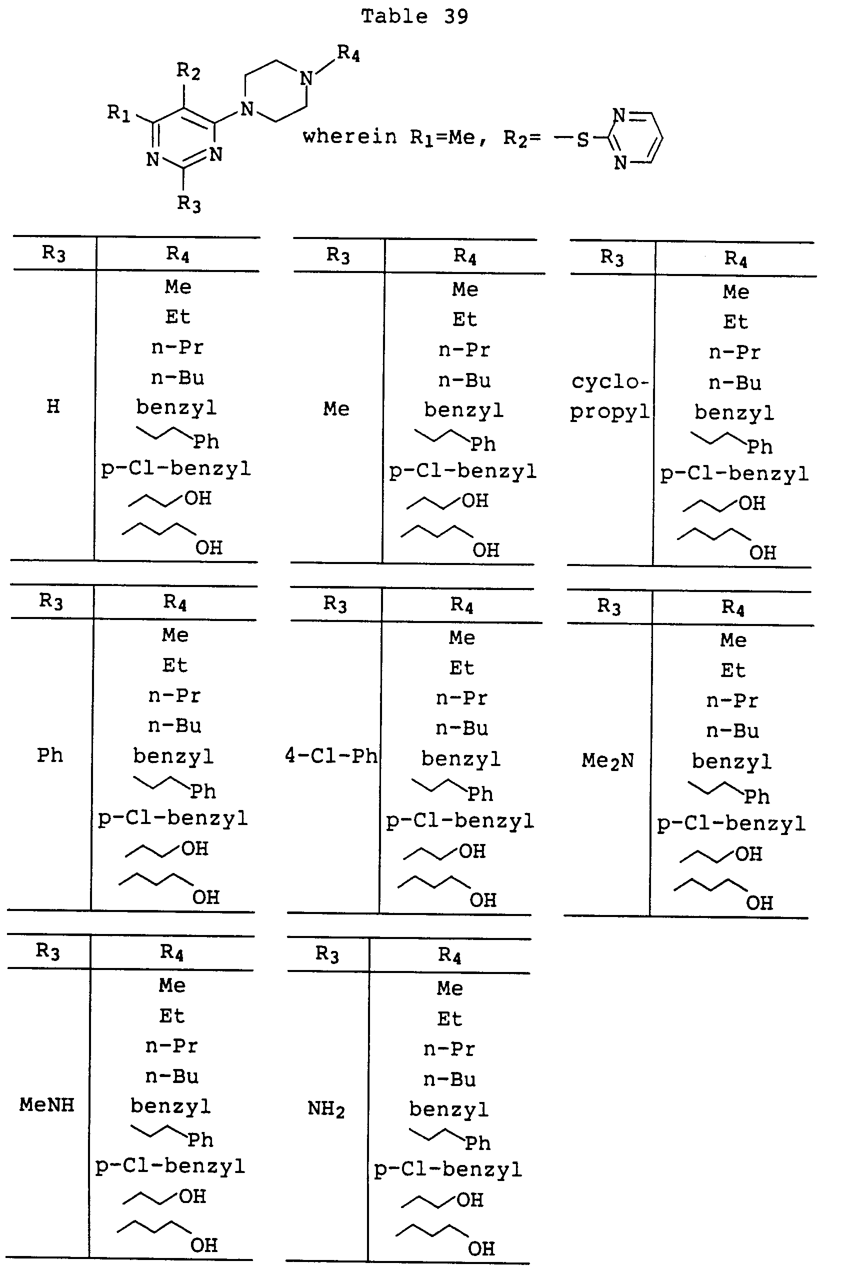

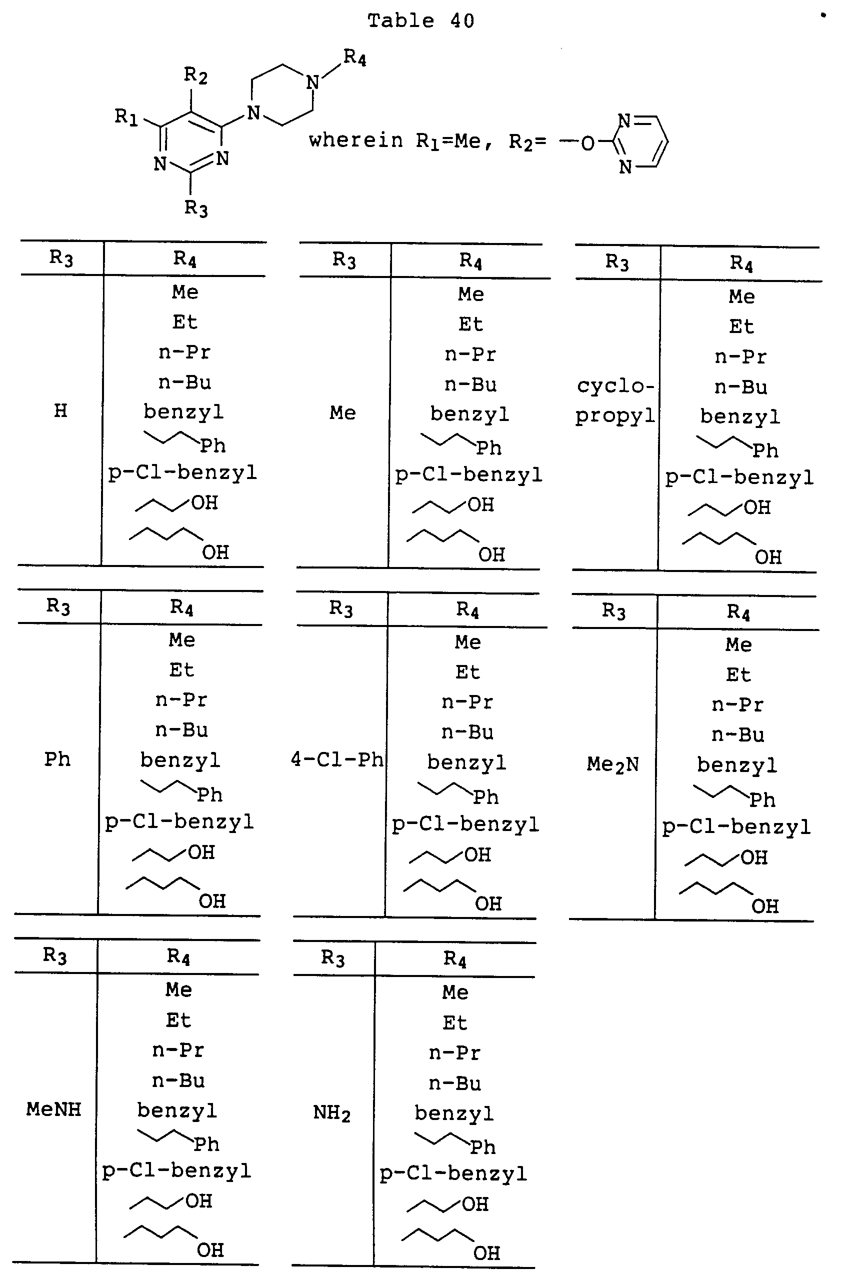

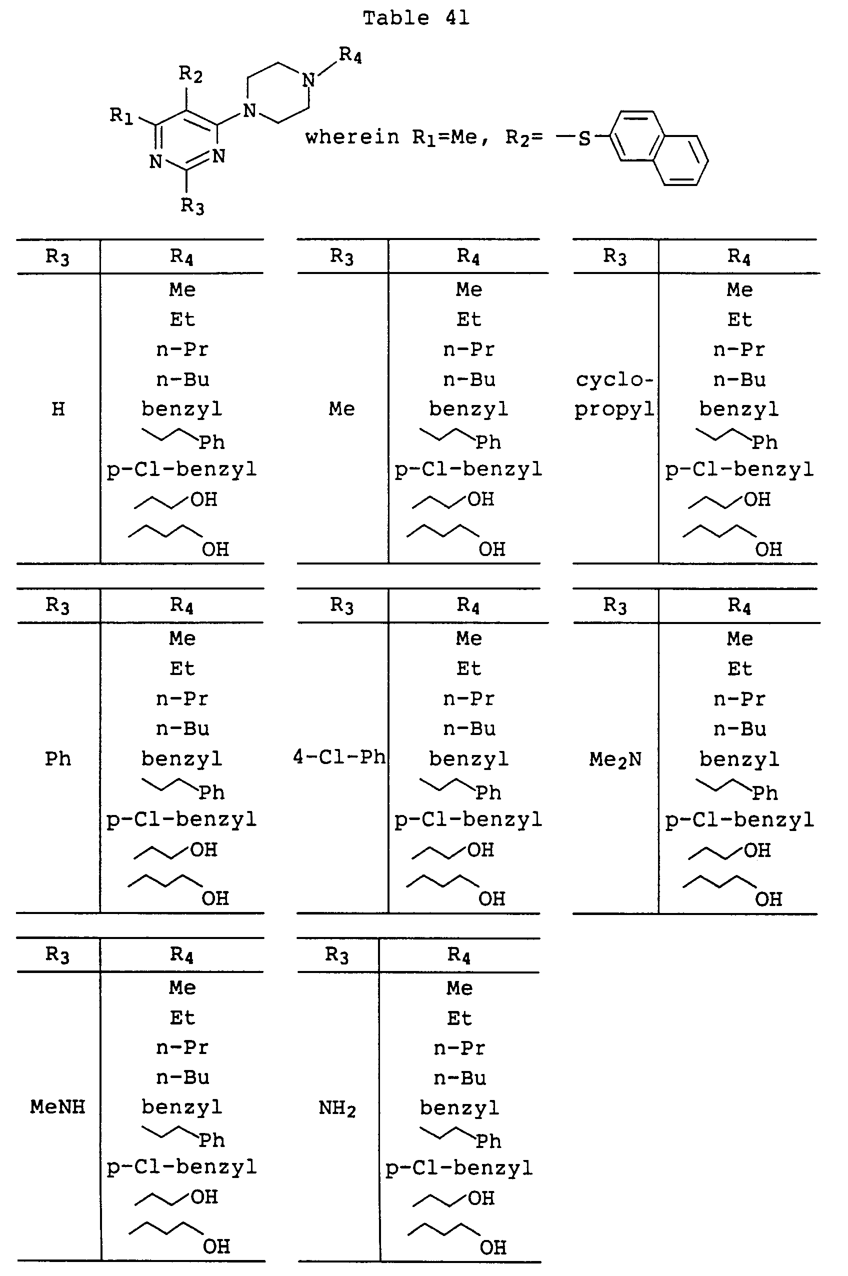

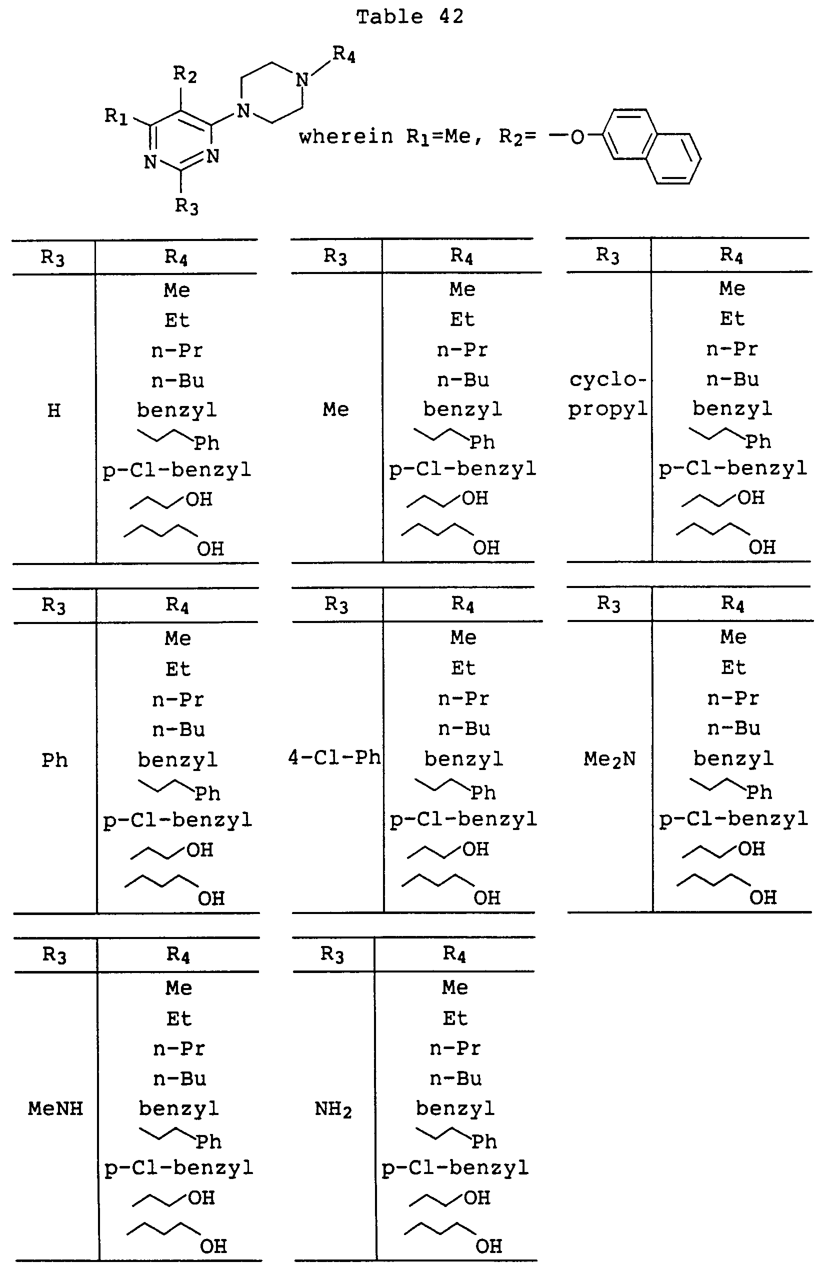

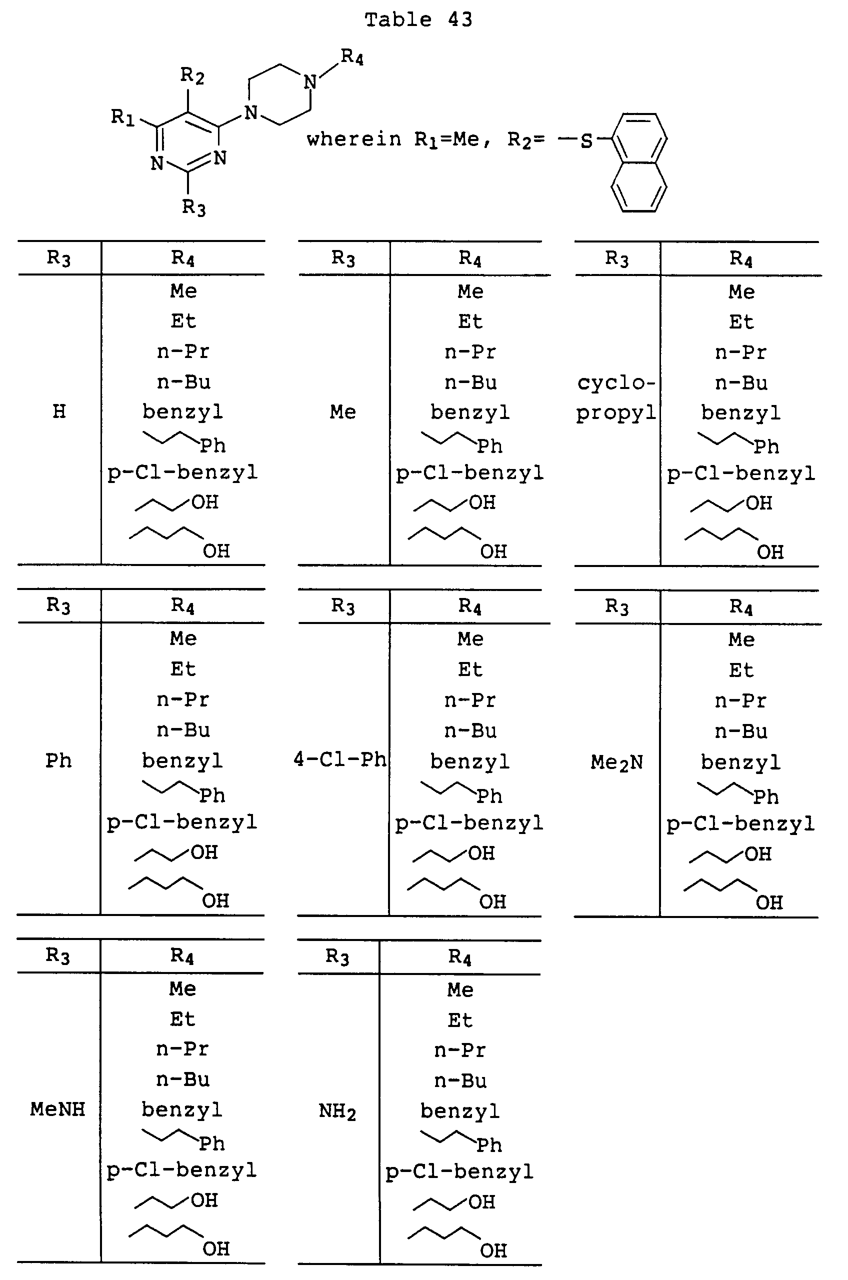

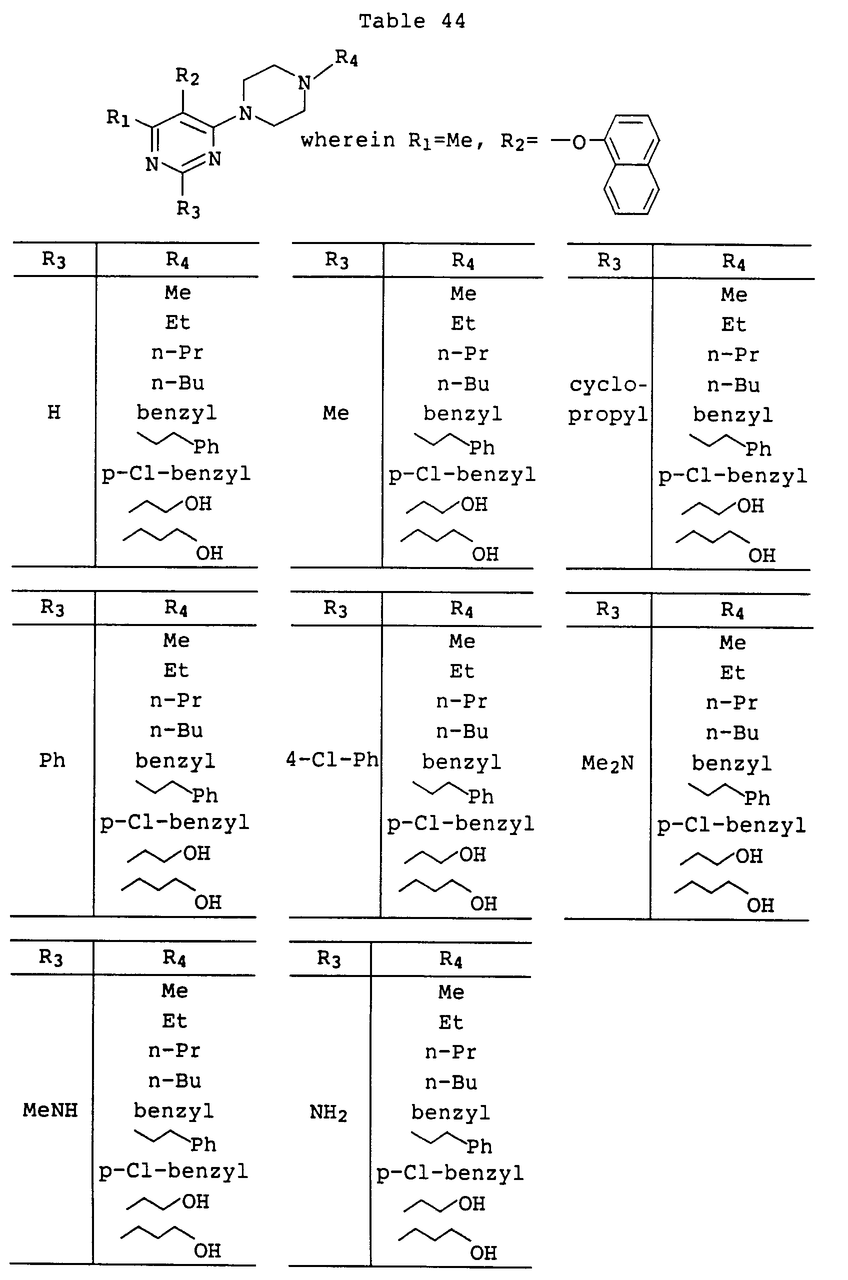

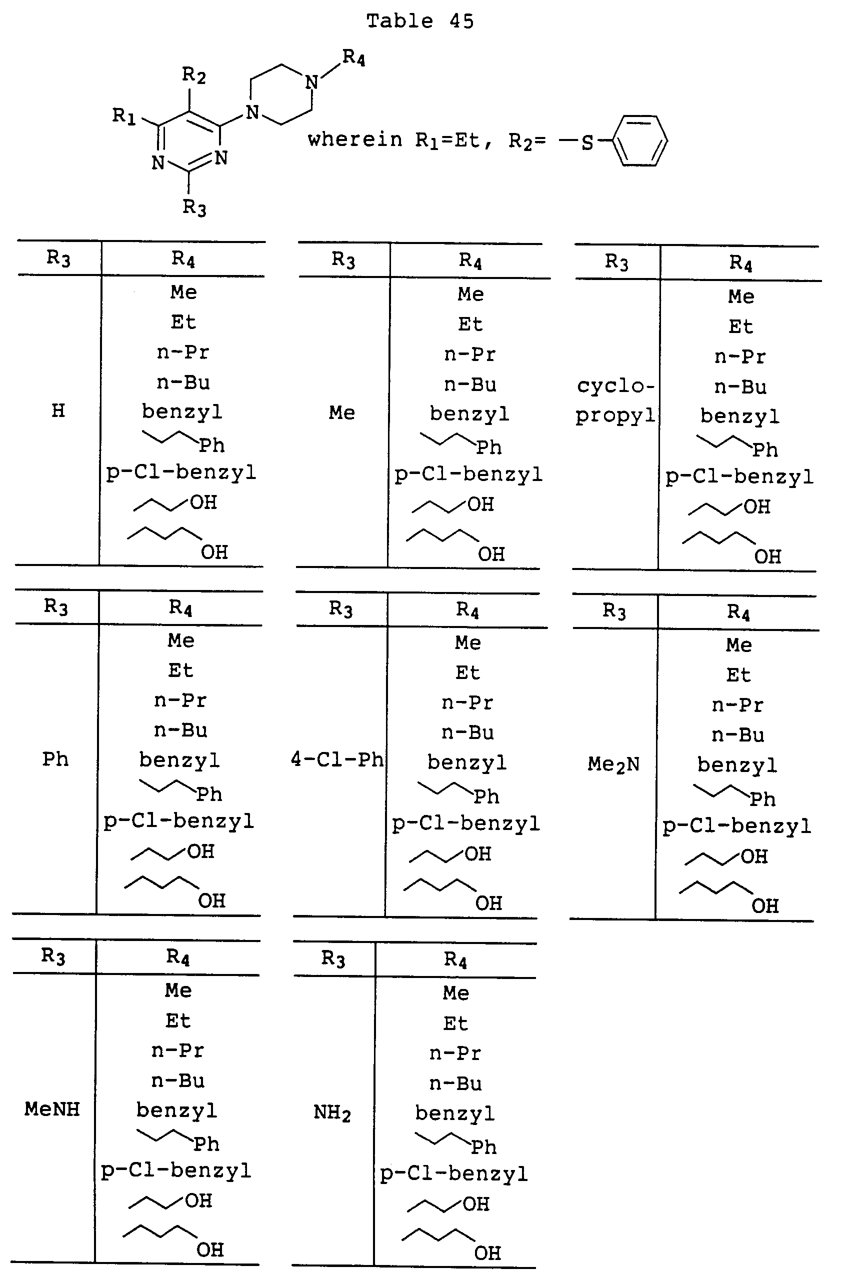

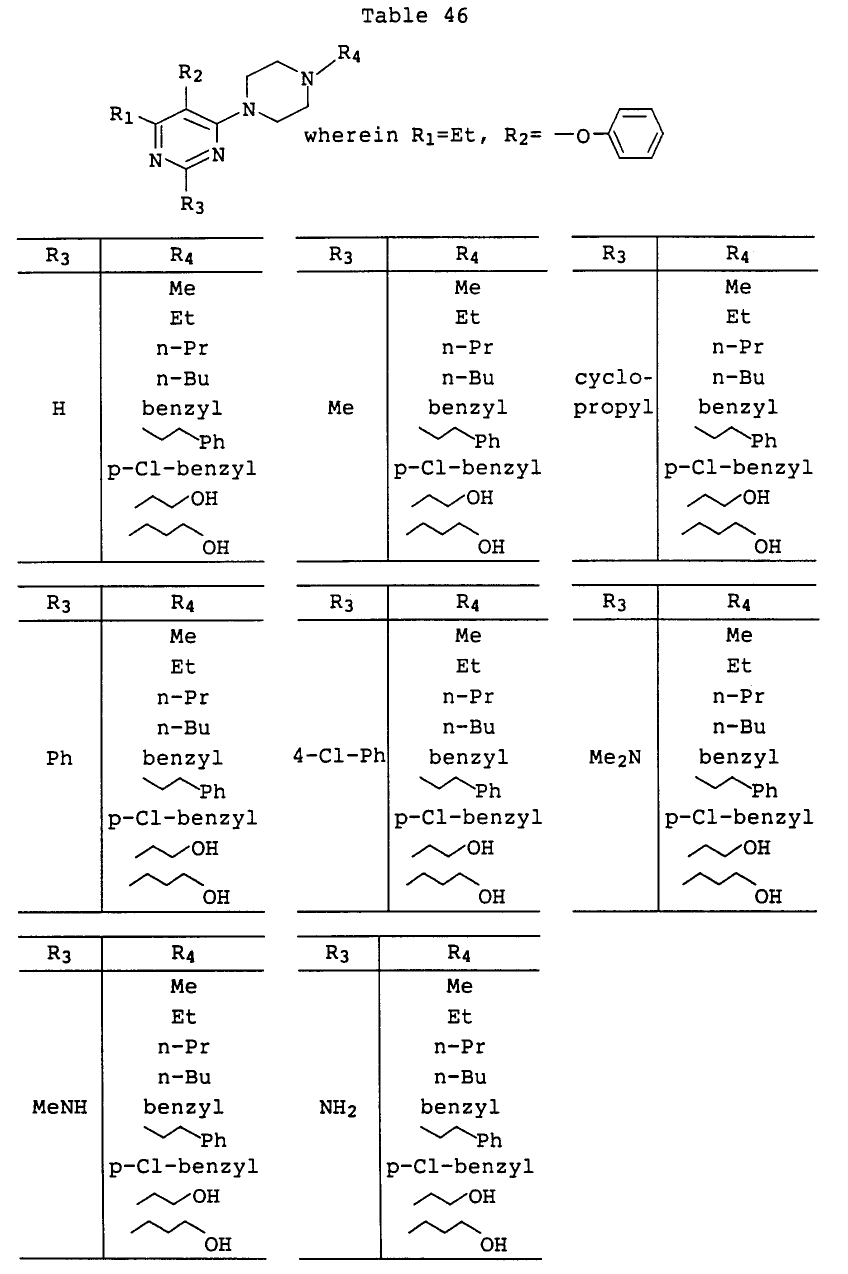

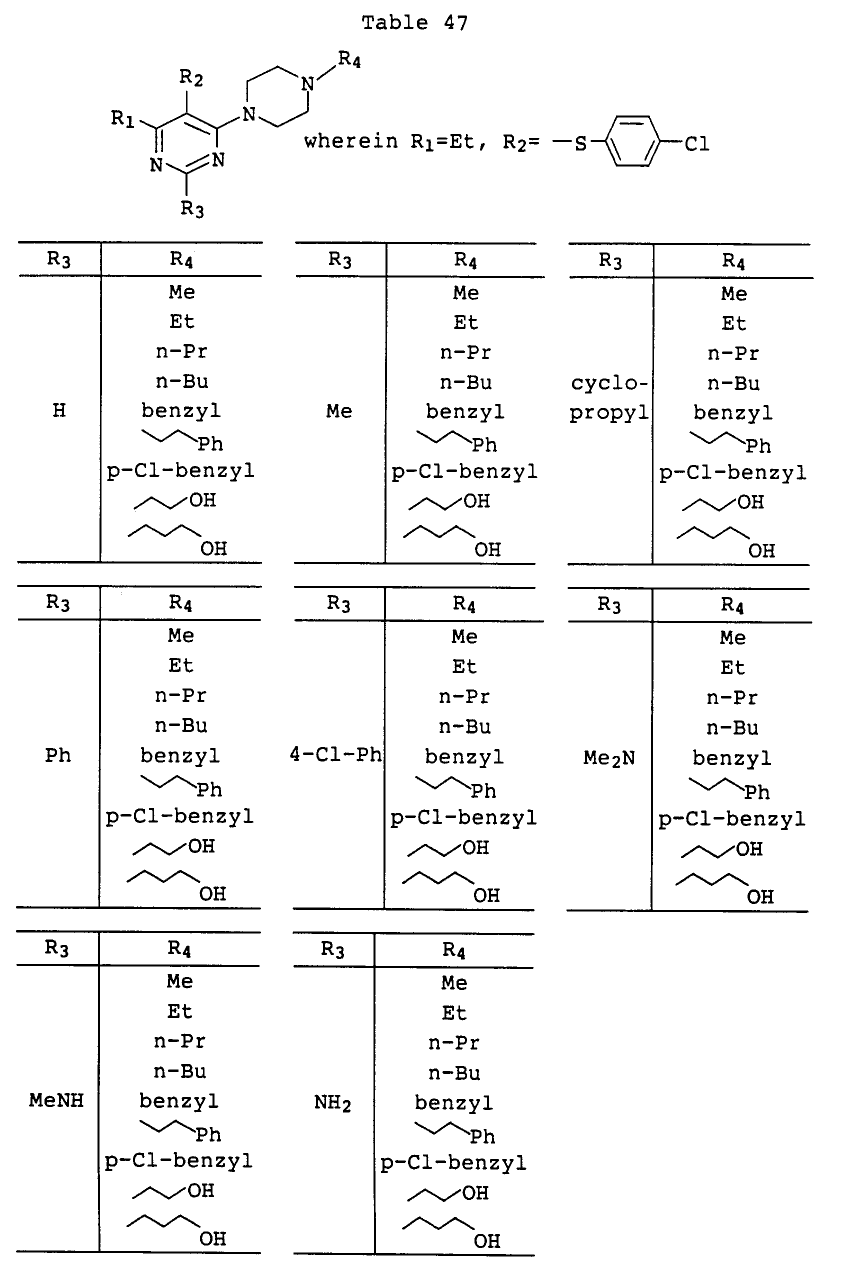

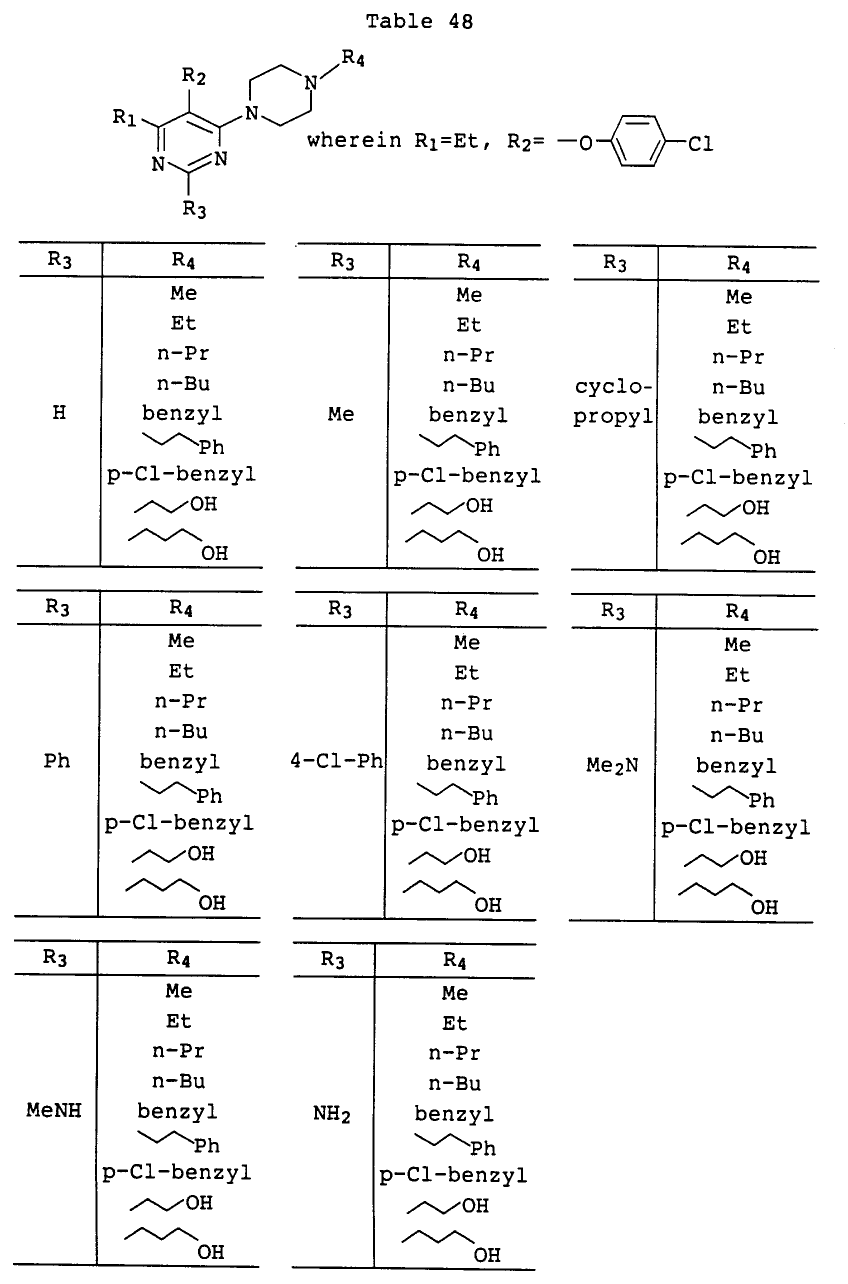

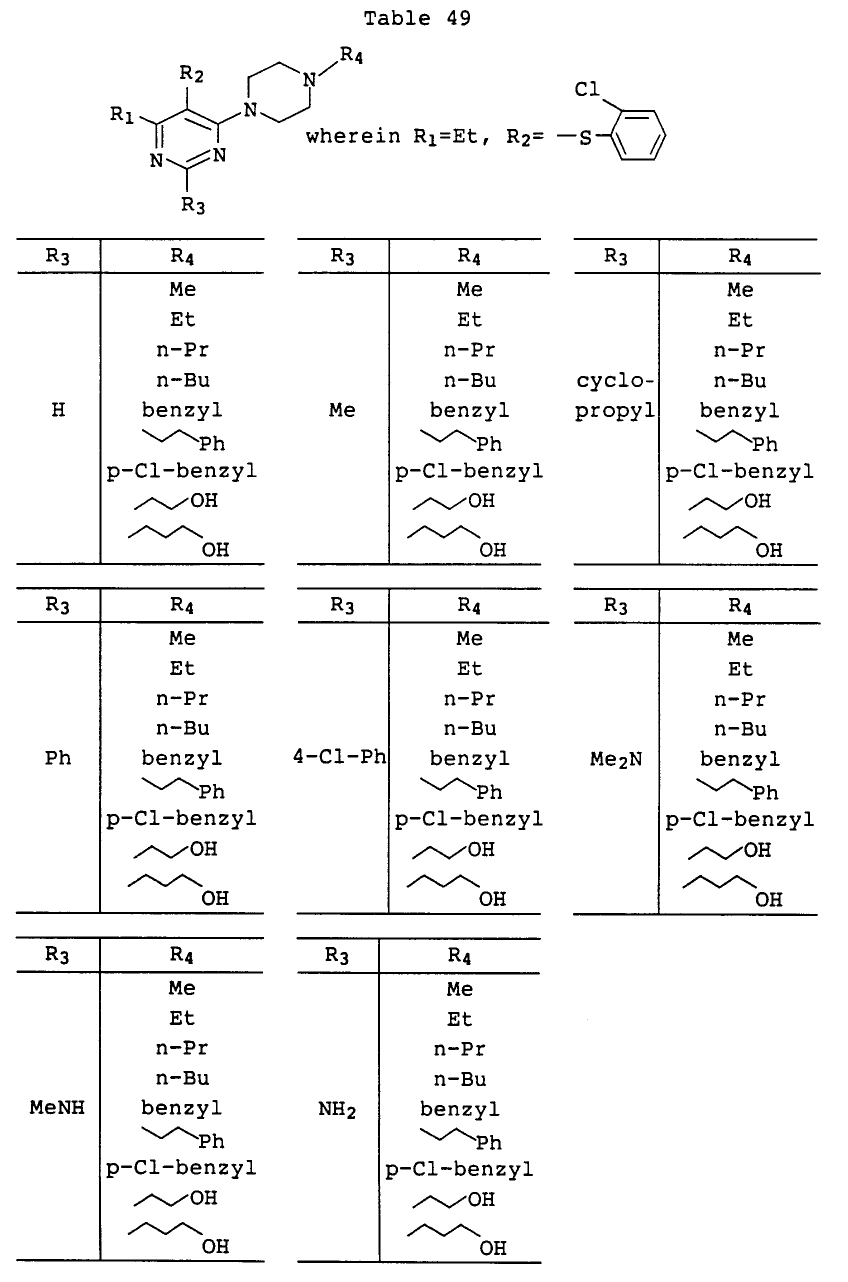

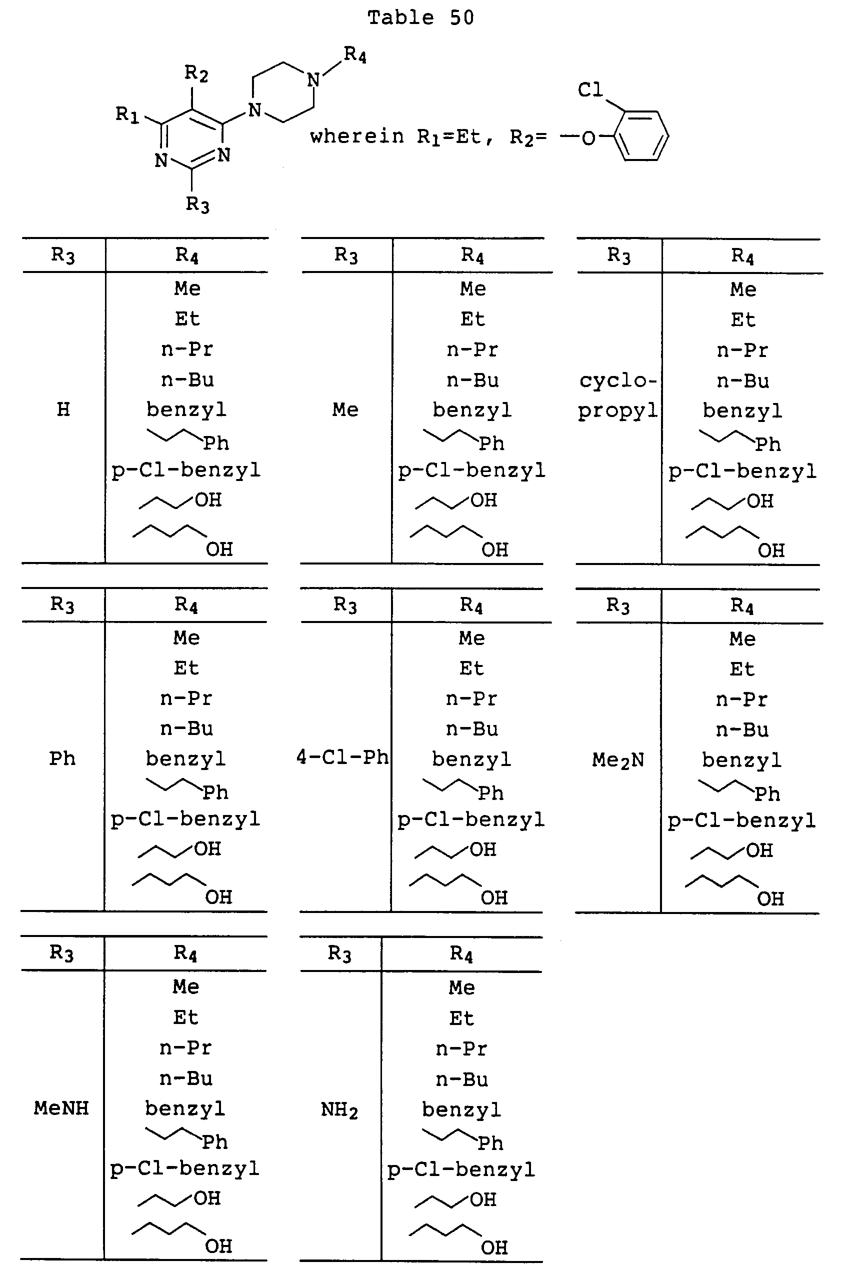

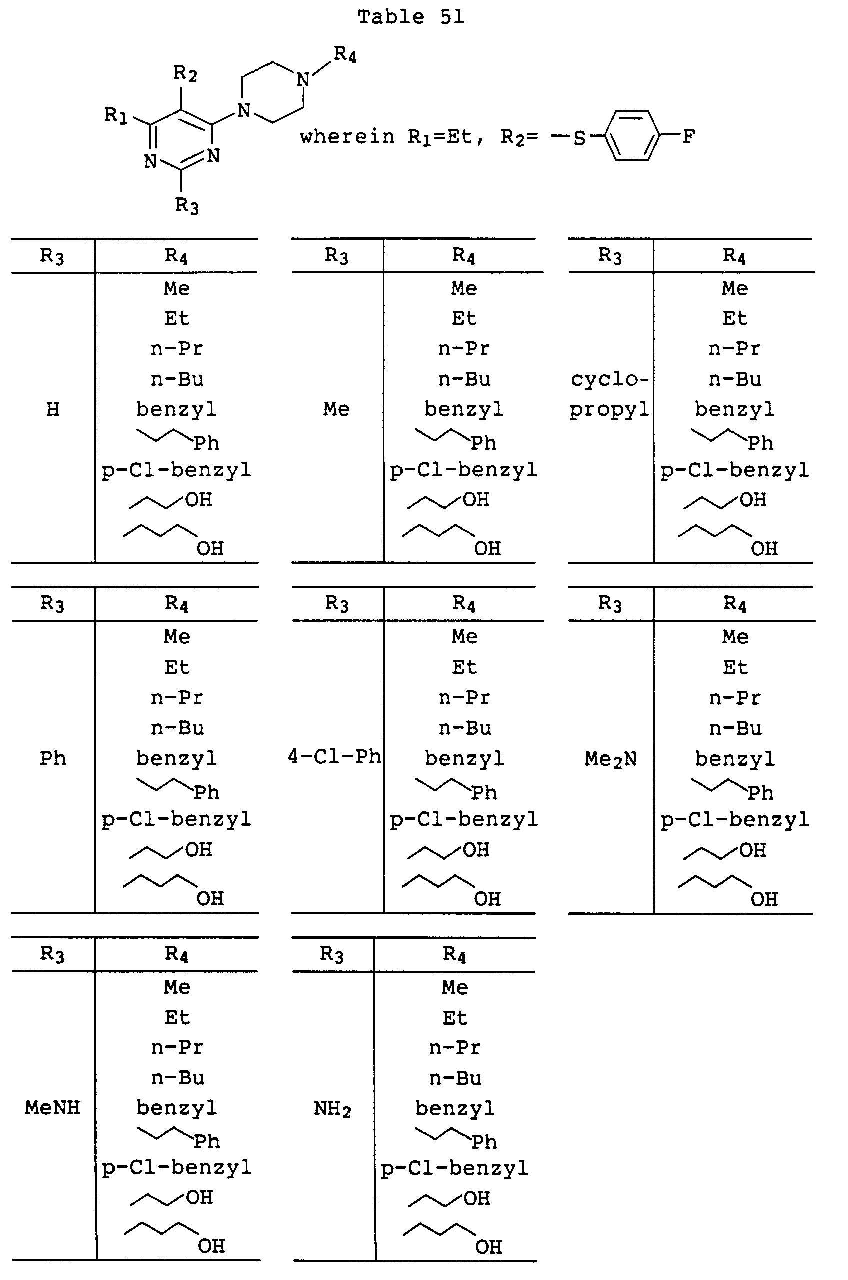

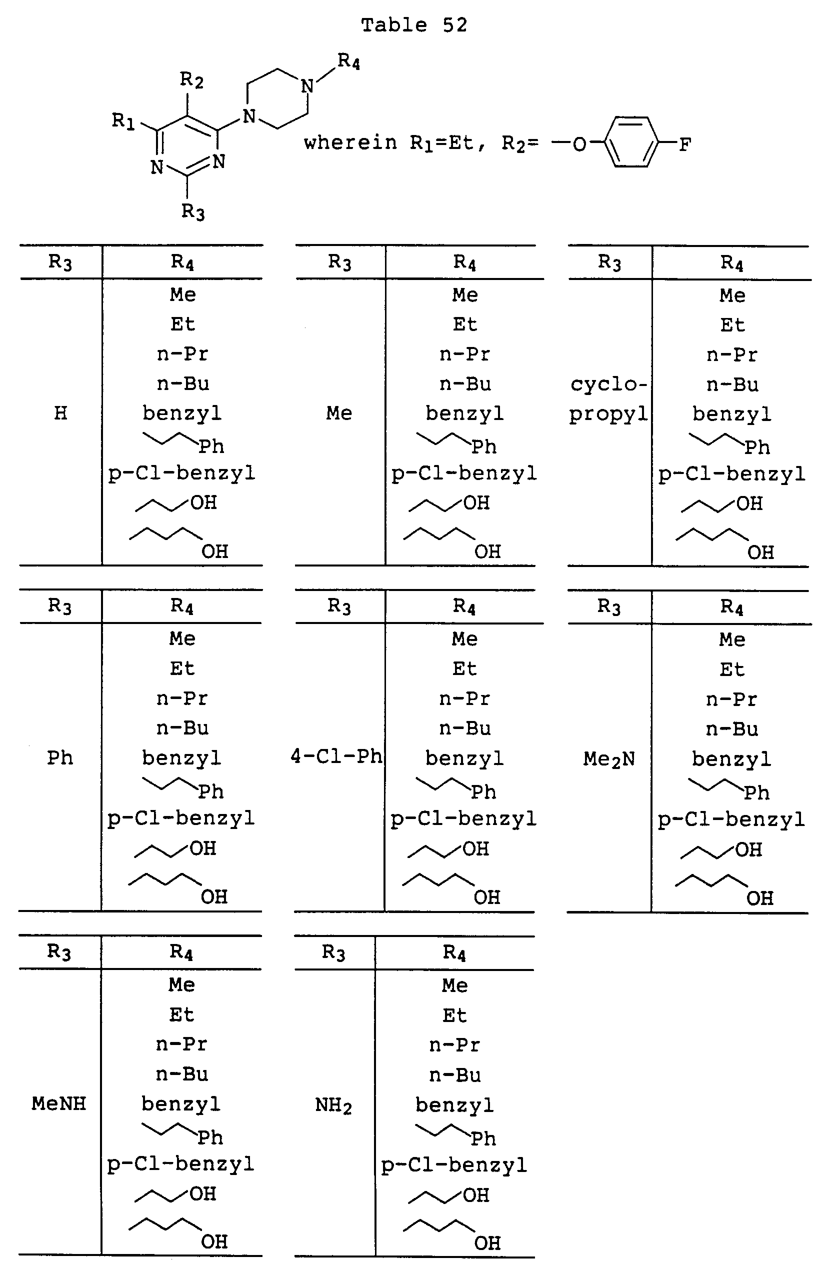

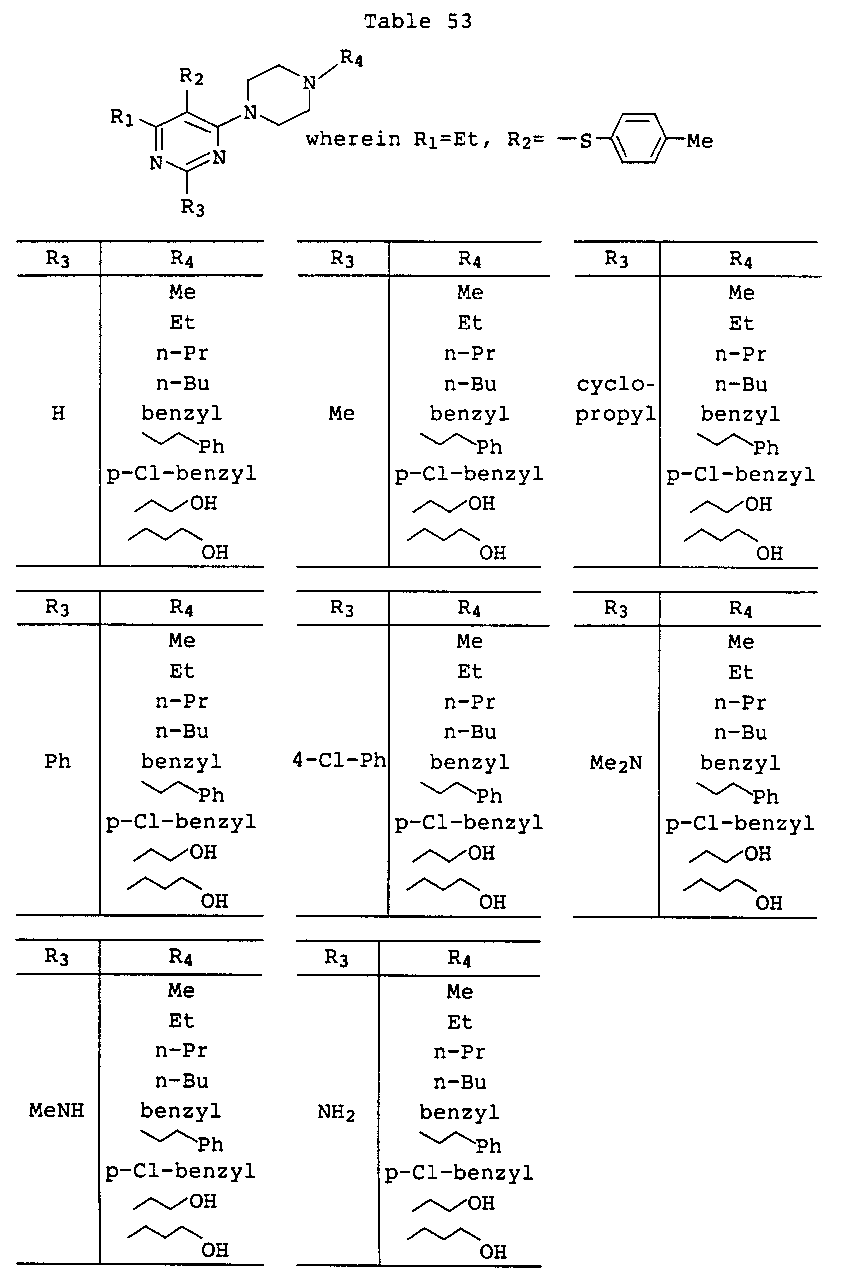

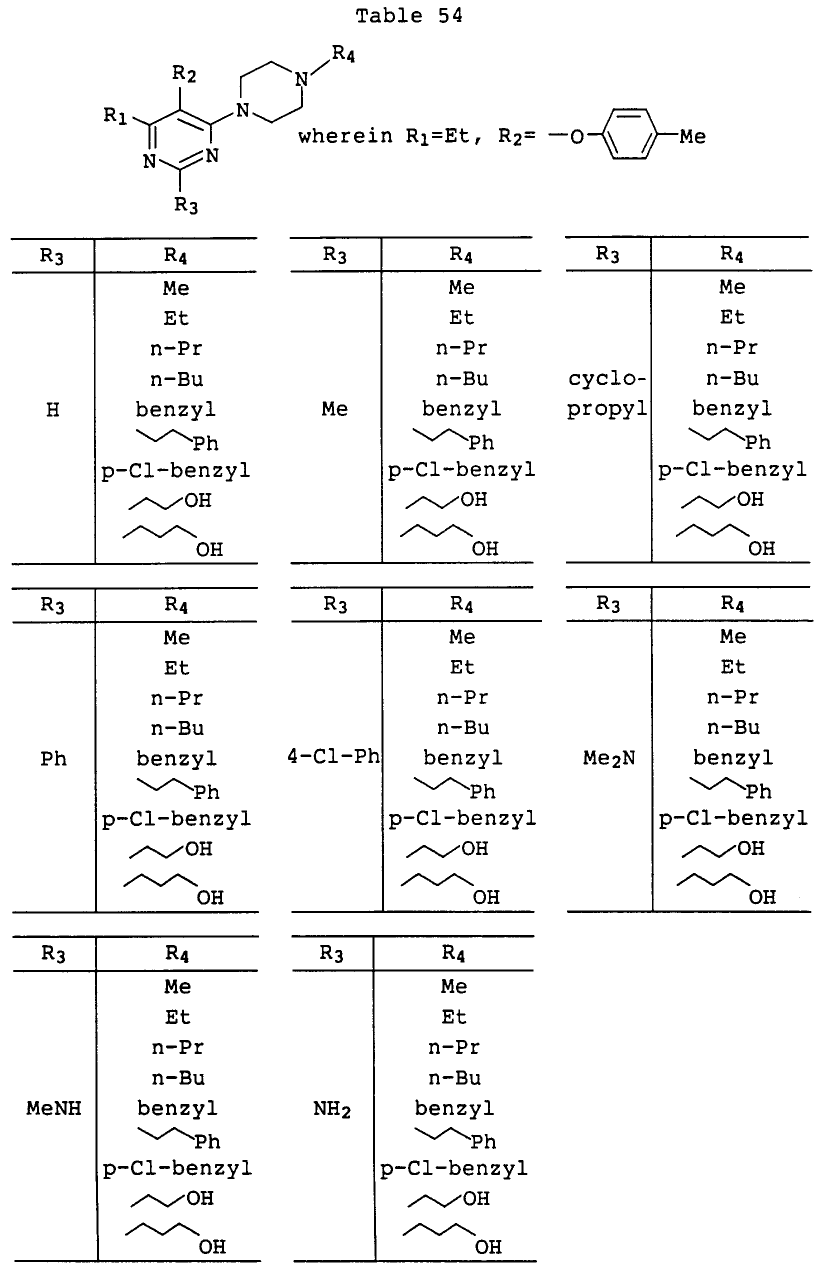

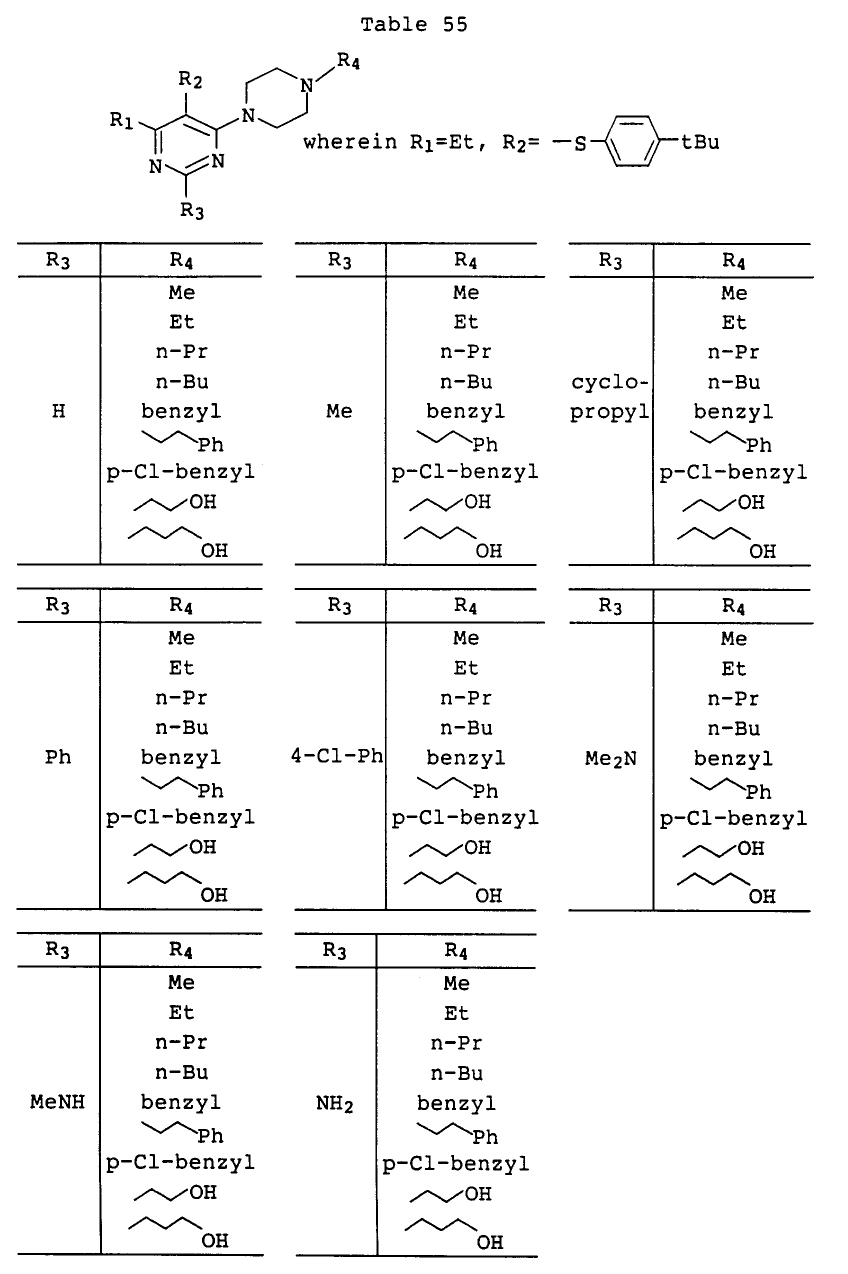

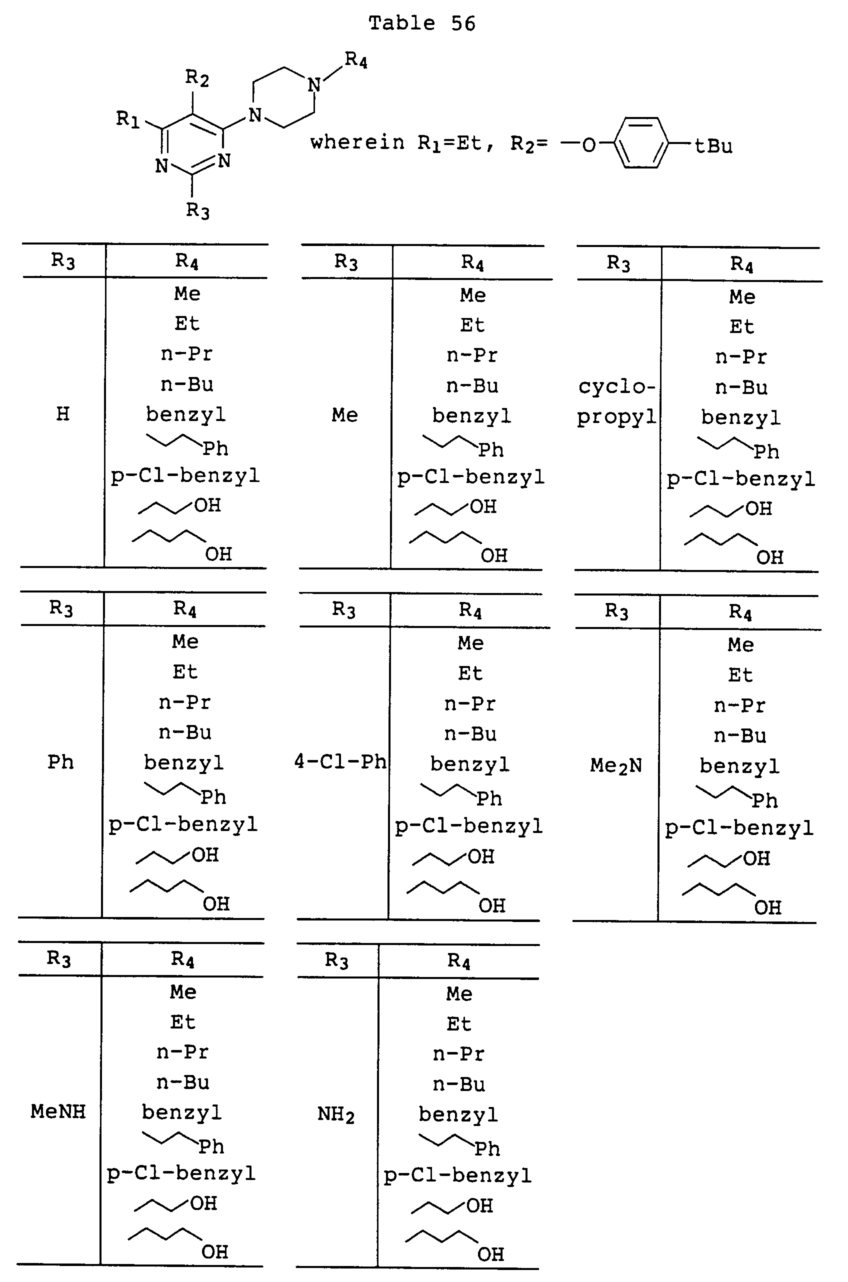

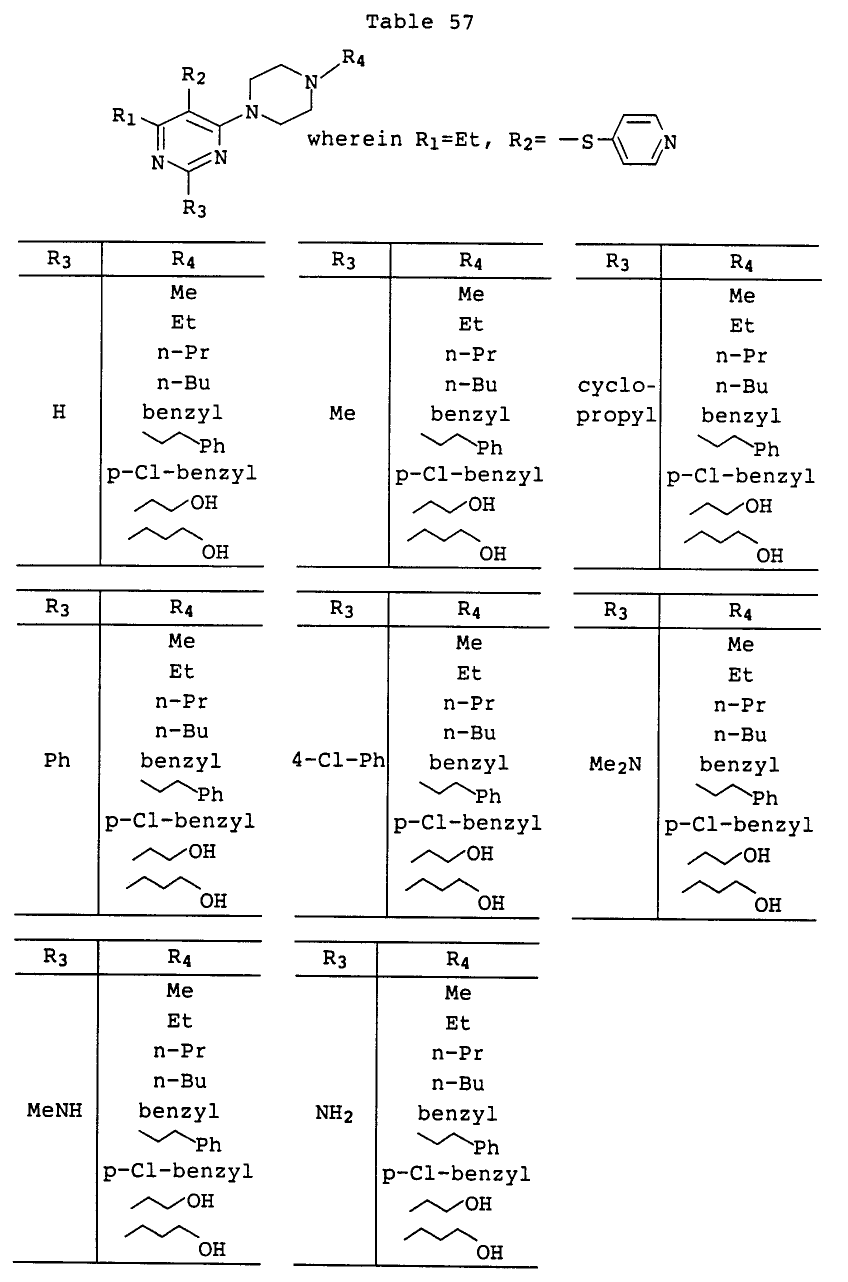

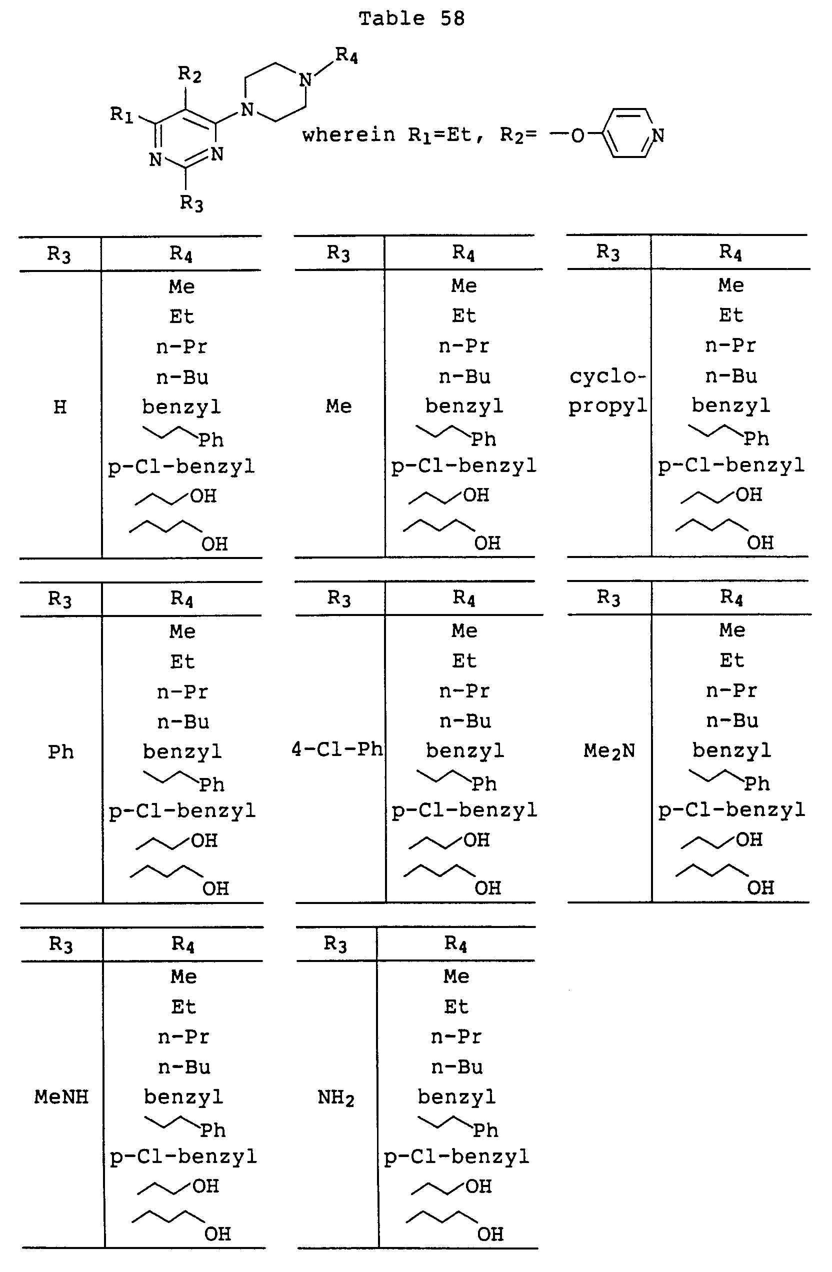

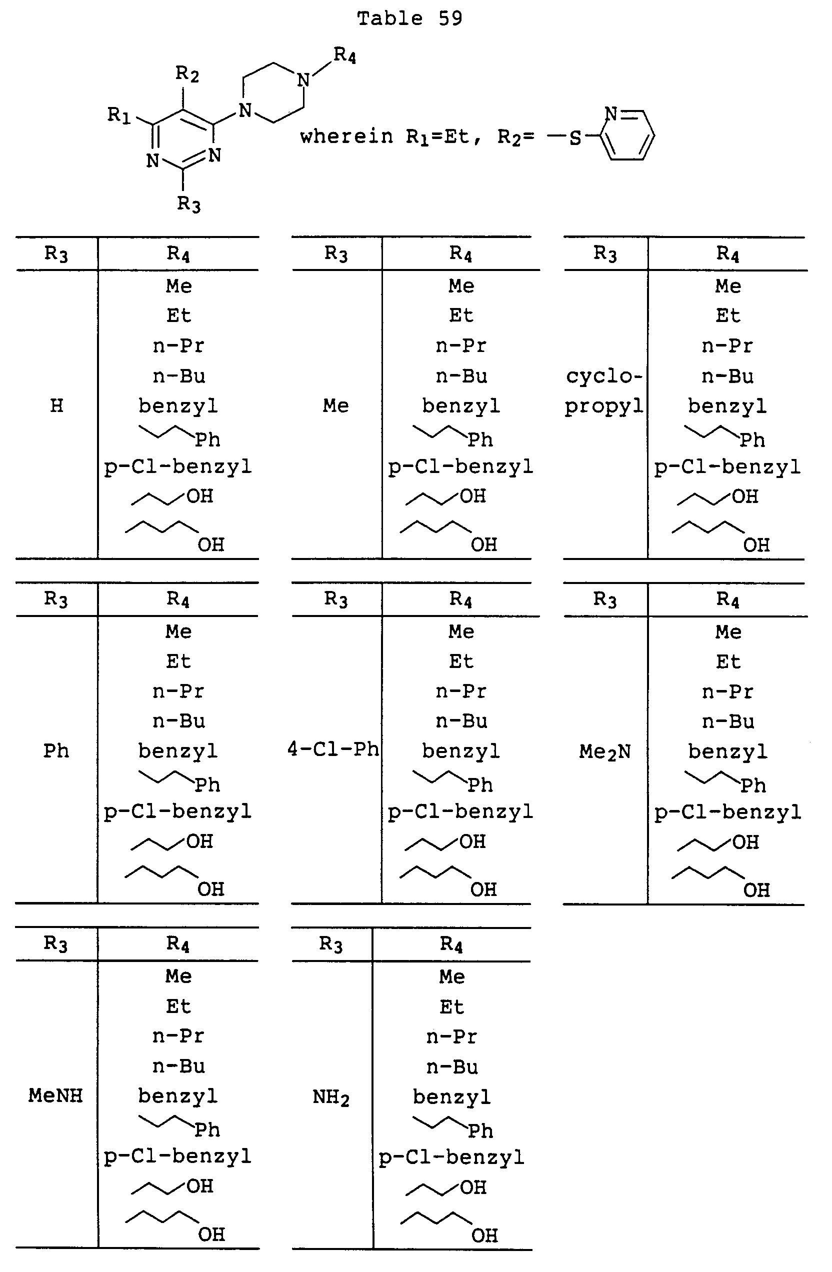

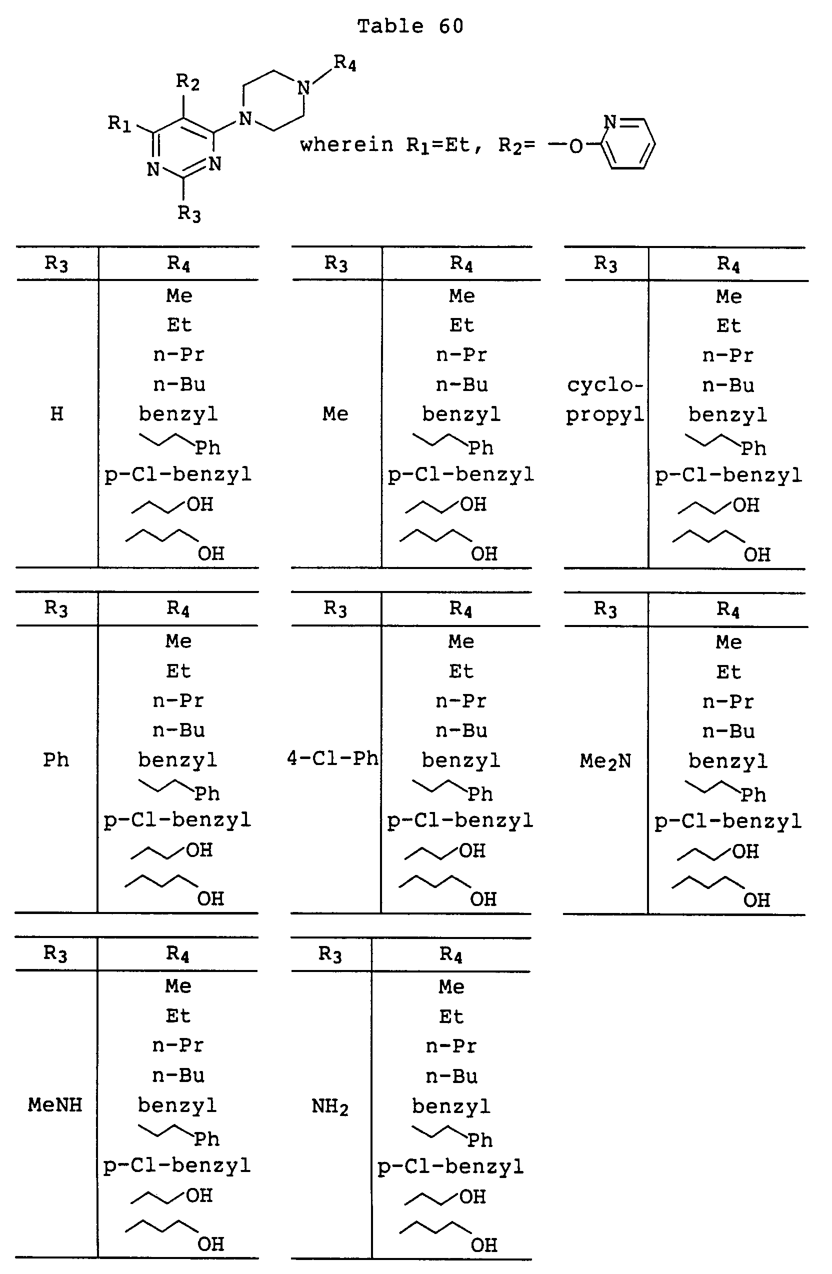

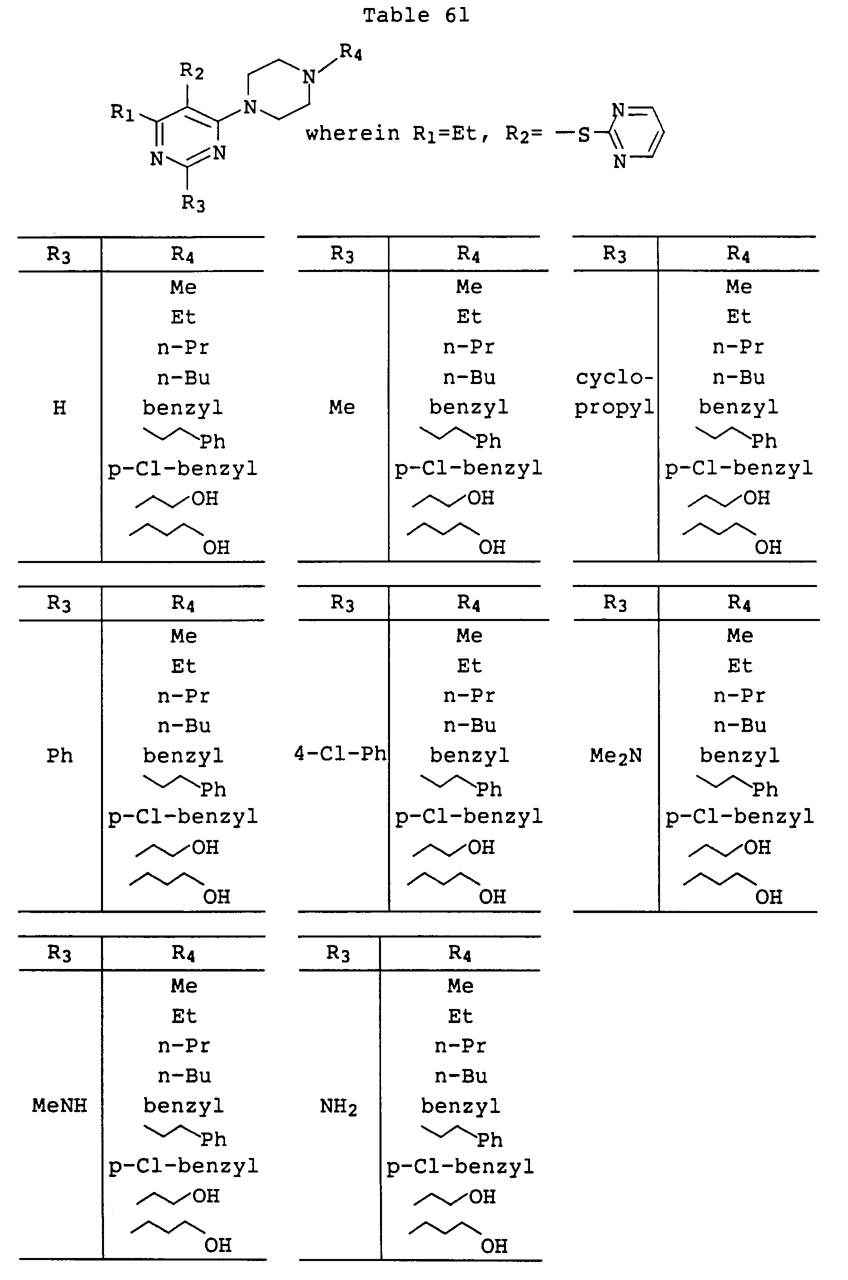

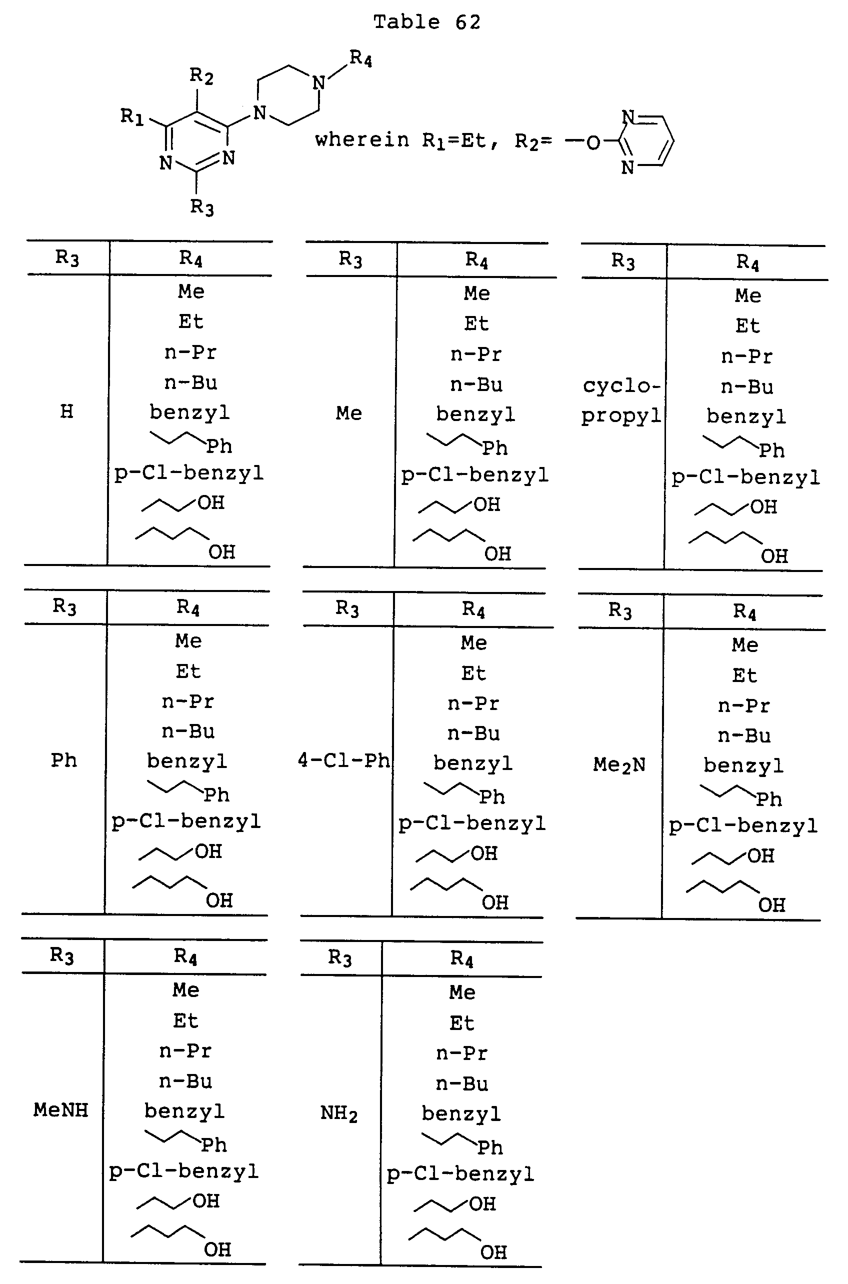

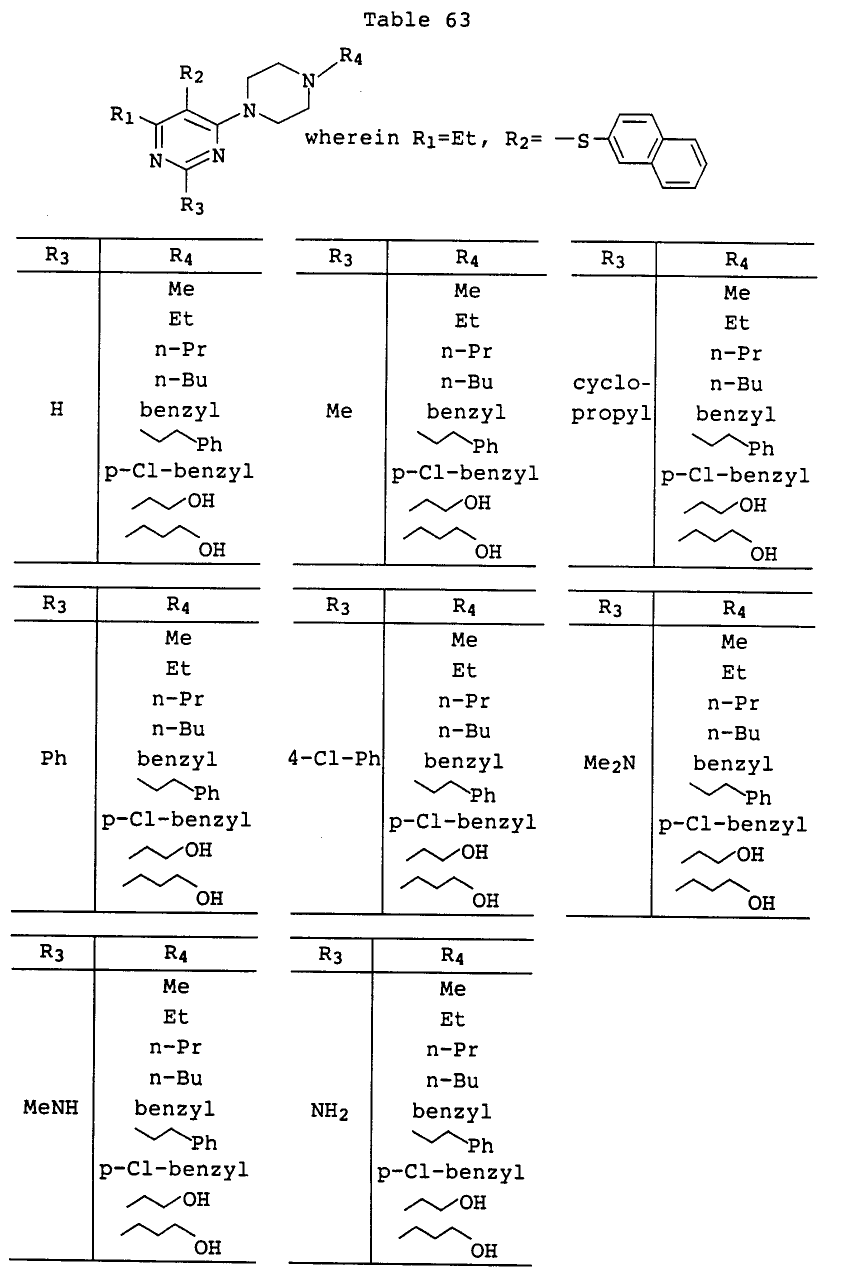

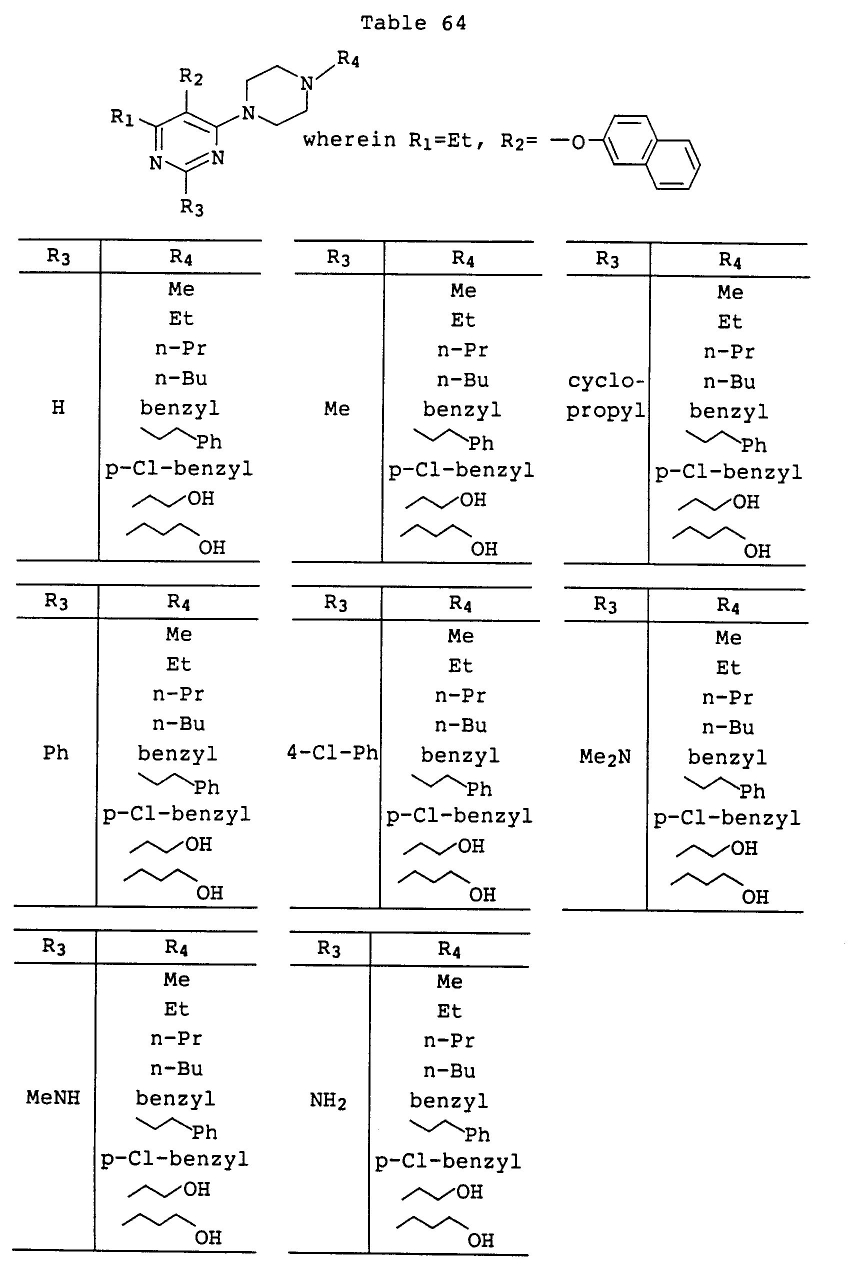

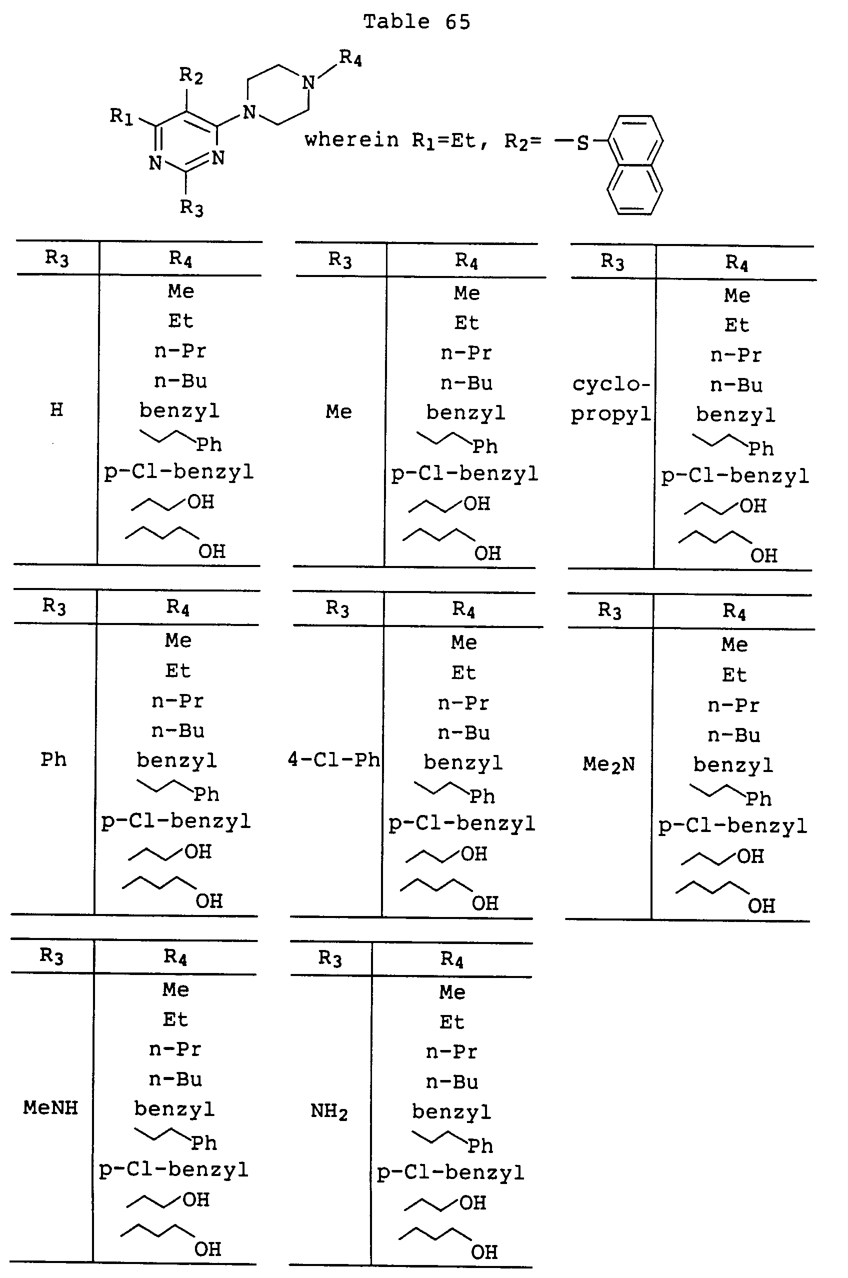

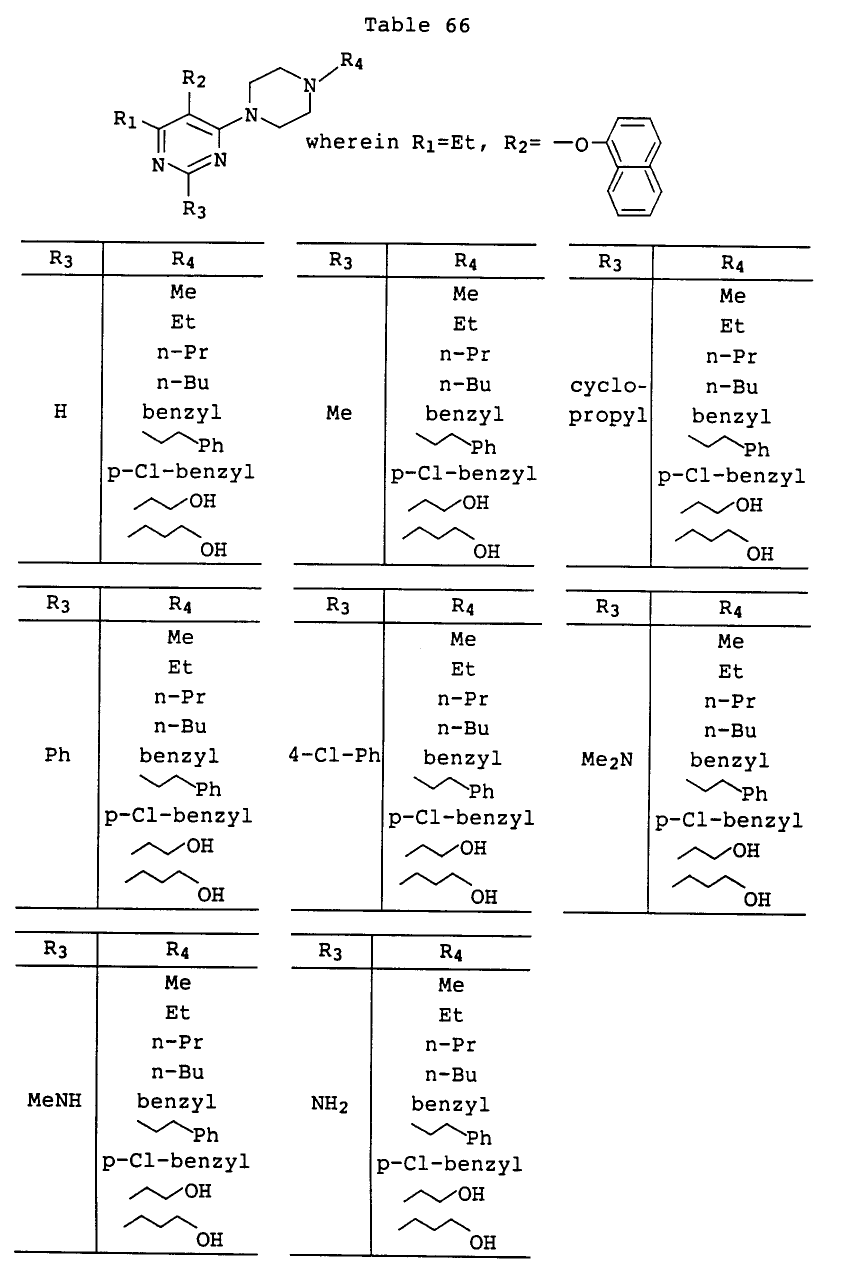

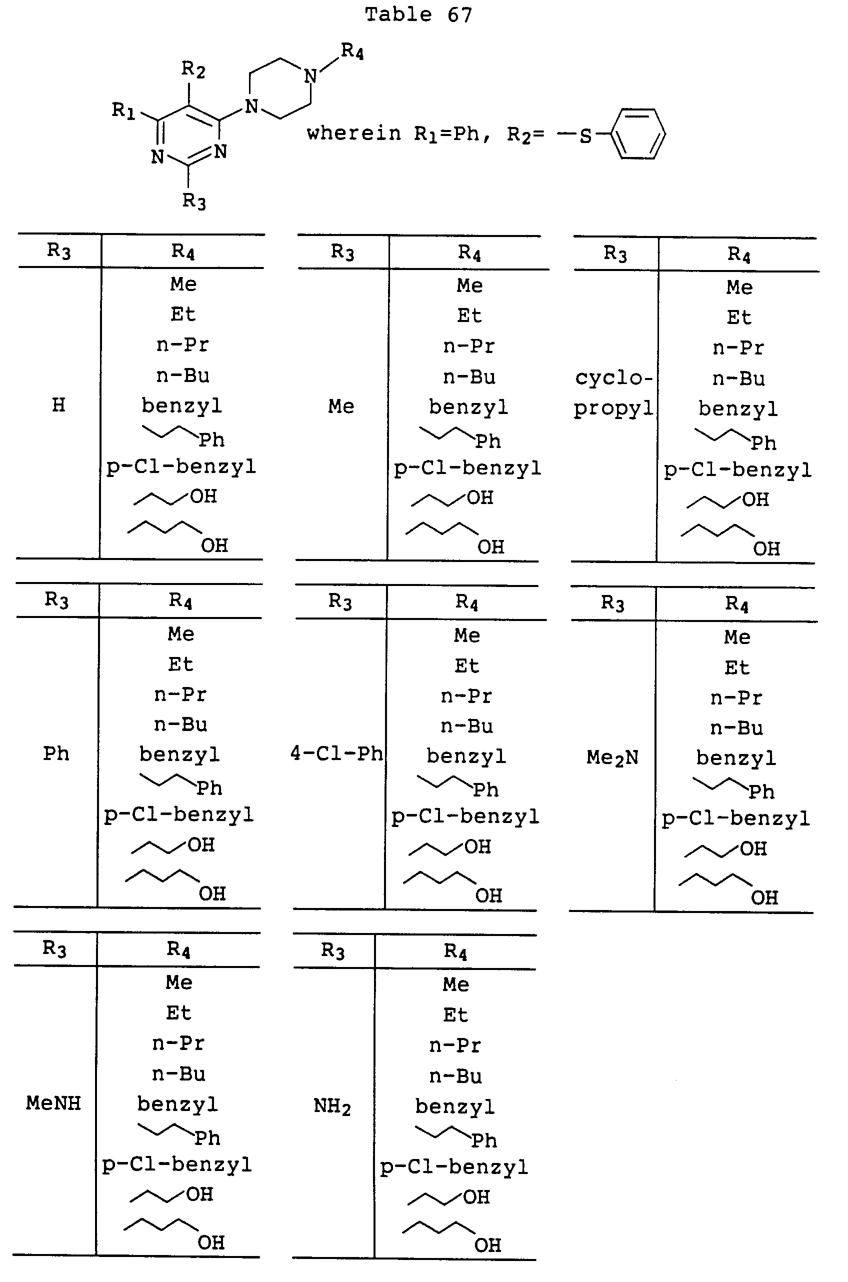

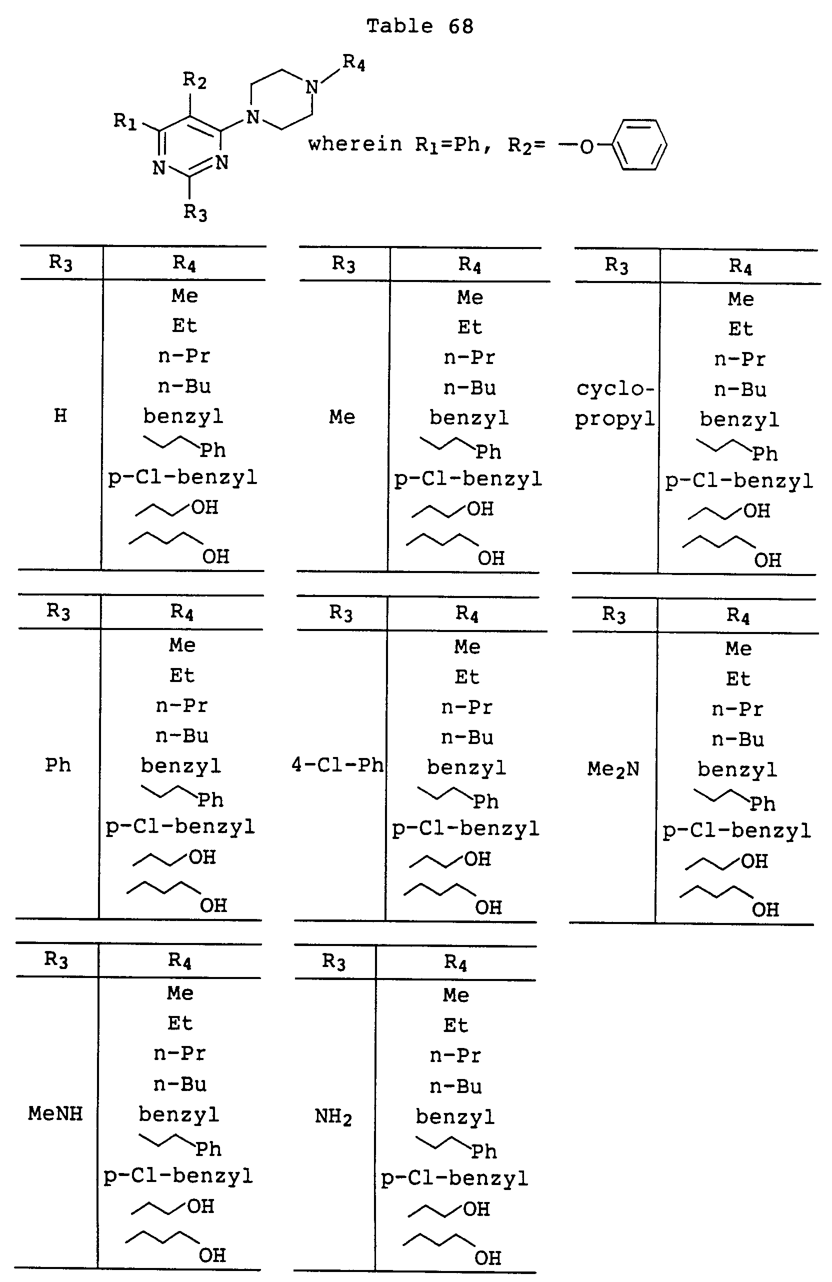

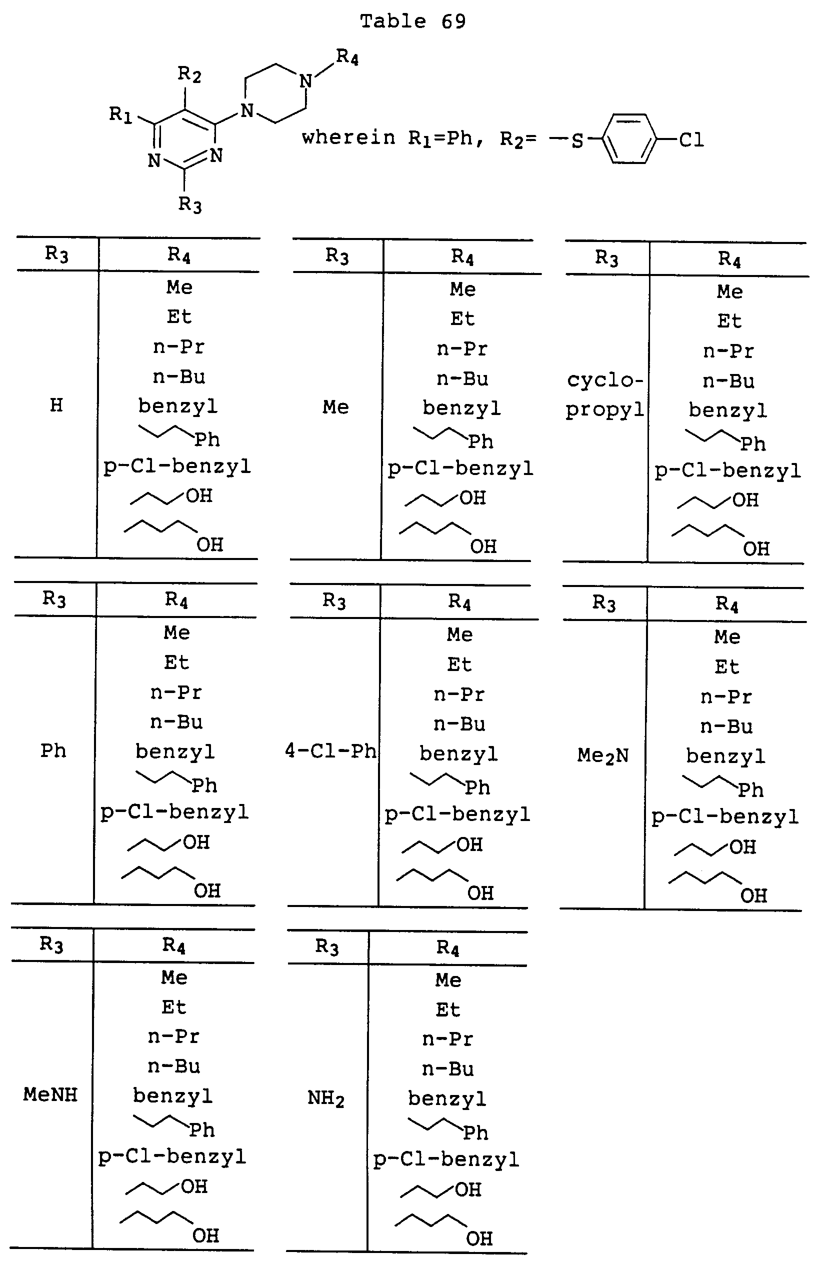

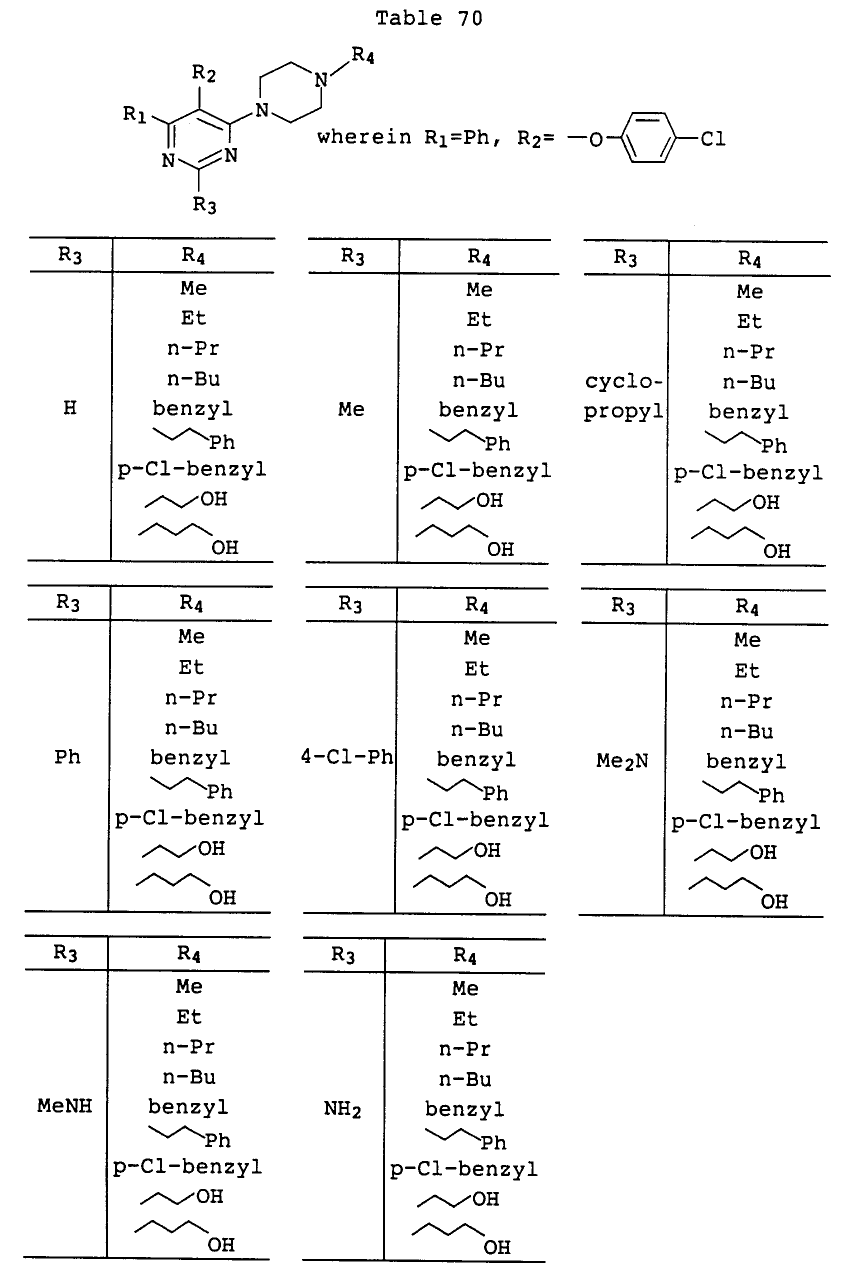

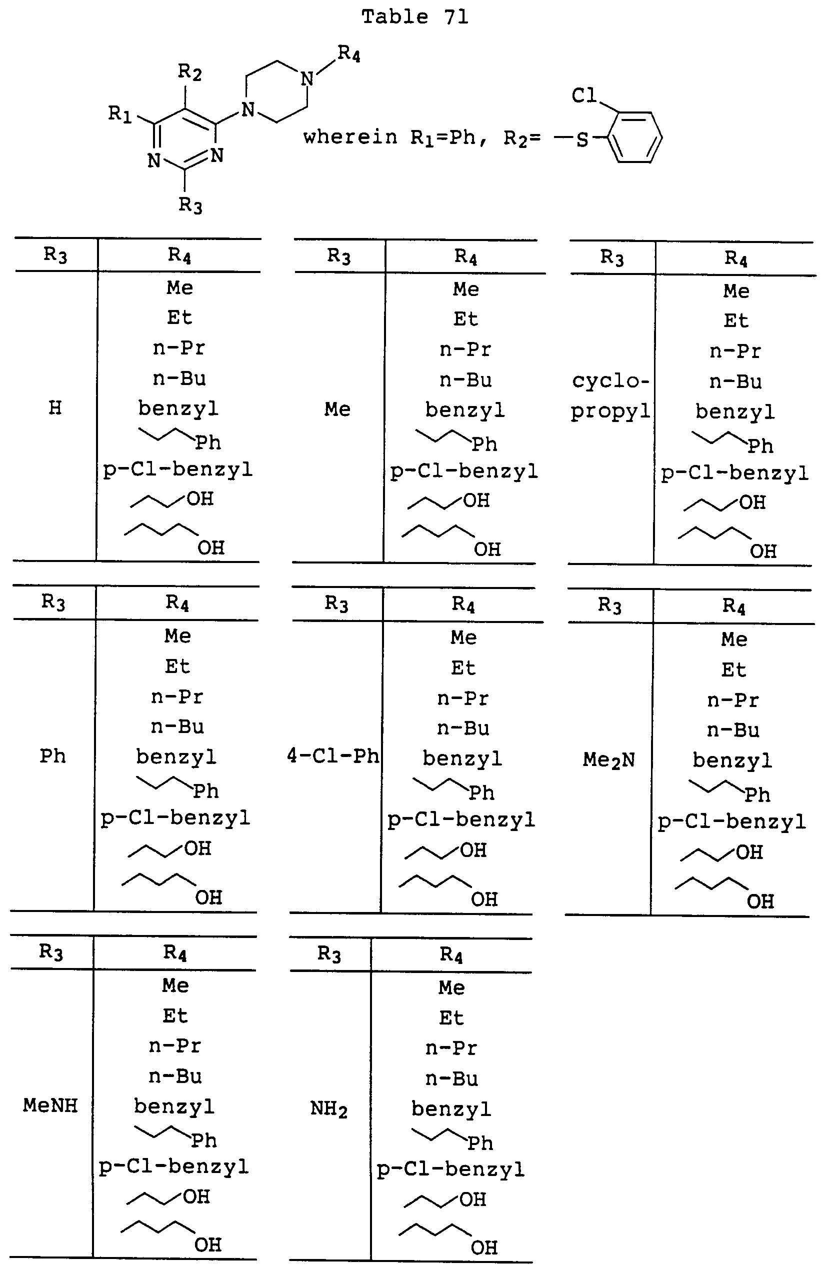

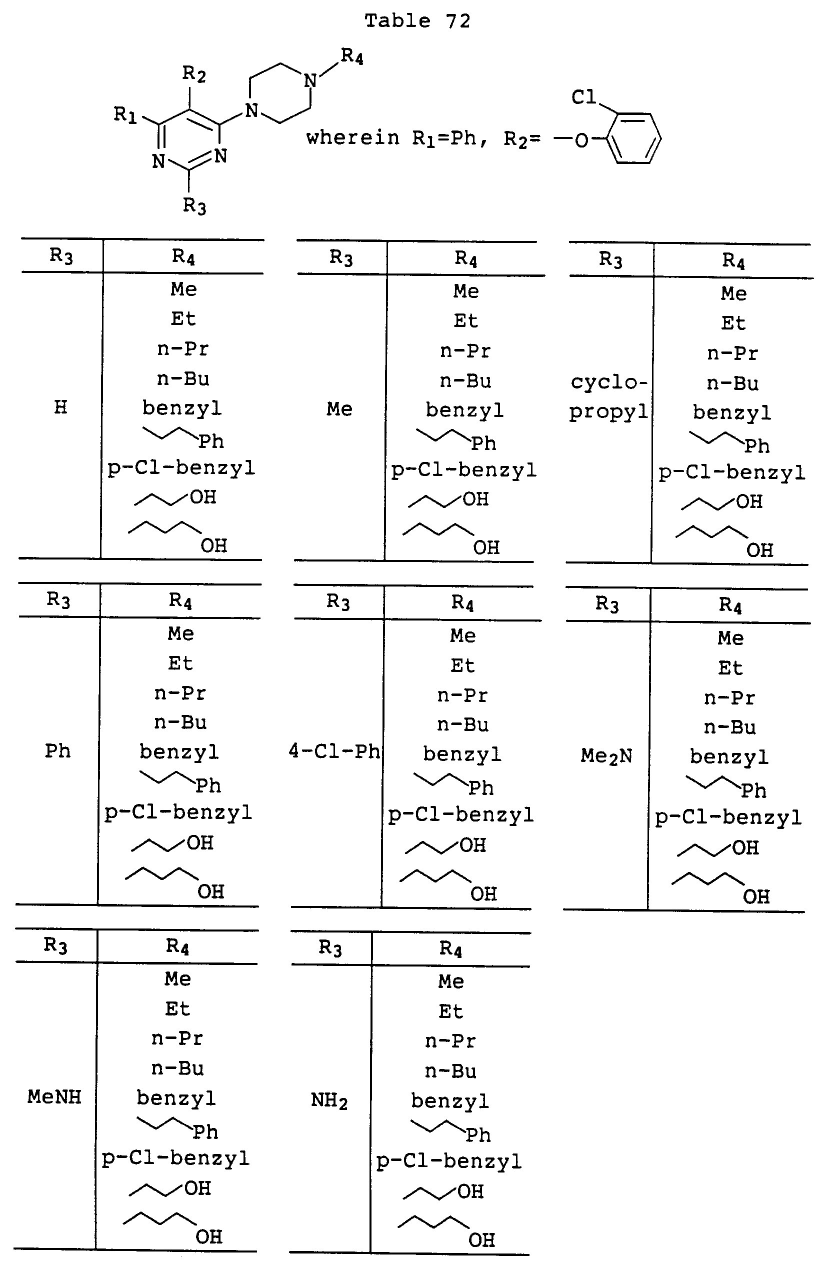

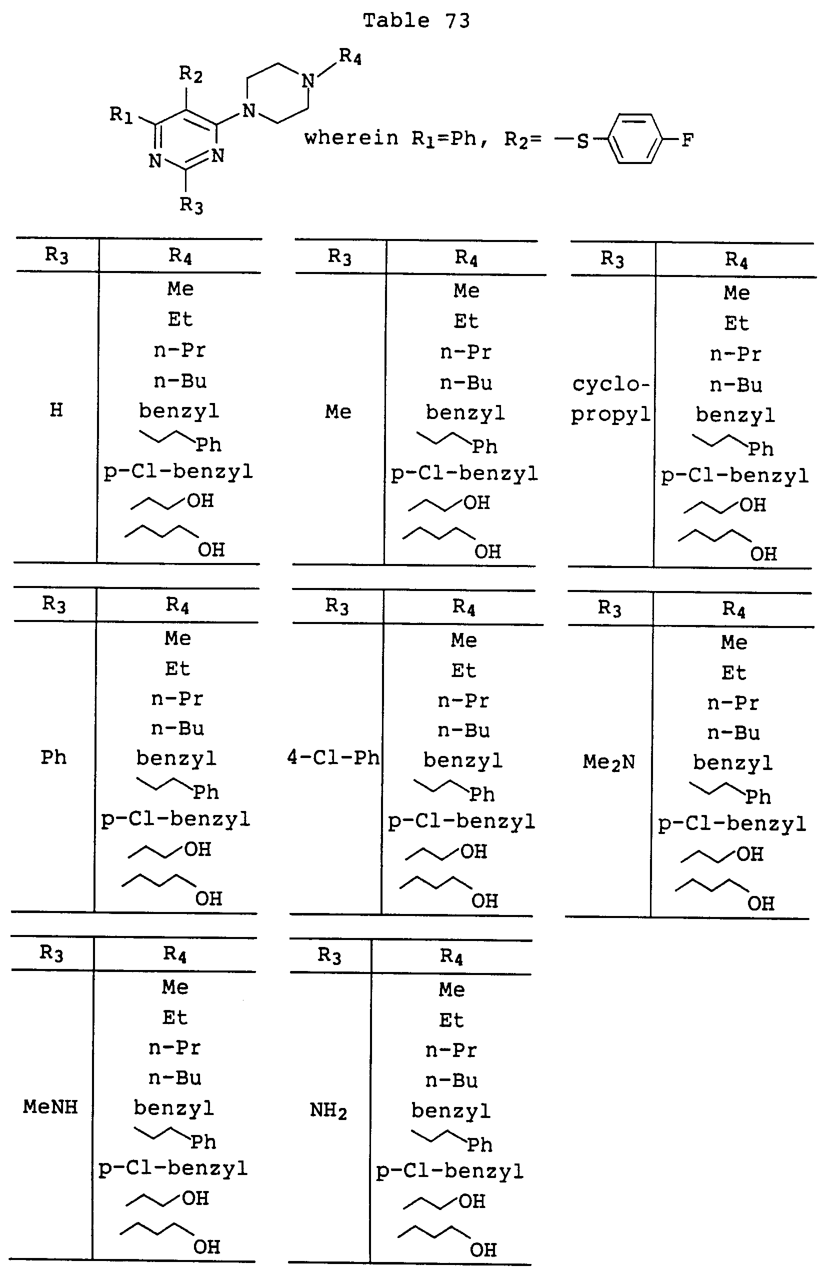

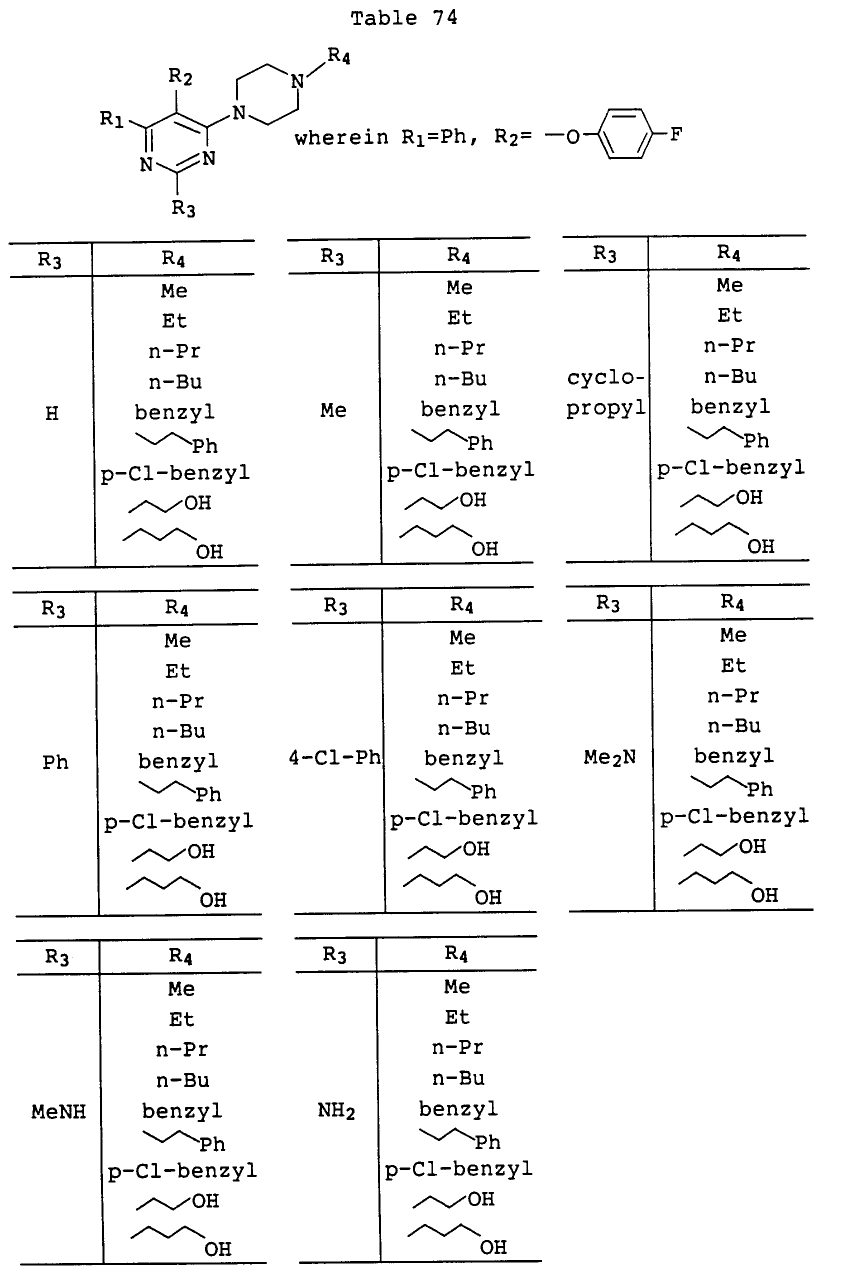

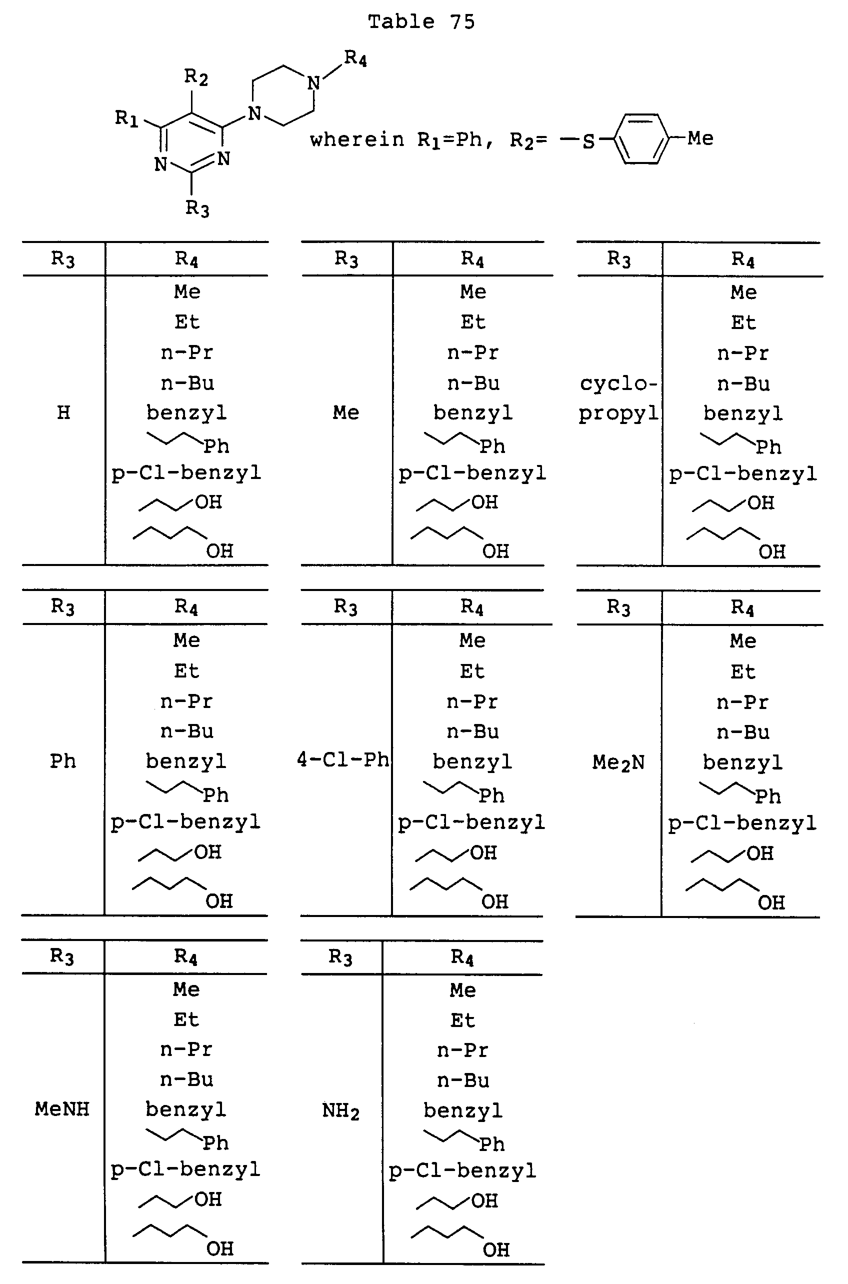

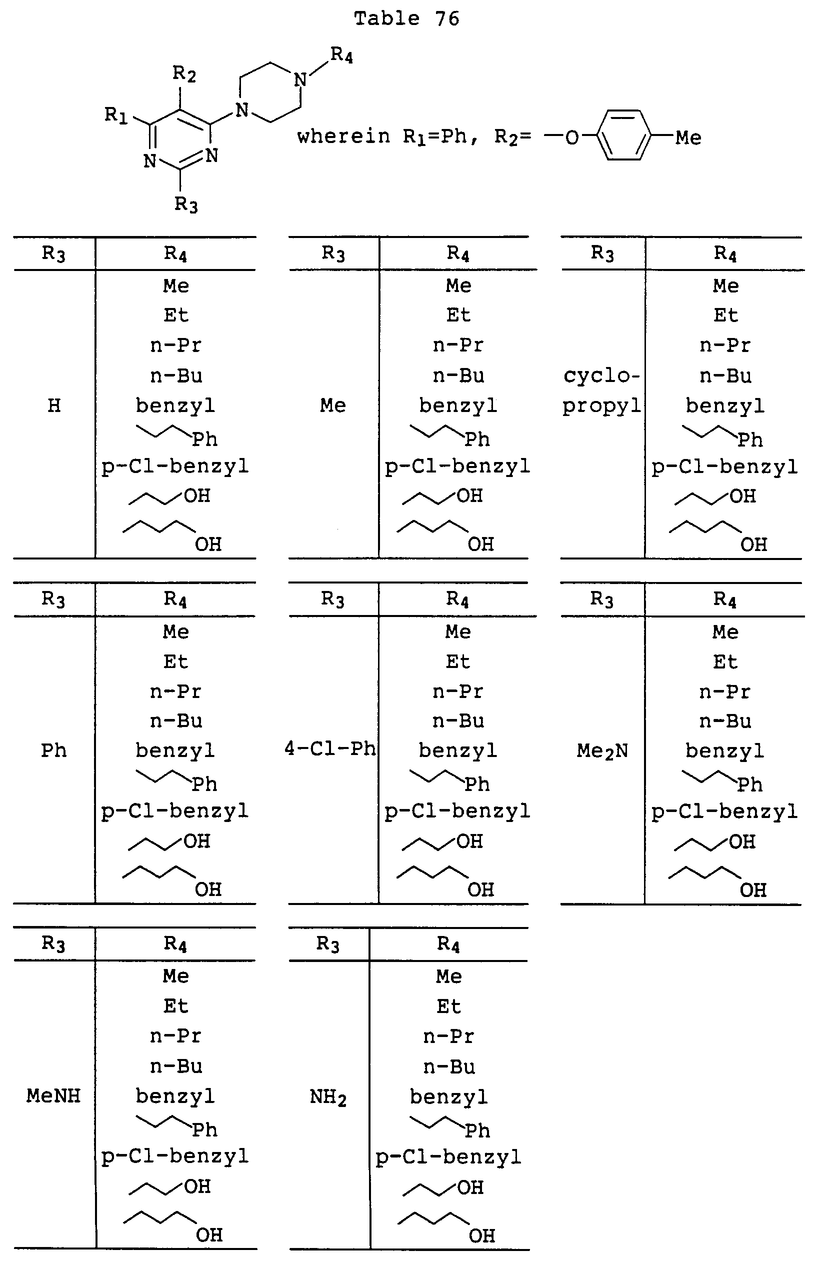

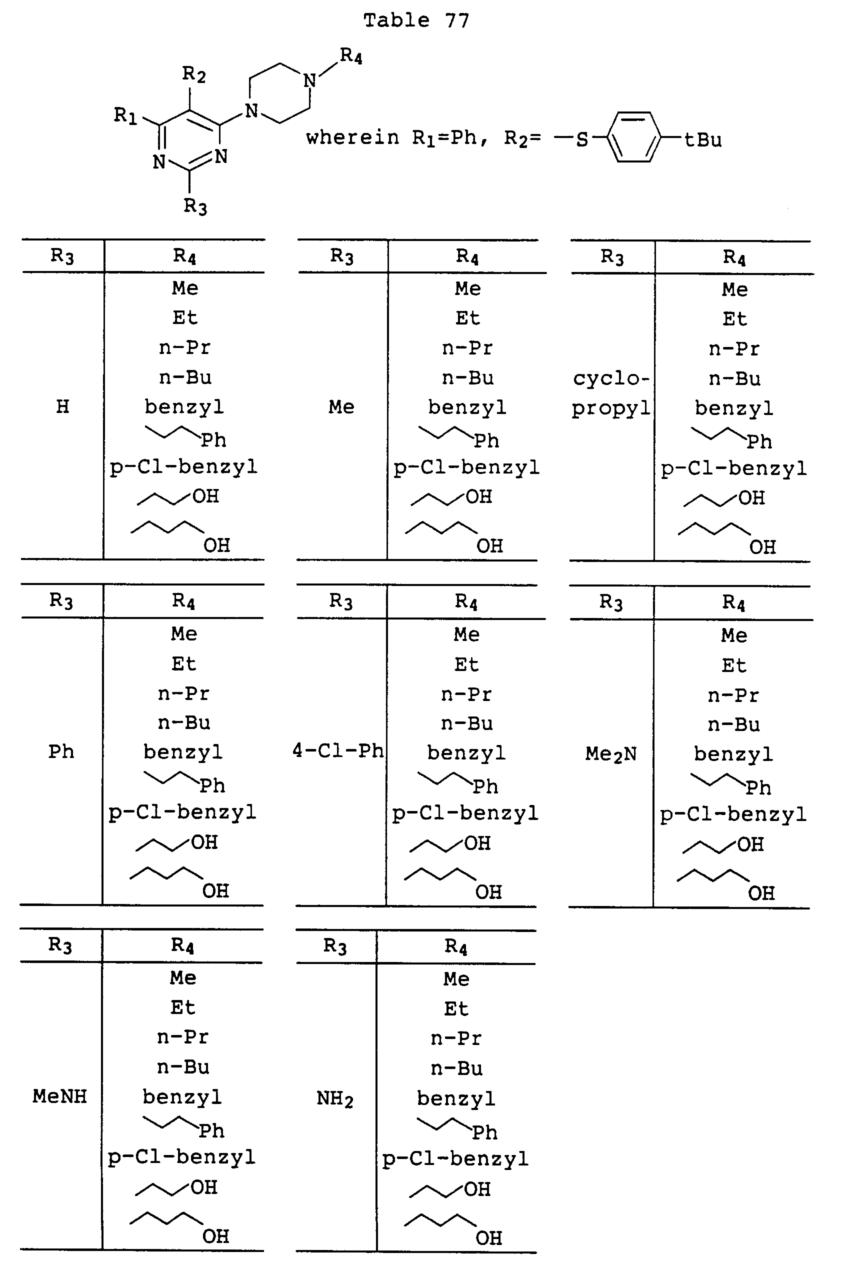

In addition, the following compounds may be exemplified as the compounds and the intermediates according to the present invention:
2,4,6-trichloro-5-(4-chlorophenylthio)pyrimidine,
2,4,6-trichloro-5-(3-chlorophenylthio)pyrimidine,
2,4,6-trichloro-5-phenylthiopyrimidine,
4,6-dichloro-5-(4-chlorophenylthio)-2-methylaminopyrimidine,
4,6-dichloro-5-(3-chlorophenylthio)-2-methylaminopyrimidine,
4,6-dichloro-2-methylamino-5-phenylthiopyrimidine,
4,6-dichloro-2-dimethylamino-5-phenylthiopyrimidine,
4-chloro-5-(4-chlorophenylthio)-2-methylamino-6-(4-methylpiperazino)pyrimidine,
4-chloro-5-(3-chlorophenylthio)-2-methylamino-6-(4-methylpiperazino)pyrimidine,
4-chloro-2-methylamino-6-(4-methylpiperazino)-5-phenylthiopyrimidine,
4-chloro-2-dimethylamino-6-(4-methylpiperazino)-5-phenylthiopyrimidine,
4-chloro-6-methyl-2-methylamino-5-phenyloxypyrimidine, and
4-methyl-2-methylamino-6-(4-methylpiperazino)-5-phenyloxypyrimidine.

An animal to which the compound of the present invention is administered is not limited, and the compound may be administered to not only a human being but also other various mammals such as a mouse, a rat, a dog, a cat, a cattle, a horse, a goat, a sheep, a rabbit, a pig, etc.

The compound may be administered to a human being or such a beast by a usual administration route, for example, orally, intramuscularly, intravenously, subcutaneously, intraperitoneally or intranasally. The dose and the frequency of administrations are not particularly limited and are varied depending on kind of animal, administration route, degree of disease, sex, body weight, etc. The compound is usually administered to an adult in a dose of 1 mg to 300 mg per day in one portion or several portions in the case of a human being. The dosage form of the compoud includes, for example, powders, fine subtilaes, tablets, capsules, suppositories, injections, trans-nasal formulations, etc. When the compound is formulated into a pharmaceutical form, formulation is carried out by a conventional method by using a conventional carrier for formulation. In detail, when an oral pharmaceutical composition is prepared, a binder, a disintegrator, a lubricant, a coloring agent, etc. are added to the compound if necessary, and the resulting mixture is made into tablets, granules, powder, capsules or the like by a conventional method. When an injection is prepared, a pH adjustor, buffer, stabilizer, solubilizer, etc. are added to the compound if necessary, and the resulting mixture is made into an injection by a conventional method.

The present invention is illustrated below in further detail with reference to examples but is not limited by the examples at all.

### Example 1

### 2,4,6-Trichloro-5-(4-chlorophenylthio)pyrimidine

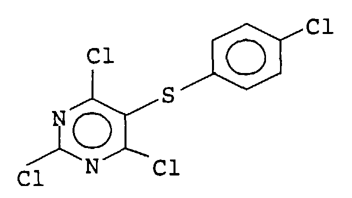

### 1) 4-Chlorophenylmethylsulfonium barbitylide

In dimethylformamide (100 ml) was dissolved 4-chlorothiophenol (7.23 g), and the reaction solution was adjusted to 5°C. NaH (2.88 g) was added and after stirring for 30 minutes, the reaction mixture was slowly heated to room temperature. A solution of methyl iodide (8.52 g) in dimethylformamide (100 ml) was added dropwise and was stirred overnight. The excess NaH was decomposed with water, followed by extraction with ethyl acetate, and the organic layer was washed with water and dried over anhydrous magnesium sulfate. The solvent was distilled off under reduced pressure to obtain a light-yellow oil (7.90 g). The oil was dissolved in water without purification, and sodium periodate (10.7 g) was added at 5°C. After the resulting mixture was stirred for 24 hours, the precipitate was filtered off and the filtrate was extracted with methylene chloride. The extract solution was dried over anhydrous sodium sulfate and distilled under reduced pressure to remove the solvent, whereby a colorless oil (4.15 g) was obtained.

This oil was dissolved in acetic acid (20 ml) without purification, followed by adding thereto barbituric acid (2.56 g) and acetic anhydride (3.68 g). The resulting mixture was heated to 90°C, and was stirred for 7 hours. 10 ml of water was added and the reaction mixture was cooled to room temperature. The precipitated colorless crystals were collected by filtration and washed with acetone to obtain the desired compound (7.10 g).
- ¹H-NMR (500 MHz (CDCl₃), TMS): δ: 3.46(3H, s), 7.66(2H, d, J=9.0Hz), 7.76(2H, d, J=9.0Hz), 10.31(2H, s).
- MS (EI, direct):: 284 (M⁺).

### 2) 2,4,6-Trichloro-5-(4-chlorophenylthio)pyrimidine

The 4-chlorophenylmethylsulfonium barbitylide (2.0 g) obtained above was dissolved in 20 ml of phosphorus oxychloride, followed by adding thereto 1 ml of dimethylaniline, and the resulting mixture was heated with stirring for 24 hours. The excess phosphorus oxychloride was hydrolyzed with water and ice, and the precipitated colorless crystals were washed with water to obtain the desired compound (2.28 g).
- ¹H-NMR (500 MHz (CDCl₃), TMS): δ: 7.23(2H, dt, J=2.0 and 8.5Hz), 7.30(2H, dt, J=2.0 and 8.0Hz).
- ¹³C-NMR (125 MHz (CDCl₃), TMS): δ: 127.94, 129.84, 130.46, 131.56, 134.53, 158.85, 167.42.

### Example 2

### 2,4,6-Trichloro-5-(3-chlorophenylthio)pyrimidine

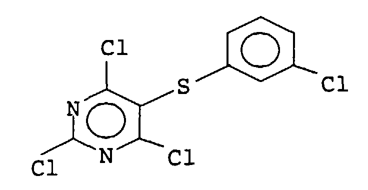

### 1) 3-Chlorophenylmethylsulfonium barbitylide

Crystals (7.08 g) of the desired compound were obtained from 3-chlorothiophenol (7.23 g) in the same manner as in Example 1 except for using the 3-chlorothiophenol in place of 4-chlorothiophenol.
- ¹H-NMR (500 MHz (CDCl₃), TMS): δ: 3.48(3H, s), 7.60-7.66(1H, m), 7.66-7.67(3H, m), 10.32(1H, s).
- MS (EI, direct):: 284 (M⁺).

### 2) 2,4,6-Trichloro-5-(3-chlorophenylthio)pyrimidine

Crystals (2.26 g) of the desired compound were obtained from the 3-chlorophenylmethylsulfonium barbitylide (2.0 g) obtained above, in the same manner as in Example 1 except for using the 3-chlorophenylmethylsulfonium barbitylide in place of the 4-chlorophenylmethylsulfonium barbitylide obtained in Example 1.
- ¹H-NMR (500 MHz (CDCl₃), TMS): δ: 7.13(1H, dt, J=1.5, 7.0Hz), 7.24-7.28(3H, m).
- ¹³C-NMR (125 MHz (CDCl₃), TMS): δ: 127.36, 127.79, 128.34, 129.43, 130.61, 133.91, 135.39, 159.09, 167.62.

### Example 3

### 2,4,6-Trichloro-5-phenylthiopyrimidine

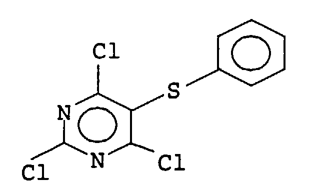

### 1) Methylphenylsulfonium barbitylide

Crystals (7.50 g) of the desired compound were obtained from methyl phenyl sulfoxide (4.90 g) and barbituric acid (3.84 g) in the same manner as in Example 1.
- ¹H-NMR (CDCl₃) δ (ppm): 3.46(3H, s), 7.57-7.59(3H, m), 7.73-7.75(2H, m), 10.27(2H, m).

### 2) 2,4,6-Trichloro-5-phenylthiopyrimidine

Crystals (5.70 g) of the desired compound were obtained from the methylphenylsulfonium barbitylide (7.50 g) obtained above, in the same manner as in Example 1 except for using the methylphenylsulfonium barbitylide in place of the 4-chlorophenylmethylsulfonium barbitylide obtained in Example 1.
- ¹H-NMR (500 MHz (CDCl₃), TMS): δ: 7.26-7.33(5H, m).
- ¹³C-NMR (125 MHz (CDCl₃), TMS): δ: 128.01, 129.56, 130.08, 132.05, 128.31, 153.48, 167.42.

### Example 4

### 4,6-Dichloro-5-(4-chlorophenylthio)-2-methylaminopyrimidine

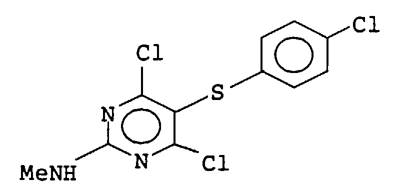

2,4,6-Trichloro-5-(4-chlorophenylthio)pyrimidine (1.14 g) obtained by the same process as in Example 1 was dissolved in methyl ethyl ketone (6 ml), followed by adding thereto 3 ml of water and methylamine (a 40% aqueous solution, 270 mg), and the reaction temperature was adjusted to 5°C or lower. The reaction mixture was stirred for 30 minutes and then slowly heated to room temperature, and 5 ml of a 30% aqueous NaOH solution was added dropwise. The resulting mixture was vigorously stirred at room temperature for 2 hours, after which the precipitated crystals (495 mg), the desired compound were collected by filtration.
- ¹H-NMR (500 MHz (CDCl₃), TMS): δ: 3.05(3H, d, J=4.5Hz), 7.05(2H, d, J=7.0Hz), 7.27(2H, d, J=8.0Hz)
- ¹³C-NMR (125 MHz (CDCl₃), TMS): δ: 28.91, 105.37, 128.25, 128.94, 129.28, 129.72, 131.00, 133.08, 160.70, 164.62.

### Example 5

### 4,6-Dichloro-5-(3-chlorophenylthio)-2-methylaminopyrimidine

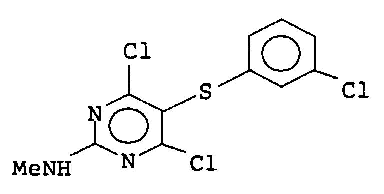

Except for using 2,4,6-trichloro-5-(3-chlorophenylthio)pyrimidine obtained by the same process as in Example 2, in place of 2,4,6-trichloro-5-(4-chlorophenylthio)pyrimidine, crystals (480 mg) of the desired compound were obtained from the 2,4,6-trichloro-5-(3-chlorophenylthio)pyrimidine (1.14 g) in the same manner as in Example 4.
- ¹H-NMR (500 MHz (CDCl₃), TMS): δ: 3.07(3H, d, J=5.0Hz), 7.05-7.06(1H, m), 7.11-7.12(1H, m), 7.15-7.17(1H, m), 7.19-7.22(1H, m).
- ¹³C-NMR (125 MHz (CDCl₃), TMS): δ: 28.70, 111.01, 125.27, 126.54, 126.80, 130.15, 135.04, 137.56, 160.85, 167.00.

### Example 6

### 4,6-Dichloro-2-methylamino-5-phenylthiopyrimidine

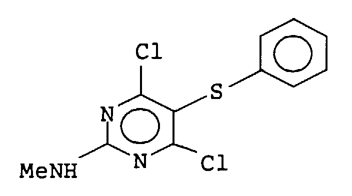

Except for using 2,4,6-trichloro-5-phenylthiopyrimidine obtained by the same process as in Example 3, in place of 2,4,6-trichloro-5-(4-chlorophenylthio)pyrimidine, crystals of the desired compound were obtained from the 2,4,6-trichloro-5-phenylthiopyrimidine in the same manner as in Example 4.
- ¹H-NMR (500 MHz (CDCl₃), TMS): δ: 3.05(3H, d, J=5.0Hz), 5.91(1H, s), 7.17-7.29(5H, m).
- ¹³C-NMR (125 MHz (CDCl₃), TMS): δ: 28.63, 112.07, 126.40, 127.50, 129.15, 135.42, 160.70, 167.23.

### Example 7

### 4,6-Dichloro-2-dimethylamino-5-phenylthiopyrimidine

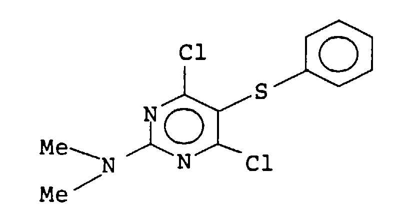

In the same manner as in Example 4, crystals (730 mg) of the desired compound were obtained from 2,4,6-trichloro-5-phenylthiopyrimidine (1.00 g) obtained by the same process as in Example 3 and dimethylamine (a 40% aqueous solution, 387 mg).
- ¹H-NMR (500 MHz (CDCl₃), TMS): δ: 3.21(6H, s), 7.5-7.20(2H, m), 7.22-7.30(3H, m).
- ¹³C-NMR (125 MHz (CDCl₃), TMS): δ: 37.35, 112.61, 135.44, 138.75, 143.46, 144.90, 167.28, 169.08.

### Example 8

### 4-Chloro-5-(4-chlorophenylthio)-2-methylamino-6-(4-methylpiperazino)pyrimidine

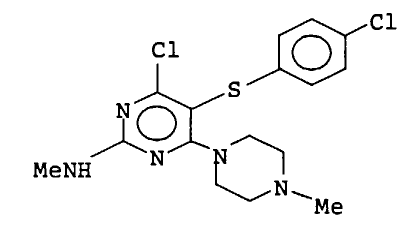

Crystals (495 mg) of 4,6-dichloro-5-(4-chlorophenylthio)-2-methylaminopyrimidine obtained by the same process as in Example 4 were dissolved in 20 ml of ethanol, followed by adding thereto N-methylpiperazine (464 mg), and the resulting mixture was heated under reflux for 3 hours. The reaction mixture was cooled to -30°C to -40°C and the precipitated crystals were recrystallized from ethanol to obtain colorless crystals (460 mg), the desired compound.
- ¹H-NMR (500 MHz (CDCl₃), TMS): δ: 2.27(3H, s), 2.39(4H, br t, J=4.5Hz), 2.98(3H, d, J=5.0Hz), 3.57(4H, br s), 5.47(1H, br s), 6.98(2H, d, J=8.5Hz), 7.22(2H, d, J=8.5Hz).
- ¹³C-NMR (125 MHz (CDCl₃), TMS): δ: 28.36, 45.92, 47.93, 54.90, 126.66, 129.10, 131.07, 136.17, 126.66, 160.54, 167.23, 169.42.
- MS (EI, direct):: 383 (M⁺).

### Example 9

### 4-Chloro-5-(3-chlorophenylthio)-2-methylamino-6-(4-methylpiperazino)pyrimidine

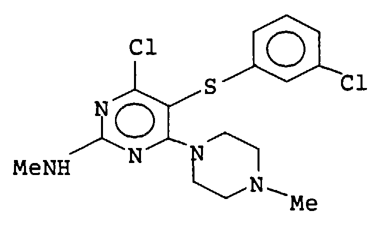

In the same manner as in Example 8, crystals (450 mg) of the desired compound were obtained from crystals (480 mg) of 4,6-dichloro-5-(3-chlorophenylthio)-2-methylaminopyrimidine obtained by the same process as in Example 5.
- ¹H-NMR (500 MHz (CDCl₃), TMS): δ: 2.26(3H, s), 2.38(4H, br t, J=4.5Hz), 2.99(3H, d, J=5.0Hz), 3.75(4H, br s), 5.40(1H, br s), 6.93(1H, ddd, J=1.5, 2.0, 8.0Hz), 7.02(1H, t, J=2.0Hz), 7.09(1H, ddd, J=1.5, 2.0, 8.0Hz), 7.17(1H, t, J=8.0Hz).
- ¹³C-NMR (125 MHz (CDCl₃), TMS): δ: 28.04, 45.96, 47.95, 54.91, 123.38, 125.01, 125.41, 130.00, 134.96, 161.00, 167.18, 167.00.
- MS (EI, direct):: 383 (M⁺).

### Example 10

### 4-Chloro-2-methylamino-6-(4-methylpiperazino)-5-phenylthiopyrimidine

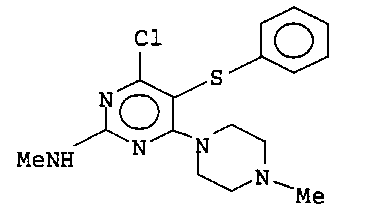

In the same manner as in Example 8, crystals (460 mg) of the desired compound were obtained from crystals (490 mg) of 4,6-dichloro-2-methylamino-5-phenylthiopyrimidine obtained by the same process as in Example 6.
- ¹H-NMR (500 MHz (CDCl₃), TMS): δ: 2.25(3H, s), 2.37(4H, t, J=4.5Hz), 2.98(3H, d, J=5.0Hz), 3.77(4H, br s), 5.40(1H, br s), 7.05-7.26(5H, m).
- ¹³C-NMR (125 MHz (CDCl₃), TMS): δ: 28.36, 45.92, 47.88, 54.90, 125.22, 125.38, 128.97, 137.49, 160.51, 167.26, 168.87.
- MS (EI, direct):: 349 (M⁺).

### Example 11

### 4-Chloro-2-dimethylamino-6-(4-methylpiperazino)-5-phenylthiopyrimidine

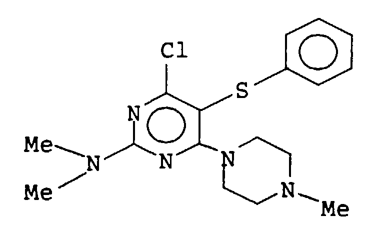

In the same manner as in Example 8, crystals (710 mg) of the desired compound were obtained from crystals (720 mg) of 4,6-dichloro-2-dimethylamino-5-phenylthiopyrimidine obtained by the same process as in Example 7.
- ¹H-NMR (500 MHz (CDCl₃), TMS): δ: 2.24(3H, s), 2.36(4H, br t, J=5.0Hz), 3.16(6H, s), 3.70(4H, br t, J=3.0Hz), 7.04(2H, d, J=8.5Hz), 7.10(1H, t, J=7.5Hz), 7.21-7.26(2H, m).
- ¹³C-NMR (125 MHz (CDCl₃), TMS): δ: 36.97, 45.93, 47.95, 54.85, 125.08, 125.36, 128.88, 137.70, 159.62, 167.10, 168.70.
- MS (EI, direct):: 363 (M⁺).

### Example 12

### 4-Chloro-6-methyl-2-methylamino-5-phenyloxypyrimidine

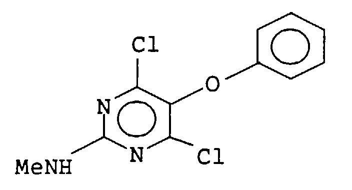

NaH (2.43 g) was suspended in benzene (60 ml), followed by adding thereto phenol (5.71 g), and the resulting mixture was heated under reflux for 5 minutes. After the reaction mixture was cooled to room temperature, a solution (10 ml) of ethyl chloroacetoacetate (10 g) in benzene was added dropwise. The reaction solution was mildly refluxed for 4 hours. The reaction solution was cooled and then poured into ice water to be washed with water. The organic layer was dried over anhydrous magnesium sulfate and then distilled under reduced pressure to remove the solvent. The residue was purified by a conventional method to obtain a colorless oil (6.26 g).

NaH (1.08 g) was dissolved in ethanol (10 ml), followed by adding thereto N-methylguanidine hydrochloride (2.58 g), and the resulting mixture was stirred at room temperature for 10 minutes. A solution (5 ml) of the aforesaid colorless oil (3.0 g) in ethanol was added dropwise, followed by heating under reflux for 1 hour. The reaction mixture was concentrated under reduced pressure, after which ice water was added to the concentrate, and the resulting mixture was made neutral with acetic acid. The precipitate was collected by filtration to obtain colorless crystals (2.52 g). The obtained crystals (1.5 g) were dissolved in phosphorus oxychloride (6 ml), and the resulting solution was heated under reflux for 4 hours. The reaction solution was allowed to cool to room temperature, poured into ice water, made basic with aqueous ammonia, and then extracted with chloroform. The organic layer was dried over anhydrous magnesium sulfate and distilled under reduced pressure to remove the solvent. The residue was purified according a conventional method to obtain the desired compound (300 mg) as yellow crystals.
- ¹H-NMR (270 MHz, CDCl₃): δ: 2.23(3H, s), 3.00(3H, d, J=4.9Hz), 5.22(1H, m), 6.79-7.32(5H, m).

### Example 13

### 4-Methyl-2-methylamino-6-(4-methylpiperazino)-5-phenyloxypyrimidine

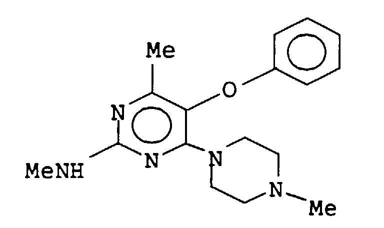

N-methylpiperazine (13.5 mg) was added to an ethanolic solution (1 ml) of 4-chloro-6-methyl-2-methylamino-5-phenyloxypyrimidine (11.2 mg) obtained by the same process as in Example 12, and the resulting mixture was heated under reflux for 8 hours. After the reaction mixture was allowed to cool to room temperature, water was added, followed by extraction with chloroform. The extract was purified by a conventional method to obtain the desired compound (14 mg) as a colorless oil.
- ¹H-NMR (270 MHz, CDCl₃): δ: 2.07(3H, s), 2.17(3H, s), 2.22(4H, m), 2.93(3H, d, J=4.9Hz), 3.67(4H, m), 4.80(1H, m), 6.76-7.27(5H, m).

### Reference Example 1

### Production of dihydropyrimidine-4,6-dione

To 36 g of sodium hydride was added dropwise 500 ml of dried ethanol. After the formed sodium ethoxide was dissolved, 40.25 g of formamidine hydrochloride was added.

The resulting mixture was stirred at room temperature for 2 hours and then allowed to stand overnight. The formed solid was filtered off and 80 g of ethyl malonate was added dropwise to the filtrate. The resulting mixture was stirred at room temperature for 8 hours, and the reaction mixture was concentrated under reduced pressure. The concentrate was poured into ice water and the precipitated crystals were collected by filtration. The crystals were washed with cold water and then dried. These crude crystals were recrystallized from water to obtain 25 g of dihydropyrimidine-4,6-dione as yellow crystals.
- NMR (DMSO-d₆) δ (ppm):: 5.21(1H, s), 8.00(1H, s), 11.77(2H, br).

### Reference Example 2

### Production of 5-bromo-pyrimidine-4,6-diol

In 25 ml of glacial acetic acid was dissolved 15 g of dihydropyridine-4,6-dione and the resulting solution was heated to 70°C. A solution of 23.3 g of bromine in 50 ml of glacial acetic acid was slowly dropped thereinto. The reaction solution was cooled to room temperature and allowed to stand overnight. After standing, the precipitated crystals were collected by filtration. These crude crystals were recrystallized from water to obtain 14.5 g of 5-bromo-pyrimidine-4,6-diol as orange crystals.
- NMR (DMSO-d₆) δ (ppm):: 8.18(1H, s), 12.60(2H, br).

### Example 14

### Production of 4,6-dichloro-5-phenylsulfanylpyrimidine

In 5 ml of dimethylformamide was suspended 207 mg of sodium hydride (a 60% oil dispersion). Thereto was added 628 mg of thiophenol. The resulting mixture was stirred at room temperature for 1 hour to obtain solution 1. On the other hand, 1.0 g of 5-bromopyrimidine-4,6-diol was dissolved in 5 ml of dimethylformamide to obtain solution 2. Solution 1 was added dropwise to solution 2. The resulting mixture was stirred at room temperature for 3 hours and then poured into ice water. The precipitated crystals were collected by filtration and dried. Then, the crystals were dissolved in 3 ml of phosphorus oxychloride and the resulting solution was heated under reflux. The reaction solution was cooled to room temperature and poured into ice water. The resulting mixture was made basic with aqueous ammonia and extracted with methylene chloride. The organic layer was washed with water and then dried over anhydrous magnesium sulfate. The drying agent was removed and the solvent was distilled off under reduced pressure to obtain crude crystals. The crude crystals were recrystallized from methanol/water to obtain 490 mg of 4,6-dichloro-5-phenylsulfanylpyrimidine as light-brown crystals.
- NMR (CDCl₃) δ (ppm):: 6.89-7.02(3H, m), 7.15-7.20(2H, m), 8.42(1H, s).

### Example 15

### Production of 4-chloro-6-(4-methylpiperazinyl)-5-phenylsulfanylpyrimidine

In dry ethanol were dissolved 200 mg of 4,6-dichloro-5-phenylsulfanylpyrimidine and 85.7 mg of N-methylpiperazine, and the resulting solution was heated under reflux for 5 hours. The reaction solution was extracted with toluene and acidified with 1N hydrochloric acid. Aqueous ammonia and toluene were added to the aqueous layer, and the toluene layer was separated, washed with a saturated aqueous sodium chloride solution, and then dried over anhydrous magnesium sulfate. The drying agent was removed and the solvent was distilled off under reduced pressure to obtain a colorless oil. The oil was purified on a TLC plate for separation (developer; chloroform : methanol = 9 : 1) to obtain 80 mg of 4-chloro-6-(4-methylpiperazinyl)-5-phenylsulfanylpyrimidine.
- NMR (CDCl₃) δ (ppm):: 2.22(3H, s), 2.25-2.34(4H, m), 3.71-3.80(4H, m), 6.99-7.04(1H, m), 7.13-7.50(4H, m), 8.36(1H, s).

### Example 16

### Production of 4,6-dichloro-5-(4-chlorophenylsulfanyl)-pyrimidine

In 5 ml of dimethylformamide was suspended 249 mg of sodium hydride (a 60% oil dispersion). Thereto was added 628 mg of 4-chlorothiophenol. The resulting mixture was stirred at room temperature for 1 hour to obtain solution 1. On the other hand, 1.0 g of 5-bromopyrimidine-4,6-diol was dissolved in 5 ml of dimethylformamide to obtain solution 2. Solution 1 was added dropwise to solution 2. The resulting mixture was heated under reflux for 8 hours, cooled to room temperature, and then poured into ice water. The precipitated crystals were collected by filtration, washed with water and then dried.

Subsequently, the crystals were dissolved in 3 ml of phosphorus oxycloride and the resulting solution was heated under reflux for 2 hours. The reaction solution was cooled to room temperature and poured into ice water. The resulting mixture was made basic with aqueous ammonia and extracted with toluene. The organic layer was washed with water and then dried over anhydrous magnesium sulfate. The drying agent was removed and the solvent was distilled off under reduced pressure to obtain 600 mg of 4,6-dichloro-5-(4-chlorophenyl)sulfanylpyrimidine as yellow crystals.
- NMR (CDCl₃) δ (ppm):: 7.21-7.40(4H, m), 8.72(1H, s).

### Example 17

### Production of 4-chloro-6-(4-methylpiperazinyl)-5-(4-chlorophenylsulfanyl)pyrimidine

In dry ethanol were dissolved 200 mg of 4,6-dichloro-5-phenylsulfanylpyrimidine and 85.7 mg of N-methylpiperazine, and the resulting solution was heated under reflux for 5 hours. The reaction solution was extracted with toluene and acidified with 1N hydrochloric acid. Aqueous ammonia and toluene were added to the aqueous layer, and the toluene layer was separated, washed with a saturated aqueous sodium chloride solution, and then dried over anhydrous magnesium sulfate. The drying agent was removed and the solvent was distilled off under reduced pressure to obtain a colorless oil. The oil was purified on a TLC plate for separation (developer; chloroform : methanol = 9 : 1) to obtain 80 mg of 4-chloro-6-(4-methylpiperazinyl)-5-(4-chlorophenylsulfanyl)pyrimidine.
- NMR (CDCl₃) δ (ppm):: 2.25(3H, s), 2.33-2.37(4H, m), 3.75-3.79(4H, m), 6.93-7.00(2H, m), 7.20-7.27(2H, m), 8.37(1H, s).

### Example 18

### Production of 4,6-dichloro-5-(2-chlorophenylsulfanyl)-pyrimidine

In 5 ml of dimethylformamide was suspended 249 mg of sodium hydride (a 60% oil dispersion). Thereto was added 628 mg of 2-chlorothiophenol. The resulting mixture was stirred at room temperature for 1 hour to obtain solution 1. On the other hand, 1.0 g of 5-bromopyrimidine-4,6-diol was dissolved in 5 ml of dimethylformamide to obtain solution 2. Solution 1 was added dropwise to solution 2. The resulting mixture was heated under reflux for 8 hours, cooled to room temperature, and then poured into ice water. The crystals precipitated were collected by filtration, washed with water and then dried.

Subsequently, the crystals were dissolved in 3 ml of phosphorus oxycloride and the resulting solution was heated under reflux for 2 hours. The reaction solution was cooled to room temperature and then poured into ice water. The resulting mixture was made basic with aqueous ammonia and extracted with toluene. The organic layer was washed with water and then dried over anhydrous magnesium sulfate. The drying agent was removed and the solvent was distilled off under reduced pressure to obtain 453 mg of 4,6-dichloro-5-(2-chlorophenyl)sulfanylpyrimidine as yellow crystals.
- NMR (CDCl₃) δ (ppm):: 6.91-7.56(4H, m), 8.75(1H, s).

### Example 19

### Production of 4-chloro-6-(4-methylpiperazinyl)-5-(2-chlorophenylsulfanyl)pyrimidine

In dry ethanol were dissolved 200 mg of 4,6-dichloro-5-phenylsulfanylpyrimidine and 75.6 mg of N-methylpiperazine, and the resulting solution was heated under reflux for 6 hours. The reaction solution was extracted with toluene and acidified with 1N hydrochloric acid. Aqueous ammonia and toluene were added to the aqueous layer, and the toluene layer was separated, washed with a saturated aqueous sodium chloride solution, and then dried over anhydrous magnesium sulfate. The drying agent was removed and the solvent was distilled off under reduced pressure to obtain a colorless oil. The oil was purified on a TLC plate for separation (developer; chloroform : methanol = 9 : 1) to obtain 50 mg of 4-chloro-6-(4-methylpiperazinyl)-5-(2-chlorophenylsulfanyl)pyrimidine.
- NMR (CDCl₃) δ (ppm):: 2.22(3H, s), 2.29-2.33(4H, m), 3.75-3.80(4H, m), 6.64-7.10(1H, m), 7.09-7.16(2H, m), 7.34-7.41(1H, m), 8.39(1H, s).

### INDUSTRIAL APPLICABILITY

The novel pyrimidine derivative of the above general formula [I] according to the present invention has a strong affinity for receptors of central nervous system, such as dopamine, serotonin and the like, and hence is useful as a psychotropic drug. For example, it is useful as a therapeutic drug for schizophrenia, manic-depressive psychosis, Parkinson's disease, psychosis caused by drug abuse, psychosis associated with senile dementia, etc.

The compound [I] of the present invention is characterized by having little effect on α₁-receptor and a strong affinity for receptors such as dopamine, serotonin and the like. Conventional psychotropic drugs such as chlorpromazine have side effects such as drop of consciousness level and orthostatic hypotension because of their α₁-receptor blocking effect. By contast, the compound of the present invention has little affinity for α₁-receptor and a strong affinity for receptors such as dopamine D3, serotonin 5-HT2, etc.

The compound of the formula [III] is important as an intermediate for producing the compound of the formula [I], and the final desired compound of the present invention represented by the formula [I] can easily be produced through the compound of the formula [III].

## Claims

1. A pyrimidine derivative represented by the formula [I], or a pharmacologically acceptable salt thereof: 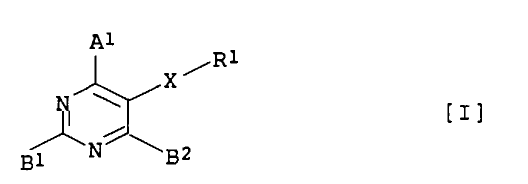
wherein X is a sulfur atom or an oxygen atom;
A¹ is a halogen atom, a lower alkyl group, a cycloalkyl group, an aryl group, a substituted lower alkyl group or a substituted cycloalkyl group in the case of X being a sulfur atom, and A¹ is a lower alkyl group, a cycloalkyl group, an aryl group, a substituted lower alkyl group or a substituted cycloalkyl group in the case of X being an oxygen atom;
B¹ is a hydrogen atom, a lower alkyl group, a cycloalkyl group, an aryl group, a substituted aryl group, an amino group or a substituted amino group;
B² is an amino group or a substituted amino group; and
R¹ is a group selected from groups represented by the formulas (1) to (6): 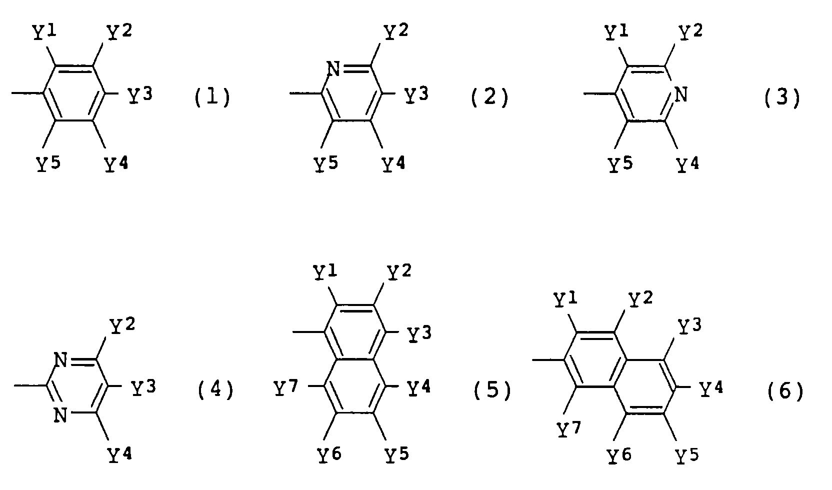
wherein Y¹, Y², Y³, Y⁴, Y⁵, Y⁶ and Y⁷, which may be the same or different, are hydrogen atoms, halogen atoms, lower alkyl groups, cycloalkyl groups, lower alkenyl groups, lower alkynyl groups, aryl groups, heterocyclic groups, substituted lower alkyl groups, substituted cycloalkyl groups, substituted lower alkenyl groups, substituted lower alkynyl groups, substituted aryl groups, or substituted heterocyclic groups.

2. A pyrimidine derivative according to claim 1 represented by the formula [II], or a pharmacologically acceptable salt thereof: 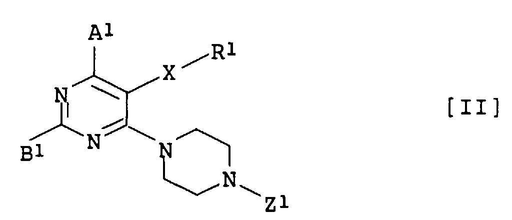 wherein A¹, B¹, X and R¹ are as defined in claim 1, and Z¹ is a hydrogen atom, a lower alkyl group or a substituted lower alkyl group.

3. A pyrimidine derivative or a pharmacologically acceptable salt thereof according to claim 2, wherein A¹ is a halogen atom and X is a sulfur atom.

4. A process for producing a pyrimidine derivative [I] or a pharmacologically acceptable salt thereof according to claim 1, characterized by reacting a compound represented by the formula [III]: 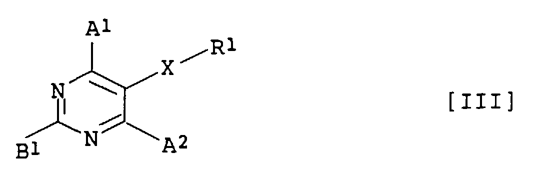 wherein A¹, B¹, X and R¹ are as defined in claim 1 and A² is a halogen atom, with an amine represented by the formula H-B² wherein B² is an amino group or a substituted amino group.

5. A compound represented by the formula [IV]: 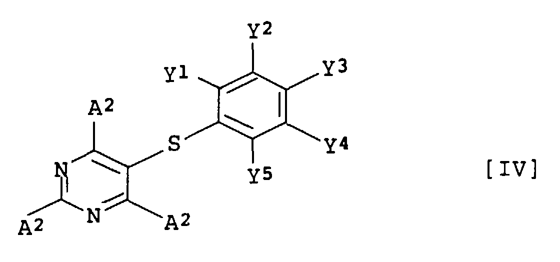 wherein A² is a halogen atom, and Y¹, Y², Y³, Y⁴ and Y⁵, which may be the same or different, are hydrogen atoms, halogen atoms, lower alkyl groups, cycloalkyl groups, lower alkenyl groups, lower alkynyl groups, aryl groups, heterocyclic groups, substituted lower alkyl groups, substituted cycloalkyl groups, substituted lower alkenyl groups, substituted lower alkynyl groups, substituted aryl groups, or substituted heterocyclic groups.
